(19) Europäisches Patentamt
European Patent Office
Office européen des brevets

(11) **EP 3 148 642 B1**

(12) **EUROPÄISCHE PATENTSCHRIFT**

(45) Veröffentlichungstag und Bekanntmachung des Hinweises auf die Patenterteilung:
**20.02.2019 Patentblatt 2019/08**

(21) Anmeldenummer: **15725335.2**

(22) Anmeldetag: **27.05.2015**

(51) Int Cl.:
**A61N 5/06** (2006.01)

(86) Internationale Anmeldenummer:
**PCT/EP2015/061747**

(87) Internationale Veröffentlichungsnummer:
**WO 2015/181251 (03.12.2015 Gazette 2015/48)**

(54) **VORRICHTUNG ZUR PRÄVENTION EINER SCHWERHÖRIGKEIT ODER GLEICHGEWICHTSSTÖRUNG**

APPARATUS FOR THE PROPHYLAXIS OF HEARING IMPAIRMENT OR VERTIGO

DISPOSITIF POUR LA PRÉVENTION DE LA SURDITÉ OU DES TROUBLES DE L'ÉQUILIBRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.05.2014 DE 102014107447**
**27.05.2014 DE 102014107448**

(43) Veröffentlichungstag der Anmeldung:
**05.04.2017 Patentblatt 2017/14**

(73) Patentinhaber:
• **Ernst, Arneborg**
**14169 Berlin (DE)**
• **Basta, Dietmar**
**14656 Brieselang (DE)**

(72) Erfinder:
• **Ernst, Arneborg**
**14169 Berlin (DE)**
• **Basta, Dietmar**
**14656 Brieselang (DE)**

(74) Vertreter: **Hertin und Partner Rechts- und Patentanwälte PartG mbB Kurfürstendamm 54/55 10707 Berlin (DE)**

(56) Entgegenhaltungen:
WO-A2-2007/115565    DE-T2- 69 728 173
US-A1- 2010 174 329    US-A1- 2011 282 417
US-A1- 2011 295 331    US-A1- 2013 023 962

**Beschreibung**

Die Erfindung betrifft eine Bestrahlungsvorrichtung zur Prävention von Hörschäden und/oder Gleichgewichtstörungen und ein System aus wirkverbundenen Vorrichtungskomponenten, die miteinander wechselwirken zur präventiven Bestrahlung des Innenohres mit Photonen, um Hörschäden und/oder Gleichgewichtsstörungen bei einem Träger der Bestrahlungsvorrichtung vorzubeugen.

Das Hörvermögen stellt für den Menschen einen wichtigen Sinn zur Wahrnehmung seiner Umwelt dar und ist Vorrausetzung für die mündliche Kommunikation mit seinen Mitmenschen. Durch verschiedene Geräusch- und Lärmquellen in seiner Umwelt ist das menschliche Ohr einer starken Belastung ausgesetzt, welche sich in Schäden an der Gehörschnecke (Kochlea) im Innenohr ausdrücken. Neben der Kochlea umfasst das Innenohr den Vestibularapparat, welcher einen zentralen Bestandteil des Gleichgewichtsorganes des Menschen darstellt. Der Gleichgewichtssinn dient der Feststellung der Körperhaltung sowie der Orientierung im Raum und ist somit essentiell für eine stabile Körperhaltung und Fortbewegung. Als neuroanatomische Anzeichen einer degenerativen Entwicklung im Hör- und Gleichgewichtssystem infolge von Umwelteinflüssen, Erkrankungen oder Alterungsvorgängen ist die Abnahme der Haaressinneszellen in der Kochlea und am Vestibularapparat sowie nachgeschalteter Neuronen bekannt. Die peripheren auditorischen und vestibulären Strukturen im Innenohr, d.h. die rezeptiven und neuronalen Strukturen, die dem Hören und der Wahrnehmung des Gleichgewichtssinnes dienen, werden dabei frühzeitig betroffen. Zunächst sterben die äußeren Haarsinneszellen sowie die Spiralganglienzellen ab. Bei fortschreitender Entwicklung werden innere Haarsinneszellen und zentralnervöse auditorische und vestibuläre Strukturen geschädigt.

Im Stand der Technik existieren Methoden zur Behandlung von Hörschäden. Prell et al. Hear Res. 226(1-2): 22-43 (2007) beschreibt die Behandlung von Lärmtraumata durch die Verabreichung antioxidativer Mittel. Die Wirkstoffeinnahme kann jedoch mit Nebenwirkungen verbunden sein.

DE 29720442 U1 bzw. DE 29808193 U1 beschreiben jeweils eine Vorrichtung, bei der ein Laser in die Hörmuschel eines Tonübertragungsgerät bzw. in eine Gehörschutzvorrichtung integriert ist und dessen Strahlung Schäden am Innenohr vorbeugen soll. Diese Methoden der Prävention sind jedoch auf Situationen beschränkt, in denen die akustische Wahrnehmung der Umgebung eingeschränkt ist und somit nicht für eine langfristige Prävention während des gesamten Alltag geeignet. Weiterhin werden die bevorzugten Parameterbereiche für die Laserausgangsstrahlung auf 1 mW bis 50 mW und für die Wellenlänge auf 600 nm - 900 nm spezifiziert. Bei Anwendung der Vorrichtung mit einer allgemeinen Wahl von Parametern aus diesen Bereichen wird keine wirkungsvolle Prävention erzielt. US 2013/0172960 A1 offenbart ein Verfahren zur partiellen Reversion von Lärmschwerhörigkeit durch eine Low-Level-Lasertherapie (LLLT) nach einem Lärmtrauma. Bei dieser wird das Innenohr bevorzugt mit Laserlicht einer Wellenlänge von 780 nm, 830 nm oder 980 nm und einer Ausgangsintensität von 165 mW/cm$^2$ täglich für eine Dauer von 60 min bestrahlt wird. Das Verfahren definiert bevorzugte Parameter für die Behandlung im Anschluss an ein Lärmtrauma und erzielt therapeutische Erfolge insbesondere nach mehr als 10 Tagen der Behandlung. Das offenbarte Verfahren eignet sich jedoch nicht für eine Prävention von Hörschäden.

Weiterhin offenbaren die US 2011/295331 A1, US 2013/023962 A1, US 2010/174329 A1 und US 2011/282417 A1 Vorrichtungen zur Bestrahlung eines Innenohres mit Licht im Infrarotbereich, um durch optische Stimulation auditorische Sinneseindrücke in den Nervenzellen zu erzeugen. Die Leistung der Bestrahlung wird in Abhängigkeit akustischer Signale moduliert und auf das Auslösen von Aktionspotentialen optimiert. Eine schützende Wirkung auf das Innenohr um Schädigungen vorzubeugen, wird durch die stimulierende Bestrahlung nicht erreicht.

Der Erfindung liegt die Aufgabe zugrunde, eine Vorrichtung und ein diese Vorrichtung umfassendes System bereitzustellen, welche eine wirkungsvolle Prävention von Hör- und/oder Gleichgewichtstörungen erreichen und dazu die Nachteile des Standes der Technik beseitigen.

Diese Aufgabe wird durch eine Photonenbestrahlungsvorrichtung gemäß Anspruch 1 gelöst. Die Bestrahlungsvorrichtung zur Prävention von Hör- und/oder Gleichgewichtstörungen ist dadurch gekennzeichnet, dass die Vorrichtung einen Photonenstrahler zur Bestrahlung eines Innenohres und eine computergesteuerte Regelungseinheit umfasst, die basierend auf Messdaten die Ausgangsleistung des Photonenstrahles steuert.

Die erfindungsgemäße Vorrichtung kann vorteilhafterweise in ein Vorrichtungssystem eingebunden ein, welches mehrere Vorrichtungskomponenten umfasst, wobei Signale über einen Träger der Bestrahlungsvorrichtung und/oder die Umgebung des Trägers gemessen werden, weiterhin eine Ausgangsleistung eines Photonenstrahlers (P) zur protektiven Bestrahlung des Innenohres basierend auf diesen Messdaten berechnet wird und die Ausgangsleistung des Photonenstrahlers auf den berechneten Wert P eingestellt wird und die Bestrahlung des Innenohres mit der Ausgangsleistung P erfolgt. D.h., bei dem erfindungsgemäßen System, welches eine Gruppe von miteinander verbundenen bzw. wirkverbundenen Vorrichtungselementen betrifft, wirken mehrere Vorrichtungskomponenten funktionell miteinander, um das erfindungsgemäße Ziel zu erreichen.

Ein Verfahren zur Prävention von Hör- und/oder Gleichgewichtsstörungen unter Verwendung der erfindungsgemäßen Bestrahlungsvorrichtung wird auch offenbart. Bevorzugte Ausführungsformen sind Gegenstände der abhängigen Ansprüche.

Gemäß Anspruch 1 ist die Bestrahlungsvorrichtung zur Prävention von Hör- und/oder Gleichgewichtstörungen dadurch gekennzeichnet, dass die Vorrichtung einen Photonenstrahler zur Bestrahlung eines Innenohres und eine automatisierte, messdatenbasierte Regelungseinheit zur Steuerung der Ausgangsleistung des Photonenstrahlers, umfasst.

Unter einem Photonenstrahler wird bevorzugt eine Strahlungsquelle verstanden, welche Photonen emittiert. Ganz bevorzugte Ausführungsformen solcher Photonenstrahler sind Photonen emittierende Laser oder Leuchtdioden.

Mit der Ausgangsleistung des Photonenstrahlers ist insbesondere die gesamte Strahlungsleistung, des durch den Photonenstrahler emittierten Photonenstrahles, gemeint. Insbesondere ist somit mit der Ausgangsleistung des Photonenstrahlers die emittierte Photonenstrahlenergie pro Zeiteinheit gemeint. Die bevorzugte Einheit für die Ausgangsleistung des Photonenstrahlers ist Watt (W) oder mW. Dem Fachmann ist bekannt, wie die Ausgangsleistung eines Photonenstrahles bevorzugt mit Hilfe eines Photometers bestimmt werden kann. Bei der Fokussierung des Photonenstrahles auf ein bestimmtes Ziel, bevorzugt auf das Innenohr, ist mit der Ausgangsleistung des Photonenstrahlers insbesondere die Strahlungsleistung gemeint, welche auf das zu bestrahlende Ziel, insbesondere das Innenohr trifft. Insbesondere gilt dies auch, wenn durch optische Elemente auf dem optischen Weg des Photonenstrahles innerhalb des Photonenstrahlers, aber auch durch optische Elemente zum Beispiel zur Leitung des Photonenstrahles, außerhalb des Photonenstrahlers, Photonen absorbiert werden. Mit der Ausgangsleistung des Photonenstrahlers ist bevorzugt die Strahlungsleistung gemeint, welche biologisch relevant ist, d.h. insbesondere die Strahlungsleistung, welche auf biologisches Gewebe, bevorzugt das Innenohr trifft. Des Weiteren ist mit der Leistung des Photonenstrahles, der Photonenstrahlleistung sowie die Strahlleistung bevorzugt die Ausgangsleistung des Photonenstrahlers gemeint. In Ausführungsformen, bei denen der Photonenstrahler auf einen bestimmten Wellenlängenbereich beschränkt wird, ist mit der Ausgangsleistung des Photonenstrahlers bevorzugt die Strahlungsleistung, integriert über diesen Wellenlängenbereich, gemeint. Des Weiteren ist mit einer erhöhten oder verringerten Photonenstrahlleistung, einer erhöhten oder verringerten Bestrahlung, einer erhöhten oder verringerten Photonenbestrahlung sowie analogen Formulierungen eine Photonenbestrahlung mit einer erhöhten oder verringerten Ausgangsleistung des Photonenstrahlers gemeint. Die Photonenstrahlintensität, die Bestrahlungsintensität oder die Intensität der Photonenbestrahlung ist insbesondere gegeben durch die Ausgangsleistung des Photonenstrahlers geteilt durch die Querschnittsfläche des Photonenstrahles, insbesondere die Querschnittsfläche des Photonenstrahles, mit welcher der Photonenstrahl bei der Bestrahlung des Innenohres auf biologisches Gewebe trifft. Die bevorzugte Einheit der Photonenstrahlintensität ist $mW/cm^2$.

Unter einer automatisierten, messdatenbasierten Regelungseinheit zur Steuerung der Ausgangsleistung des Photonenstrahlers wird bevorzugt eine Vorrichtung verstanden, welche basierend auf Messdaten zum Beispiel über die Umgebungslautstärke, über die Reproduzierbarkeit von evozierten otoakustischen Emissionen oder über die Veränderung von Körperpositionen des Trägers die Ausgangsleistung des Photonenstrahlers steuert. Insbesondere, steuerte die automatisierte, messdatenbasierten Regelungseinheit dabei die Ausgangsleistung des Photonenstrahlers auf einen Wert, welcher basierend auf den Messdaten durch die Regelungseinheit berechnet wurde. Mit Messdaten sind insbesondere solche Daten oder Informationen gemeint, welche durch Messgeräte aufgenommen werden können, wie z.B. Mikrofone oder Gyrometer. Besonders bevorzugt betreffen die Messdaten dabei Informationen über die Umgebung des Trägers der Bestrahlungsvorrichtung zum Beispiel über akustische Signale, Umgebungslärm oder den Schalldruckpegel in der Umgebung. Weiterhin betreffen die Messdaten bevorzugt Informationen über den Träger selbst, z.B. über das Hörvermögen des Trägers oder über Körperschwankungen des Trägers. Ganz bevorzugt betreffen die Messdaten solche Informationen, welche Auskunft geben über den Zustand des Innenohres des Trägers der Bestrahlungsvorrichtung oder aber über Faktoren, die den Zustand des Innenohres des Trägers beeinflussen. Des Weiteren erfolgt die Steuerung der Leistung des Photonenstrahlers bevorzugt automatisiert, d.h. bevorzugt ohne einen Input durch den Träger der Bestrahlungsvorrichtung, insbesondere bevorzugt ohne einen manuellen Input durch den Träger durch Betätigung eines Drehknopf, eines Regelschiebers oder andere Vorrichtungen zur manuellen Steuerung der Ausgangsleistung des Photonenstrahlers. Bevorzugt wird unter einer automatisierten, messdatenbasierten Regelungseinheit zudem eine Vorrichtung verstanden, an welche nicht nur die Messdaten übertragen werden können, sondern die Messdaten zudem bearbeitet werden können. Bevorzugt umfasst die automatisierte, messdatenbasierte Regelungseinheit zur Steuerung der Ausgangsleistung des Photonenstrahlers daher eine Vorrichtung zur Aufnahme, Verarbeitung und Übertragung von Daten, insbesondere Messdaten. Die automatisierte, messdatenbasierten Regelungseinheit umfasst somit bevorzugt eine elektronische Schaltung, einen Computerchip oder eine andere Datenverarbeitungsvorrichtung. Erfindungsgemäß ist mit einer computergesteuerten Regelungseinheit, einer Steuerungseinheit, einer messdatenbasierten Regelungseinheit, einer automatisierten Regelungseinheit bevorzugt eine automatisierte, messdatenbasierte Regelungseinheit zur Steuerung der Ausgangsleistung des Photonenstrahlers gemeint.

Die automatisierte, messdatenbasierte Regelungseinheit zur Steuerung der Ausgangsleistung des Photonenstrahles ermöglicht vorteilhafterweise eine Optimierung der Photonenbestrahlung des Innenohres zur Steigerung der präventiven Wirkung der Bestrahlungsvorrichtung. Dabei steuert die automatisierte, messdatenbasierte Regelungseinheit die Ausgangsleistung des Photonenstrahlers zur Bestrahlung des Innenohres basierend auf Signalen über den Träger der Bestrahlungsvorrichtung und/oder dessen Umgebung. Die messdatenbasierte Regelung der Leistung der Photonenbestrahlung des Innenohres ermöglicht somit insbesondere eine Anpassung der Bestrahlungsleistung an den Zustand der auditorischen und/oder vestibulären Strukturen des Innenohres des Trägers der Bestrahlungsvorrichtung sowie an Faktoren aus der Umwelt, welche auf den Zustand des Innenohres wirken. Die messdatenbasierte Steuerung der Photonenstrahlung ermöglicht dadurch eine überraschend wirkungsvolle präventive Photonenbestrahlung zur Vorbeugung von Hör- und/oder Gleichgewichtsschäden.

Unter der protektiven Wirkung der Photonenbestrahlung auf das Innenohr wird insbesondere verstanden, dass die Photonenbestrahlung des Innenohres zu einer Erhöhung der Schutzfunktion des Innenohres führt. Die protektive Wirkung führt somit insbesondere dazu, dass das Innenohr gegenüber potentiellen Schädigungen geschützt ist. Die protektive Wirkung setzt dabei insbesondere auch gleichzeitig mit der Photonenbestrahlung ein, das bedeutet, dass die protektive Wirkung bereits den Schutz des Innenohres für Belastungen erhöht, die das Innenohr während der Photonenbestrahlung erfährt. Ganz besonders hält die protektive Wirkung der Photonenbestrahlung auf das Innenohr jedoch über einen längeren Zeitraum an, welcher insbesondere mindestens einen Monat betragen kann. Die protektive Wirkung führt somit zu einem aufgebauten Schutz des Innenohres, insbesondere der auditiorischen und vestibulären Strukturen des Innenohres umfassend Haarsinneszellen, Spiralganglienzellen oder Neuronen, welcher über einen längeren Zeitraum insbesondere über mindestens einen Monat erhalten ist. Dieser erhöhte Schutz führt dazu, dass eine Belastung des Innenohres nicht oder nur in geringem Maße zu einer degenerativen Entwicklung der auditorischen und/oder vestibulären Strukturen führt. Insbesondere ist die protektive Wirkung der Photonenbestrahlung nicht gleich einer therapeutischen Wirkung einer Photonenbestrahlung, welche bereits vorhandene Hörschäden oder Gleichgewichtstörungen reversiert. Bevorzugt ist somit nicht vorgesehen, dass die Bestrahlung des Innenohres zeitlich deutlich (z.B. einen Tag später) nach dem Schädigungsereignis erfolgt. Insbesondere müsste für eine therapeutische Behandlung von Hörschäden die Photonenstrahlleistung anders eingestellt werden, als dies der Fall für eine präventive Photonenbestrahlung ist. Erfindungsgemäß wird die Leistung der Bestrahlungsvorrichtung bevorzugt für eine hohe protektive Wirkung und somit für eine präventive Photonenbestrahlung optimiert.

Unter der präventiven Photonenbestrahlung wird bevorzugt verstanden, dass die Photonenbestrahlung erfolgt, um Hör- und/oder Gleichgewichtsstörungen vorzubeugen. Dabei wird die präventive Photonenbestrahlung bevorzugt zeitlich vor oder zeitgleich mit einer potentiellen Schädigung des Hör- und/oder Gleichgewichtsinnes durchgeführt. Insbesondere betrifft die präventive Photonenbestrahlung dabei bevorzugt keine therapeutische Photonenbestrahlung zur Behandlung von Hörschäden oder Gleichgewichtsschäden nachdem diese aufgetreten sind. Die präventive Photonenbestrahlung basiert dabei bevorzugt auf der entdeckten und besser verstandenen protektiven Wirkung der Photonenbestrahlung. Da der Zeitpunkt einer potentiellen Schädigung des Hör- und/oder Gleichgewichtssinnes oft nicht vorhersehbar ist, erfolgt die präventive Photonenbestrahlung bevorzugt über längere Zeiträume während des Alltags. Eine kontinuierliche, präventive Photonenbestrahlung stärkt insbesondere die Schutzfunktion der auditorischen und/oder vestibulären Strukturen. Da die Schutzwirkung der Photonenbestrahlung auch nach der Bestrahlung über einen längeren Zeitraum anhält, kann durch eine präventive Photonenbestrahlung im gesamten Alltag, insbesondere auch solchen Schäden des Innenohres vorgebeugt werden, welche nicht vorhersehbar sind. Durch die präventive Photonenbestrahlung wird somit bevorzugt einer degenerativen Entwicklung des Hör- und/oder Gleichgewichtssinnes langfristig vorgebeugt. Die präventive Photonenbestrahlung betrifft bevorzugt daher keine therapeutische Behandlung von Hörschäden oder Gleichgewichtsschäden mit dem Ziel der Reversion dieser.

Unter dem Zustand des Innenohres wird bevorzugt der Zustand der auditorischen oder vestibulären Strukturen des Innenohres umfassend Haarsinneszellen, Spiralganglienzellen oder Neuronen verstanden. Dabei ist mit dem Zustand des Innenohres insbesondere die Funktionalität des Innenohres für den Gleichgewichtsinn oder für den Gehörsinn gemeint. Ein guter Zustand des Innenohres kennzeichnet somit insbesondere eine hohe Funktionalität des Innenohres, d.h. insbesondere ein gutes Hörvermögen und einen guten Gleichgewichtssinn. Ein geringeres oder beeinträchtigtes Hörvermögen, wie bei Schwerhörigkeit oder Tinnitus, kennzeichnet insbesondere einen schlechten Zustand des Innenohres. Ebenso kennzeichnet ein geringer Gleichgewichtssinn, wie zum Beispiel im Falle von häufig auftretendem Schwindel, einen schlechten Zustand des Innenohres. Der Zustand des Innenohres ist biologisch insbesondere abhängig von dem Zustand der auditorischen oder vestibulären Strukturen des Innenohres umfassend Haarsinneszellen, Spiralganglienzellen oder Neuronen. Eine Verminderung der Funktionalität von auditorischen oder vestibulären Strukturen des Innenohres, insbesondere eine Verminderung der Funktionalität von Haarsinneszellen oder eine Verminderung der Anzahl der Haarsinneszellen, kennzeichnet somit eine Verschlechterung des Zustandes des Innenohres.

Unter Faktoren, die den Zustand des Innenohres beeinflussen werden insbesondere externe und interne Faktoren verstanden, welche auf das Innenohr wirken, Insbesondere werden darunter solche Faktoren verstanden, welche auf die auditorischen oder vestibulären Strukturen des Innenohres umfassend Haarsinneszellen, Spiralganglienzellen oder Neuronen wirken. Insbesondere werden darunter solche Faktoren verstanden, welche den Zustand des Innenohres verschlechtern können, d.h. welche das Innenohr insbesondere die auditorischen oder vestibulären Strukturen des Innenohres schädigen können. Eine hohe Lärmbelastung durch eine erhöhte Umgebungslautstärke ist insbesondere ein externer Faktor, welcher das Innenohr schädigt. Interne Faktoren, welche das Innenohr schädigen können, sind insbesondere Krankheiten, welche die Funktionalität der Haarsinneszellen verschlechtern oder zum Absterben von

Haarsinnenszellen führen können. Hierzu gehören insbesondere vertebrobasiläre Durchblutungsstörungen, Herz-Kreislauf-Erkrankungen, Arteriosklerose, Stoffwechselstörungen, Autoimmunerkrankungen, Anämie, Entzündungskrankheiten oder Diabetes mellitus.

Vorteilhafterweise bewirkt eine Photonenbestrahlung des Innenohres in den Zellen des Innenohres insbesondere den Haarsinneszellen bestimmte photochemische Reaktionen, welche eine protektive Wirkung auf die Zellen haben, vor allem wenn der Photonenstrahler zur Bestrahlung eines Innenohres eine automatisierte, messdatenbasierte Regelungseinheit zur Steuerung der Ausgangsleistung des Photonenstrahlers, umfasst. Diese photochemischen Reaktionen werden auch als Biostimulation, Photostimulation oder Photobiostimulation bezeichnet. Die Erkenntnis, dass die folgend beschriebenen photochemischen Reaktionen eine besonders hohe protektiven Wirkung auf erfindungsgemäß bestrahlte Zellen haben war neu und überraschend. In den Zellen des bestrahlten Gewebes, insbesondere des Innenohres, werden die auftreffenden Lichtphotonen insbesondere von den Chromophoren absorbiert. Durch den Absorptionsvorgang werden Elektronen des Chromophores angeregt und springen von einem niedrigenergetischen Orbital in ein höherenergetisches Orbital. Die dadurch gewonnen Energie wird von dem biologischen System genutzt, um eine Vielzahl von verschiedener zellulärer Aufgaben auszuführen. Insbesondere werden Chromophore in den Mitochondrien durch die Photostimulation in einer derartigen Weise angeregt werden. So führt die erfindungsgemäße Bestrahlung von Gewebe mit Photonen zu einer überraschenden Zunahmen von Produkten der Mitochondrien wie zum Beispiel Adenosintriphosphat (ATP), NADH (die reduzierte Form von Nicotinamidadenindinukleotid (NAD)), Proteinen und Ribonukleinsäure (RNS) sowie zu einer reziproken Zunahme des Sauerstoffverbrauches. Vorteilhafterweise wird die Zellatmung erhöht wird, wenn Mitochondrien durch eine erfindungsgemäße Bestrahlung mit Photonen angeregt werden. Durch eine Optimierung der Aktionsspektren für die photoinduzierte biologische Aktivität auf die Absorptionsspektren von vier membrangebundenen Komplexen in den Mitochondrien konnte das Chromophore identifiziert werden, welches eine zentrale Rolle in der erfindungsgemäßen Photostimulation spielt. Dieses Verfahren identifizierte den Komplex IV, auch als Cytochromc-Oxidase (CCO) bezeichnet, als einen zentralen Chromophor für die protektive Wirkung der Photonenbestrahlung.

CCO ist ein großer Transmembranprotein-Komplex, welcher zwei Kupferzentren sowie zwei Häm-Eisenzentren als Komponenten der Atmungskette umfasst. In der Elektronentransportkette der Atmung werden hochenergetischen Elektronen von Elektronenträger zu einer Reihe von Transmembran-Komplexe (einschließlich CCO) bis hin zum endgültigen Elektronen-Akzeptor transportiert. Dadurch wird ein Protonengradient erzeugt, welcher genutzt wird, um ATP zu erzeugen. Die Bestrahlung durch Photonen beeinflusst durch die Anregung eines transmembranen Komplexes in der Elektronentransportkette direkt die ATP Produktion. Vorteilhafterweise führt die erfindungsgemäße Photonenbestrahlung zu einer Erhöhung der Produktion von ATP. Weiterhin wird in den erfindungsgemäß photonenbestrahlten Zellen insbesondere Stickstoffmonoxid (NO) freigesetzt. Diese Beobachtung deutet darauf hin, dass CCO und die Freisetzung von Stickstoffmonoxid auf mindestens zwei mögliche Arten miteinander verbunden sind. Zum einen kann die erfindungsgemäße Photostimulation zu einer photoinduzierten Dissoziation von Stickstoffmonoxid führen. Die Zellatmung wird durch die Produktion von Stickstoffmonoxid durch mitochondriale Stickstoffmonoxid-Synthasen (mtNOS, einer mitochondrienspezifisichen Isoform von NOS) runterreguliert, indem Stickstoffmonoxid an CCO bindet und es inhibiert. Durch die Bindung an CCO verdrängt Stickstoffmonoxid Sauerstoff und inhibiert dadurch die Zellatmung sowie die Produktion von ATP. Die Photonenbestrahlung oder Photostimulation hingegen dissoziiert Stickstoffmonoxid von CCO und führt somit zu einer Erhöhung der ATP Produktion. Zum anderen kann ein alternativer Mechanismus zu einer Freisetzung von Stickstoffmonoxid durch Photonenbestrahlung führen. CCO kann enzymatisch als eine Nitritreduktase fungieren und Nitrit zu Stickstoffmonoxid reduzieren. Dies geschieht insbesondere, wenn der Sauerstoffpartialdruck gering ist. Durch die photoinduzierte Anregung der Funktion von CCO als Nitritreduktase kann somit die Konzentration von Stickstoffmonoxid erhöht werden. Insbesondere haben Studien gezeigt, dass eine Photonenbestrahlung die CCO/NO Synthese bei physiologischen Nitritkonzentrationen unter ischämischen Bedingungen erhöht. Weiterhin ist insbesondere der Effekt der erfindungsgemäßen Photonenbestrahlung auf die Elektronentransportkette nicht auf die ATP Produktion der Zellen beschränkt. Insbesondere ist mit der erfindungsgemäßen Photonenstrahlung die Strahlung gemeint, deren Strahlungsleistung messdatenbasiert durch die Steuerung der Ausgangsleistung des Photonenstrahlers geregelt wird. Es war überraschend, dass insbesondere diese geregelte Photonenstrahlung vor allem über die oben genannte Zunahme von Produkten der Mitochondrien eine überraschend gute Lösung der erfindungsgemäßen Aufgabe erlaubt, insbesondere bei Trägern bzw. Personen, denen mit Vorrichtungen des Standes der Technik im Hinblick auf die Prävention von Hörschäden und Gleichgewichtstörungen nicht geholfen werden konnte. Völlig überraschend war es, dass so Gehörschäden und Gleichgewichtsstörungen gleichermaßen vorgebeugt werden konnte. Sauerstoff wirkt als ein Elektronenakzeptor in der Elektronentransportkette und wird in diesem Prozess zu Wasser umgewandelt. Ein Teil des metabolisierten Sauerstoffs erzeugt dabei als natürliches Nebenprodukt reaktive Sauerstoffspezies (ROS). ROS sind chemisch aktive Moleküle, welche eine wichtige Rolle in zellulären Signalkaskaden, in der Regulierung des Zellzyklus, der Aktivierung von Enzymen und der Synthese von Nukleinsäure sowie Proteinen spielen. Da die Photonenbestrahlung zu einer Zunahme von Sauerstoff führt, bewirkt sie auch eine Erhöhung der Produktion von ROS. Die ROS aktivieren weiterhin Transkriptionsfaktoren für die Expression verschiedener stimulierender und protektiver Gene. Diese stimulierenden und protektiven Gene regulieren insbesondere die zelluläre Proliferation und Migration sowie die Produktion von Zytokinen und Wachstumsfaktoren. Vorteilhafterweise stimuliert die erfindungsgemäße Photonenbestrahlung diese Prozesse. Weiterhin sind ROS Teil der zellulären Mechanismen, welche zum Absterben und oder zum Verschlechtern des Zustandes der Haarsinneszellen des Innenohres führen. Dies ist insbesondere der Fall, wenn Lärm zu einer Schädigung auditorischer und/oder vestibulärer Strukturen führt. Überraschenderweise wurde festgestellt, dass die protektive Wirkung der Photonenbestrahlung bevorzugt eine starke zweiphasige Abhängigkeit von der Bestrahlungsleistung und/oder Bestrahlungszeit aufweist. Wenn die Bestrahlung mit einer zu geringen Leistung oder aber über einen zu kurzen Zeitraum erfolgt, führt diese nur in geringem Maße zu einer Photostimulation. Wird andererseits das Innenohr mit einer zu hohen Leistung bestrahlt oder erfolgt die Bestrahlung über einen zu langen Zeitraum wird die effektive Photostimulation der Zellen inhibiert und negative Nebenwirkung können zusätzlich eine protektive Wirkung der Photonenbestrahlung hemmen. Die beschriebene zweiphasige Abhängigkeit von der Bestrahlungsleistung und/oder Bestrahlungszeit begründet die Notwendigkeit einer Regelung der Bestrahlungsleistung und/oder Bestrahlungszeit in Abhängigkeit äußerer und/oder innerer Einflüsse um die Photonenstimulation im optimalen Dosisbereich der protektiven Wirkung durchführen zu können. Insbesondere wurde erfindungsgemäß erkannt, dass der Wert der Photonenstrahlleistung für eine optimale protektive Wirkung, von dem Zustand des Innenohres des Trägers oder aber Faktoren, die den Zustand des Innenohres beeinflussen, abhängt. Beispielsweise wurde festgestellt, dass im Fall einer Belastung des Innenohres durch Lärm zelluläre Prozesse ausgelöst werden, die dem Wirkmechanismus der protektiven Wirkung entgegenstehen. So wird durch die Belastung der Haarsinneszellen durch eine Beschallung beispielsweise die zelluläre ATP Produktion gesenkt. Überraschenderweise kann durch eine bevorzugte Erhöhung der Photonenbestrahlung, insbesondere durch die geregelte Photonenstrahlung während der Beschallung dieser Effekt kompensiert werden und eine optimale protektive Wirkung erreicht werden. Weiterhin ist das Anregungsvermögen der Elektronentransportkette insbesondere des transmembranen Komplex Cytochrom-C-Oxidase, durch eine Photonenbestrahlung vermindert bei Haarsinneszellen, die eine geringe Funktionalität aufweisen. Völlig überraschend konnte durch eine Erhöhung der Photonenstrahlleistung bei der Bestrahlung von Haarsinneszellen mit einer verminderten Funktionalität eine optimale protektive Wirkung erzielt werden. Dies führt insbesondere zu einer neuen klinischen Situation. Auch eine präventive Bestrahlung von Patienten wird ermöglicht, welche bereits an Hörschäden oder aber Gleichgewichtsstörungen leiden, insbesondere solchen Krankheiten, die durch eine verminderte Funktionalität der Haarsinneszellen verursacht werden. Dies betrifft insbesondere Patienten mit Schwerhörigkeit, Tinnitus oder auftretendem Schwindel. Für derartige Patienten gab es bisher mit den Mitteln des Standes der Technik keine präventiven Bestrahl ungsmöglichkeiten.

Vorteilhafterweise kann somit erfindungsgemäß insbesondere durch die messdatenbasierte Regelungseinheit die Bestrahlungsstärke und der Bestrahlungszeitraum für eine optimale protektive Wirkung der Photonenbestrahlung auf die auditorischen und/oder vestibulären Strukturen des Innenohres eingestellt werden. Die messdatenbasierte Regelung der Leistung und/oder der Dauer der Photonenbestrahlung des Innenohres führt dadurch zu einer überraschend wirkungsvollen Prävention von Hör- und/oder Gleichgewichtsstörungen, insbesondere solchen, die durch Schädigungen der insbesondere der Haarsinneszellen, der Spiralganglienzellen oder nachgeschalteter Neuronen verursacht werden.

Des Weiteren ermöglicht die messdatendatenbasierte Anpassung der Leistung des Photonenstrahlers insbesondere einen langfristigen Einsatz der Bestrahlungsvorrichtung als Präventionsmittel für Hör- und Gleichgewichtsstörungen, während des gesamten Alltags mit bis zu 24 h täglich. Dies ist möglich da die messdatendatenbasierte Steuerung der Photonenleistung eine Anpassung an die individuellen Bedürfnisse des Trägers sowie an die sich verändernden Umgebungsbedingungen ermöglicht. Die Bestrahlungsleistung kann computergesteuert basierend auf Messdaten für eine optimale, präventive Wirkung zur Vermeidung von Hör- und Gleichgewichtstörungen eingestellt werden. Dadurch wird eine unnötige Belastung des Innenohres durch eine zu starke Photonenbestrahlung vermieden, wie sie bei einer langfristigen Bestrahlung des Innenohres im Rahmen einer präventiven Photonenbestrahlung auftreten kann. Weiterhin wird eine zu geringe Bestrahlung des Innenohres vermieden, welche eine zu geringe präventive Wirkung zur Folge hat.

Eine bevorzugte Verwendung der Bestrahlungsvorrichtung über den gesamten Alltag ist daher mit bis zu 24 h täglich über mehrere Tage, Wochen oder Monate möglich, wobei die Leistung des Photonenstrahlers auf das Hör- und/oder Gleichgewichtsvermögen des Trägers sowie auf Umweltbedingungen, insbesondere auf solchen Umweltbedingungen, die den Hör- oder Gleichgewichtssinn schädigen können, angepasst werden. Die Verwendung der Bestrahlungsvorrichtung über einen langen Zeitraum mit täglich mindestens 10 Stunden über mehrere Tage, Monate oder Jahre mit einer auf den Träger und/oder die Umweltbedingungen angepassten Photonenstrahlleistung führt überraschenderweise zur einer besonders stark erhöhten protektiven Wirkung, welche besonders wirkungsvoll sowohl Hörschädigungen als auch Gleichgewichtsstörungen vorbeugt. Die protektive Wirkung der Photonenbestrahlung betrifft insbesondere den Schutz der Haarsinneszellen des Innenohres vor einer degenerativen Entwicklung. Dies führt vorteilhafterweise dazu, dass die Photonenbestrahlung sowohl Hörschädigungen als auch Gleichgewichtsstörungen vorbeugt. Dabei führte die Möglichkeit der individualisierten Anpassung der Leistung des Photonenstrahles überraschenderweise zu einer überraschend erhöhten Trageakzeptanz für den Träger der Vorrichtung. Die erhöhte Trageakzeptanz führt weiterhin zu einer täglichen Verwendung über einen längeren Zeitraum und erhöht somit die protektive Wirkung der Vorrichtung im Praxistest. Des Weiteren wird durch eine derart angepasste Photonenstrahlleistung die Energieeffizienz der Bestrahlungsvorrichtung optimiert und ein längerer Einsatz von täglich bis zu 24h über mehrere Tage der Bestrahlungsvorrichtung ermöglicht. In Ausführungsformen in denen die Bestrahlungsvorrichtung mit einer Batterie betrieben wird, ist somit insbesondere ein längerer Betrieb ohne Wechsel der Batterien möglich. Zudem kann mit der Bestrahlungsvorrichtung die Bestrahlungsenergie zeitlich optimiert über den Alltag verteilt werden. Insbesondere ist die protektive Wirkung der Photonenstrahlung auf Haarsinneszellen des Innenohres von dem Zustand der Haarsinneszellen sowie von äußeren Faktoren abhängen, welche auf den Zustand der Haarsinneszellen wirken. Die Anpassung der Photonenstrahlung auf den Zustand der Haarsinneszellen und Faktoren, welche den Zustand Haarsinneszellen beeinflussen, ermöglicht eine individualisierte, langfristige Prävention von Hör- und Gleichgewichtsstörungen. Insbesondere, erzielt eine messdatenbasierte Anpassung der Photonenstrahlleistung eine überraschend höhere protektive Wirkung, als eine präventive Photonenbestrahlung mit Vorrichtungen, welche beispielsweise mit einer konstanten Photonenstrahlleistung das Innenohr bestrahlen.

Offenbart wird zudem die Verwendung der erfindungsgemäßen Bestrahlungsvorrichtung zur Bereitstellung eines Mittels zur präventiven Photonenbestrahlung mit den genannten technischen Merkmalen der erfindungsgemäßen Vorrichtung. Insbesondere erfolgt die präventive Photonenbestrahlung automatisiert, so dass bevorzugt kein Arzt für die Bereitstellung des Mittels und/oder die Durchführung der präventiven Photonenbestrahlung nötig ist.

In bestimmten Ausführungsformen betrifft die Erfindung zudem einen Vorrichtung umfassend einen Photonenstrahler mit steuerbarer und/oder regelbarer Ausgangsleistung zur Bestrahlung des Innenohres eines Trägers der Vorrichtung, um Hör- und/oder Gleichgewichtsschäden vorzubeugen. Insbesondere betrifft die Erfindung zudem einen Vorrichtung umfassend einen Photonenstrahler mit steuerbarer und/oder regelbarer Ausgangsleistung zur Bestrahlung des Innenohres eines Trägers der Vorrichtung um Hör - und/oder Gleichgewichtsschäden vorzubeugen in Kombination mit einem Messgerät zur Messung akustischer Signale, einem Messgerät zur Messung evozierter otoakustischer Emissionen und/oder einem Messgerät zur Bestimmung der Veränderung der Körperposition des Trägers der Vorrichtung. Es war völlig überraschend, dass diese Kombinationen zu einer besonders guten Lösung der erfindungsgemäßen Aufgabe führen. Durch die genannten Kombinationen wird ein unerwarteter Erfolg erzielt.

In einer weiteren bevorzugten Ausführungsform umfasst die erfindungsgemäße Bestrahlungsvorrichtung ein Gerät zur Messung akustischer Signale der Umgebung. Es ist bekannt, dass ein erhöhter Umgebungsgeräuschpegel zu einer degenerativen Entwicklung des Hör- und Gleichgewichtsorganes führt. Insbesondere schädigt ein erhöhter Umgebungsgeräuschpegel auditorische und vestibuläre Strukturen im Innenohr umfassend Haarsinneszellen, Spiralganglienzellen oder Neuronen der aufsteigenden Hörbahnen. Untersuchungen haben ergeben, dass die protektive Wirkung der Photonenbestrahlung auf diese Strukturen erhöht wird, wenn die Leistung der Strahlung an die Belastung des Innenohres angepasst wird, d.h. bevorzugt, wenn die Photonenstrahlleistung mit steigender Belastung des Innenohres durch Umgebungslärm erhöht wird. Zur Messung der Umgebungslautstärke umfasst das Gerät zur Messung akustischer Signale bevorzugt einen Schallpegelmesser umfassend bevorzugt ein Messmikrofon, wobei der Schallpegelmesser den Schalldruckpegel der Umgebung bestimmt. Die Messdaten über die akustischen Signale in der Umgebung des Trägers zur Bestimmung der Umgebungslautstärke werden bevorzugt in Form von Parameterwerten über den Schalldruckpegel in der Umgebung an die automatisierte, messdatenbasierte Regelungseinheit übertragen. Basierend auf den Parameterwerten über den Schalldruckpegel kann durch die automatisierte, messdatenbasierte Regelungseinheit somit die Photonenbestrahlung zur Protektion des Innenohres computergesteuert in Echtzeit auf die Belastung des Innenohres angepasst werden. Insbesondere kann die Photonenbestrahlung monoton steigend, aber nicht notwendigerweise stetig steigend, mit steigender Umgebungslautstärke erhöht werden. Vorteilhafterweise ist die protektive Wirkung der Photonenstrahlung auf das Innenohr insbesondere auf die auditorischen und/oder vestibulären Strukturen des Innenohres umfassend Haarsinneszellen, Spiralganglienzellen sowie von Neuronen, besonders hoch ist, wenn die Bestrahlung, während der Belastung des Innenohres durch einen erhöhten Geräuschlärmpegel, erfolgt. Während das Innenohr einer potentiell schädigenden Lärmbeschallung ausgesetzt ist, ist insbesondere eine erhöhte Strahlungsleistung der Photonenstrahlen nötig um eine protektive Wirkung zu erzielen und somit potentiellen Schädigungen des Innenohres insbesondere durch Lärmbeschallung vorzubeugen. Die Erkenntnis, dass es vorteilhaft ist die Photonenbestrahlung zu erhöhen, wenn das Innenohr während der Photonenbestrahlung einer Lärmbelastung ausgesetzt ist, war völlig überraschend. Eine mögliche Erklärung ist, dass die Beschallung Haarsinneszellen belastet und zelluläre Prozesse auslöst, die dem Wirkmechanismus der protektiven Photonenbestrahlung entgegenwirken. Die gleichzeitige Erhöhung der Photonenstrahlleistung während der Lärmbelastung kann diesen Effekt kompensieren.

In einer bevorzugten Ausführungsform wird der Geräuschlärmpegel, welcher auf das Innenohr wirkt durch einen Schalldruckpegel in einem bevorzugten Schallfrequenzbereich von 50 Hz bis 20 000 Hz besonders bevorzugt von 250 Hz bis 8 000 Hz bestimmt. Durch eine Lärmbeschallung im Bereich 50 Hz bis 20 000 Hz insbesondere von 250 Hz bis 8 000 Hz tritt eine verstärkte Schädigung der auditorischen und vestibulären Strukturen des Innenohres auf. Weiterhin haben Experimente ergeben, dass eine leistungsstärkere Photonenbestrahlung des Innenohres vorteilhaft ist, um das Innenohr besonders wirkungsvoll vor Schädigungen durch starke Lärmbelastung zu schützen, wenn das Innenohr einer starken Lärmbelastung durch Beschallung insbesondere in einem Schallfrequenzbereich von 50 Hz bis 20 000 Hz ganz insbesondere von 250 Hz bis 8 000 Hz ausgesetzt ist. Eine Erhöhung der Leistung der protektiven Photonenbestrahlung bei einer Steigerung des in der Umgebung gemessenen Schalldruckpegels in einem Frequenzbereich von insbesondere 50 Hz bis 20 000 Hz ganz insbesondere von 250 Hz bis 8 000 Hz bewirkt somit eine besonders hohe vorbeugende Wirkung. Weiterhin wird der Schalldruckpegel bevorzugt mit Hilfe eines Bewertungsfilters bevorzugt unter dB (A) Gewichtung entsprechend DIN EN 61672-1:2014-07 bestimmt. Die dB (A) Gewichtung oder äquivalente Bewertungsfilter reflektierten besonders gut die biologische Wirkung des Schalles auf die Haarsinneszellen eines Innenohres insbesondere eines menschlichen Innenohres.

Die Quantifizierung der Umgebungslautstärke im Schallfrequenzbereich von 250 Hz bis 8000 Hz und Bestimmung des Schalldruckpegels in diesem Frequenzbereich in dB (A) Gewichtung erlaubt somit ganz insbesondere die Bestimmung eines biologisch relevanten Parameters, welcher die Wirkung des Schalles auf den Zustand der Haarsinneszellen mit besonders hoher medizinischer Relevanz wider spiegelt. Eine Anpassung der Photonenstrahlung erlaubt somit eine besonders effektive Anpassung der Photonenstrahlleistung an die biologische Schallwirkung auf die Haarsinneszellen und den Zustand der Haarsinneszellen. Die Anpassung der Photonenstrahlung an den Umgebungsgeräuschpegel ermöglicht eine individualisierte Präventionsbestrahlung des Innenohres, welche auf das Schädigungspotential durch den Umgebungslärm angepasst ist. Dadurch wird weiterhin insbesondere die Trageakzeptanz des Gerätes erhöht und eine langfristige alltagsbegleitende Präventionsbestrahlung ermöglicht. Überraschenderweise führt dies zu einer deutlich wirkungsvolleren Vorbeugung von Hör- oder Gleichgewichtsschädigungen im Vergleich zu einer präventiven Bestrahlung, deren Bestrahlungsleistung unabhängig vom Umgebungslärm erfolgt.

In einer bevorzugten Ausführungsform umfasst die Bestrahlungsvorrichtung ein Gerät zur Messung von evozierten otoakustischen Emissionen des Innenohres. Geeignete Geräte und Methoden zur Durchführung solcher Messungen sind dem Fachmann bekannt und werden u.a. in Standardbücher wie Robinette RM (Hrsg.), Glattke T. (Hrsg.), Otoacoustic Emission - Clinical Applications, 3. Ausgabe New York: Thieme 2007 offenbart.

Messungen von evozierten otoakustischen Emissionen beruhen auf dem Prinzip, dass Strukturen im Innenohr, insbesondere die äußeren Haarsinneszellen des Innenohres, aktiv akustische Signale aussenden. Diese Signale werden als otoakustische Emissionen bezeichnet. Otoakustische Emissionen können spontan auftreten oder evoziert, d.h. durch akustische Reize angeregt, werden. Das Gerät zur Messung von evozierten otoakustischen Emissionen des Innenohres umfasst daher bevorzugt einen Schallgeber, bevorzugt einen Lautsprecher, welcher akustische Signale erzeugen kann, die geeignet sind, um die Haarsinneszellen des Innenohres anzuregen, sowie ein Messmikrofon, welches die otoakustischen Emissionen des Innenohres aufnehmen kann, somit bevorzugt akustische Signal zwischen -5 dB und 5 dB aufnehmen kann.

Dem Fachmann ist u.a. aus oben genannter Standardliteratur bekannt, wie aus diesen Messdaten Parameter bestimmt werden können, welche Auskunft über den Zustand der Innenohres geben, insbesondere welche den Zustand der äußeren Haarsinneszellen bzw. die Anzahl der äußeren Haarsinneszellen beschreiben. Ein bevorzugter Parameter ist dabei die sogenannte Reproduzierbarkeit. Zur Bestimmung der Reproduzierbarkeit wird eine Mehrzahl akustischer Signale, bevorzugt durch einen Schallgeber oder Lautsprecher, an das Innenohr ausgesandt und für jedes dieser akustischen Signale wird, bevorzugt durch ein Messmikrofon bestimmt, ob eine otoakustische Emission durch das akustische Signal evoziert wurde. Die Reproduzierbarkeit ist gleich dem Verhältnis der Anzahl von detektierten evozierten otoakustischen Emissionen zu der Anzahl der abgesandten akustischen Signale. Werden zum Beispiel insgesamt 100 akustische Signalen ausgesandt und zu diesen 100 Signalen 60 otoakustische Emissionen detektiert, so beträgt die Reproduzierbarkeit 60%. Vorteilhafterweise reflektiert die Reproduzierbarkeit als ein besonders aussagekräftiger Parameter die Funktionalität des Innenohres auf akustische Signale zu reagieren. Die Reproduzierbarkeit ist daher ein besonders relevanter Parameter zur Bestimmung der biologischen Funktionalität des Innenohres, insbesondere des Zustandes oder der Anzahl der Haarsinneszellen.

Die Bestrahlungsvorrichtung umfasst bevorzugt weiterhin eine Datenverarbeitungsvorrichtung, welche aus den Messdaten der evozierten otoakustischen Emissionen, obig beschriebene Parameter automatisch berechnet. Die Messdaten der evozierten otoakustischen Emission und/oder die Parameter, welche aus den Messdaten berechnet werden, werden bevorzugt an die automatisierte, messdatenbasierte Regelungseinheit übertragen. Durch die computergesteuerte Regelungseinheit ist es somit möglich, die Ausgangsleistung der Photonenstrahlung basierend auf, d.h. in Abhängigkeit von den Parametern einzustellen, welche durch die Messung von otoakustischen Emissionen bestimmt wurden und bevorzugt dem Zustand oder der Anzahl der äußeren Haarsinneszellen entsprechen. Unter dem Zustand der Haarsinneszellen wird bevorzugt die Fähigkeit der Haarsinneszellen verstanden auf akustische Signale zu reagieren, d.h. beispielsweise akustische Signale zur Wahrnehmungen eines Hörereignisses an Neuronen weiterzuleiten oder basierend auf den akustischen Signalen otoakustische Emissionen zu generieren. Untersuchungen haben ergeben, dass die Photonenstrahlung zur Prävention von Hör- oder Gleichgewichtsstörungen insbesondere auf die Haarsinneszellen des Innenohres wirken und dort einen protektiven Effekt erzielen. Eine bevorzugte Anpassung der Photonenstrahlung auf den Zustand der Haarsinneszellen oder auf die Anzahl der Haarsinneszellen ermöglicht die protektive Wirkung auf die Haarsinneszellen überraschend zu erhöhen. Insbesondere kann bei einer Abnahme der Anzahl der Haarsinneszellen oder aber einer Verschlechterung des Zustandes der Haarsinneszellen die Bestrahlung der Photonenstrahlung erhöht werden. Eine derartige Anpassung der Leistung der Photonenstrahlung auf den Zustand und/oder die Anzahl der Haarsinneszellen erzielt eine überraschend hohe Protektion des Innenohres und beugt dadurch insbesondere langfristig Hör- und Gleichgewichtsstörungen vor, als dies im Stand der Technik möglich war.

In einer weiteren bevorzugten Ausführungsform umfasst das Gerät zur Messung evozierter otoakustischen Emissionen einen oder mehrere Schallgeber zur Generation akustischer Signale, welche die äußeren Haarzellen des Innenohres zur otoakustischen Emission anregen, und ein Messmikrofon zur Messung dieser otoakustischen Emissionen. In einer besonders bevorzugten Ausführungsform ist das Gerät zur Messung evozierter otoakustischen Emissionen geeignet, um distorsivproduzierte otoakustische Emissionen (DPOAE) zu messen. Dem Fachmann ist u.a. aus oben genannter Standardliteratur über "*Otoacoustic Emission - Clinical Applications*" bekannt, wie DPOAE gemessen werden können. Insbesondere kann das Gerät mit Hilfe eines oder mehrerer Schallgeber zwei simultane Sinustöne einer Frequenz f1 und f2 erzeugen und das Verzerrungsprodukt der otoakustische Emission bei mindestens einer Frequenz f3 mit Hilfe eines Messmikrofons messen. Diese bevorzugte Ausführungsform umfassend ein Gerät zur Durchführung von DPOAE ist besonders gut geeignet, um den Zustand und/oder die Anzahl der Haarsinneszellen zu bestimmen. Insbesondere hat sich gezeigt, dass die Messungen von DPOAE aufgrund der erhöhten Frequenzspezifität in überraschendem und besonderem Maße geeignet sind, um Messungen von otoakustischen Emissionen in Umgebungen mit einem hohen Umgebungsgeräuschpegel vorzunehmen. Insbesondere erlaubt das bevorzugte Gerät zur Messung von DPOAE somit eine Messung des Zustandes und/oder der Anzahl von Haarsinneszellen des Innenohres in verschiedenen Alltagssituation, z.B. in den Wohnräumen, auf Arbeit, beim Konzert oder auf Reisen. Dadurch ist eine Anpassung der Leistung der Photonenstrahlung an die internen auditorischen und/oder vestibulären Strukturen in besonderem Maße während des Alltages mit geringem Aufwand möglich. Die dadurch besonders gute alltagstaugliche Verwendung der Bestrahlungsvorrichtung führt zu einer besonders wirkungsvollen Protektion des Innenohres vor etwaigen Hör- und/oder Gleichgewichtsstörungen.

In einer weiteren bevorzugten Ausführungsform wird das Messmikrofon zur Messung der otoakustischen Emission ebenfalls zur Messung der Umgebungslautstärke eingesetzt. In dieser besonders bevorzugten Ausführungsform ist das Messmikrofon somit eine integraler Bestandteil sowohl des Gerätes zur Messung akustischer Signale aus der Umgebung als auch des Gerätes zur Messung der otoakustischen Emissionen. Diese zweifache Nutzung des Messmikrofones zur Bestimmung von Messdaten verbessert insbesondere den Aufwand für die Herstellung der Bestrahlungsvorrichtung sowie für den Betrieb der Bestrahlungsvorrichtung. Insbesondere ist dadurch eine kostengünstigere Produktion der Bestrahlungsvorrichtung möglich. Weiterhin wird durch die synergetische Nutzung eines Messmikrofones für unterschiedliche Funktionen der Energieaufwand zum Betrieb der Bestrahlungsvorrichtung überraschend vermindert. Die durch das Gerät zur Messung der otoakustischen Emissionen ermöglichte individualisierte Anpassung der Photonenstrahlung auf den Zustand des Innenohres erhöht des Weiteren insbesondere die Trageakzeptanz. Dies lässt sich nach Untersuchungen zumindest teilweise darauf zurückführen, dass ein Träger der Vorrichtung sich bewusst ist, dass die Bestrahlung für sein Innenohr weder zu hoch eingestellt ist und somit eventuell zu unerwünschten Nebenwirkungen führt, noch zu niedrig eingestellt ist und somit keine optimale protektive Wirkung zeigt. Die erhöhte Trageakzeptanz ermöglicht einen langfristigen Einsatz des Präventionsmittels über den gesamten Alltag und kann dadurch eine wirkungsvollere Vorbeugung von Schwerhörigkeit oder Gleichgewichtsstörungen erzielen, als dies im Stand der Technik möglich war.

In einer Vorzugsvariante umfasst die Bestrahlungsvorrichtung ein Gerät zur Messung der Veränderung einer Körperposition des Trägers der Bestrahlungsvorrichtung, welches Messdaten an die Regelungseinheit zur Steuerung der Ausgangsleistung des Photonenstrahlers überträgt. Durch ein solches Messgerät ist es bevorzugt möglich, die protektive Photonenbestrahlung an den Zustand des Gleichgewichtssinnes oder aber an Faktoren, die den Zustand des Gleichgewichtssinnes beeinflussen, anzupassen. Insbesondere wurde eine vorteilhafte protektive Wirkung festgestellt, wenn die Photonenbestrahlung erhöht wird, wenn eine Störung des Gleichgewichtssinnes vorliegt. Eine Störung des Gleichgewichtssinnes manifestiert sich bei Personen insbesondere durch eine erhöhte Veränderung der Körperposition wie sie beispielsweise bei Schwankungen oder Stürzen auftritt. Bei diesen Personen konnte durch eine Erhöhung der Leistung der Photonenstrahlung in Abhängigkeit der Schwere der Gleichgewichtsstörung besonders effektiv einer weiteren Verschlechterung der Gleichgewichtsstörung vorgebeugt werden. Häufig ist eine Gleichgewichtsstörung auf eine verminderte Funktionalität oder Anzahl der Haarsinneszellen im Innenohr insbesondere im Vestibulum zurückzuführen. Eine Steigerung der Photonenstrahlung im Falle von Gleichgewichtsstörungen passt sich daher dem Zustand und/oder der Anzahl der Haarsinneszellen an und erhöht dadurch die protektive Wirkung auf die Haarsinneszellen. Durch eine bevorzugte Erhöhung der Photonenbestrahlung in Abhängigkeit der gemessenen Veränderung der Körperposition, insbesondere einer gemessenen Verringerung der Kontrolle des Körperschwerpunktes, wird somit die protektive Wirkung erhöht.

In einer weiteren bevorzugten Ausführungsform bestimmt das im vorherigen Absatz beschriebene Gerät die Veränderung der Körperposition des Trägers der Bestrahlungsvorrichtung dreidimensional im Raum als Veränderung der Winkelgeschwindigkeit von Vorwärts-, Rückwärts- und Seitwärtsbewegungen des Körperschwerpunktes am Träger. Mehrere Versuche weisen darauf hin, dass eine derartige Bestimmung der Veränderung der Körperposition besonders realistisch die Körperschwankung und/oder die Körperbewegungen beschreiben, die gesteigert auftreten, wenn der Gleichgewichtssinn einer Person gestört ist. Insbesondere dient die Veränderung der Winkelgeschwindigkeit von Vorwärts-, Rückwärts- und Seitwärtsbewegungen des Körperschwerpunktes des Trägers als aussagekräftiger Parameter zur Unterscheidung von willkürlichen Bewegungen und solchen Bewegungen, die durch Gleichgewichtsstörungen verursacht werden.

In einer bevorzugten Ausführungsform wird die Veränderung der Winkelgeschwindigkeit von Vorwärts-, Rückwärts- und Seitwärtsbewegungen des Körperschwerpunktes in 3 Dimensionen dabei von einem 3-Achsen-Gyrometer-Chip bestimmt. Der 3-Achsen-Gyrometer-Chip umfasst dabei bevorzugt drei Gyrometer, welche orthogonal zueinander angeordnet sind. Ein besonders bevorzugter 3-Achsen-Gyrometer-Chip ist der L3G4200D von STMicroelectronics. Gyrometer sind dabei bevorzugt als Geräte zu verstehen, welche Drehbewegungen insbesondere Winkelgeschwindigkeiten der Drehbewegungen messen können. Geeignete Gyrometer sind dem Fachmann bekannt und können kommerziell erworben werden. Insbesondere sind im Stand der Technik Gyrometer bekannt, welche die Corioliskraft, als Trägheitskraft in rotierenden Bezugsystemen, bestimmen. Dabei werden in diesen Gyrometern abhängig von der Veränderung der Corioliskraft während einer Bewegung Kapazitätsveränderungen registriert. Da die Corioliskraft in einer festen Beziehung zur Winkelgeschwindigkeit der Rotation steht, können die gemessenen Kapazitätsveränderungen mithilfe eines Mikroprozessors in numerische Werte der Winkelgeschwindigkeit umgewandelt werden. Ebenso können 3-Achsen-Gyrometer-Chips kommerziell erworben werden. Durch die orthogonale Anordnung von drei Gyrometern in den 3-Achsen-Gyrometer-Chips, kann die Winkelgeschwindigkeit einer Drehung des Gerätes zur Messung der Veränderung einer Körperposition in 3 Dimensionen, d.h. entlang von drei Bezugsachsen, bestimmt werden. Bevorzugt werden die derart bestimmten Winkelgeschwindigkeiten als sogenannte Verschiebungsvektoren in der Form a=(x,y,z) dargestellt. Hierbei kennzeichnen x, y und z jeweils die Werte der Winkelgeschwindigkeiten in Grad pro Sekunde (°/s) in den jeweiligen Raumdimensionen des kartesischen Koordinatensystems, welches durch die Bezugsachsen der Gyrometer aufgespannt wird. Bevorzugt entspricht die z-Bezugsachse dabei der Körperachse des Trägers der Bestrahlungsvorrichtung und der z-Wert des Verschiebungsvektors kennzeichnet bevorzugt Auf- und Abbewegungen des Trägers. Die Winkelgeschwindigkeit entlang der x-Bezugsachse, der x-Wert des Verschiebungsvektors, beschreibt bevorzugt Seitwärtsbewegungen des Träger und die Winkelgeschwindigkeit entlang der y-Bezugsachse, der y-Wert des Verschiebungsvektors, beschreibt bevorzugt Vorwärts- und Rückwärtsbewegungen des Trägers.

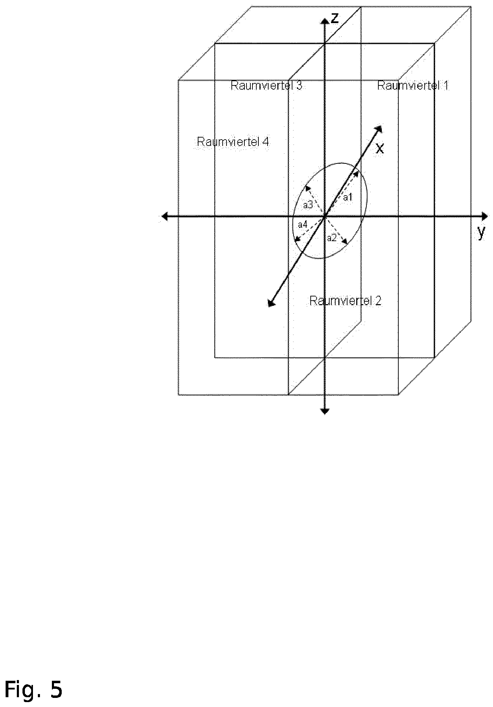

Insbesondere werden bevorzugt Veränderungen der Körperposition des Trägers der Bestrahlungsvorrichtung als Verschiebungsvektoren in der Form a = (x,y,z) für jedes Raumviertel bestimmt (vergleiche Fig. 5).

In einer weiteren Ausführungsform umfasst das Gerät zur Messung der Veränderung einer Körperposition zwei Gyrometer, welche orthogonal zueinander angeordnet sind. Diese beiden Gyrometer bestimmen die Winkelgeschwindigkeit in zwei orthogonalen Bezugsachsen. Bevorzugt bestimmen die beiden Gyrometer die x und y Werte des oben definierten Verschiebungsvektors a=(x,y,z). Der Wert der Winkelgeschwindigkeit z in der dritten Bezugsachse, welche orthogonal zu den anderen beiden Achsen ist, wird in dieser Ausführungsform berechnet. Für die Berechnung können dabei insbesondere folgende mathematische Formeln angewandt werden:

$$\alpha' = \arccos \frac{(b'^2 + c'^2 - a'^2)}{2b'c'}$$

mit

$$a' = \frac{b}{\cos\alpha} - b$$

$$b' = \sqrt{c^2 - b^2}$$

$$c'^2 = a'^2 + b'^2 - 2a'b'\cos\gamma'$$

$$c = a' + b$$

$$\gamma' = 180° - 90° - \alpha$$

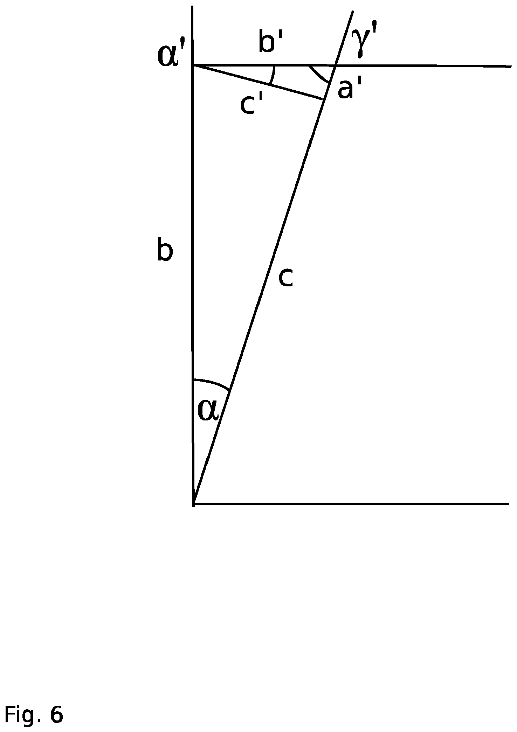

Dabei entspricht wie in Fig. 6 dargestellt der Drehpunkt $\alpha$ dem Winkel entlang der x- oder y-Achse. Für die Berechnung des Winkels entlang der z- Achse ($\alpha'$) mithilfe der trigonometrischen Funktionen wird dieser um eine Entfernung b verlegt. Dies hat jedoch keinen Einfluss auf das Ergebnis der Winkelberechnung. Insbesondere bedeutet dies, dass b für die Berechnung beliebige Werte größer als 0 annehmen kann ohne, dass sich das Ergebnis der Berechnung von α ändert.

Zur Berechnung einer Winkelgeschwindigkeit in °/s entlang der z-Achse aus bekannten Winkelgeschwindigkeiten in der x- und γ- Achse in °/s, werden die berechneten Winkel durch den gemeinsamen Bezug auf die Zeiteinheit von einer Sekunde zu Bewegungsmaßstäben im Sinne der Winkelgeschwindigkeit. Insbesondere wird zur Berechnung einer Winkelgeschwindigkeit entlang der z-Achse aus bekannten Winkelgeschwindigkeiten in der x- und γ- Achse der größere Wert der Winkelgeschwindigkeit entlang der x- und γ-Achse verwandt. In obigen Formeln setzt man diesen Wert der Winkelgeschwindigkeit gleich dem Winkel α unter Bezugnahme auf die Zeiteinheit (s). Die gesuchte Größe des Wertes der Winkelgeschwindigkeit in der dritten Bezugsachse entlang der z-Achse entspricht dem Winkel α unter Bezugnahme auf die Zeiteinheit (s).

Vorteilhafterweise kann durch der Berechnung der Winkelgeschwindigkeit in der dritten orthogonal Bezugsachse (z) aus bekannten Winkelgeschwindigkeiten entlang der x- oder γ-Achse, ein Gyrometer gespart werden. D.h. in dieser Ausführungsform werden statt drei Gyrometern wie bei dem 3-Achsen-Gyrometer nur zwei Gyrometer verwandt. Dies führt insbesondere zu einer leichteren Konstruktion des Gerätes zur Bestimmung der Veränderung der Körperposition und ist besonders kosteneffizient.

Ein Vorteil des Gerätes zur Messung der Veränderung einer Körperposition des Trägers der Bestrahlungsvorrichtung ist es, dass die Veränderungen der Körperposition in Ruhe und in Bewegung im Raum bestimmt werden können.

In einer bevorzugten Ausführungsform wird das Gerät zur Messung der Veränderung der Körperposition in der Mitte des Körpers des Trägers der Bestrahlungsvorrichtung angebracht. In einer besonders bevorzugten Ausführungsform geschieht dies mit Hilfe eines Gürtels, welcher das Gerät zur Messung der Veränderung der Körperposition in der Nähe der Hüfte arretiert. In weiteren bevorzugten Ausführungsformen erfolgt die Befestigung mit einem Gummiband oder mit einem gummifaserhaltigen Textilband erfolgt. Die Befestigung am Körper kann selbstverständlich auch über ein Ledergeschirr, Textilgeschirr oder über ein Synthetikledergeschirr erfolgen.

Vorteilhafterweise entspricht die gemessene Veränderung der Körperposition besonders genau der Veränderung der Position des Körperschwerpunktes des Trägers, wenn eine Positionierung des Messgerätes in der Nähe der Hüfte erfolgt. Insbesondere hat sich gezeigt, dass eine derart bestimmte Veränderung der Körperposition sehr realistisch Schwankungen und ggf. Stürze detektiert. Weiterhin wird durch eine derartige Positionierung des Messgerätes besonders gut eine kontrollierte Bewegung von einer unkontrollierten Bewegung unterschieden, welche bei Trägern der Vorrichtung mit einer Gleichgewichtsstörung verstärkt auftreten.

In einer besonders bevorzugten Variante sind bei dem Gerät zur Messung der Veränderung weiterhin Normwerte für Verschiebungsvektoren während unterschiedlicher Tätigkeiten hinterlegt. Die Normwerte werden dabei durch eine Verwendung des Gerätes zur Messung der Veränderung der Körperposition bei einer Vielzahl von Personen verschiedener Altersgruppen bestimmt, welche den angegebenen Tätigkeiten nachgingen. Insbesondere entsprechen die Normwerte den Maximalwerten von bestimmten Verschiebungsvektoren in den jeweiligen Raumvierteln wie dargestellt in Fig. 5. Die Normwerte für die maximalen Verschiebungsvektoren a1 - a4 für die jeweiligen Raumviertel spannen somit eine Ellipse auf, welche durch die maximalen, räumlichen Verschiebungsvektoren bestimmt ist. Insbesondere wurde mit Hilfe des Gerätes an 100 gesunden Personen (48 Frauen, 52 Männer) altersspezifischen Normwerte für die maximalen räumlichen Verschiebungsvektoren bei konkreten Bewegungsabläufen für jedes Raumviertel (a1-a4, siehe Fig. 5) in der Form a = (x,y,z) erstellt (siehe Tabelle 1). Durch die Hinterlegung von Normwerten für die verschiedensten Tätigkeiten von u.a. Aufstehen, Balancieren, Gehen oder Treppensteigung verfügt das Gerät zur Bestimmung der Veränderung der Körperposition über ein weites Spektrum von Referenzwerten.

Vorteilhafterweise ist es dadurch möglich, die Bewegung des Trägers der Bestrahlungsvorrichtung besonders gut in einer Vielzahl von Alltagsituationen einzuschätzen. Dies erlaubt eine besonders gute Einschätzung, ob die Bewegung einer gewollten Bewegung im Rahmen der Tätigkeit entspricht. Dies ist insbesondere dann der Fall, wenn der gemessene Verschiebungsvektor kleiner ist, als der hinterlegte Normwert des Verschiebungsvektors in dem jeweiligen Raumviertel für die jeweilige Tätigkeit. Eine ungewollte Bewegung ist weiterhin insbesondere kennzeichnend für den Verlust des Gleichgewichtes. Eine solche ungewollte Bewegung wird insbesondere durch ein Überschreiten des gemessen Verschiebungsvektor im Vergleich zu dem Normwert des Verschiebungsvektors in dem jeweiligen Raumviertel detektiert sowie für die jeweiligen Tätigkeiten und Altersgruppen.

In bevorzugten Varianten sind an dem Gerät zur Messung der Veränderung der Körperposition Programme wählbar, welche dem Gerät anzeigen, welche Tätigkeit der Träger der Vorrichtung ausübt oder ausüben will. Die Programmwahl kann dabei bevorzugt manuell erfolgen oder über eine Spracherkennung erfolgen. Weiterhin kann das Gerät die Programme auch selbständig aktivieren, indem beispielsweise Bewegungsabläufe mithilfe der Auswertung von Hirn- oder Muskelaktivitäten erkannt werden.

In einer besonders bevorzugten Ausführungsform werden die gemessenen Verschiebungsvektoren nicht-linear transformiert um die Veränderung der Körperposition des Trägers der Bestrahlungsvorrichtung zu bestimmen. Grundlage dieser besonders bevorzugten Ausführungsform ist die neue Erkenntnis, dass sich bei der Verlagerung des Körperschwerpunktes die Raumachsen nicht linear zu einander verhalten. Insbesondere entspricht eine Bewegung in der x-Achse oder y-Achse nicht in demselben Maße einer ungewollten Bewegung, wie beispielsweise im Falle von Stürzen oder Schwankungen, wie dies für eine Bewegung entlang der z-Achse der Fall ist. Bevorzugt werden daher die gemessenen Verschiebungsvektoren der Form a=(x,y,z) transformiert um die Bewegung der Körperposition zu bestimmen. Hierbei wird die Winkelgeschwindigkeiten in der x-, y- oder z-Achse angepasst, um möglichst realistisch die Veränderung des Körperschwerpunktes zu quantifizieren.

Dazu wird bevorzugt folgendes kompensatorisches Verfahren angewandt: Zunächst wird mit Hilfe mit einer neu entwickelten Formel der Wert f in Abhängigkeit der x und y Werte der räumlichen Verschiebungsvektoren a = (x,y,z) für jedes Raumviertel bestimmt. Falls x>y, so ist f eine Funktion von x. Bevorzugt ist f eine Funktion von x der Form f = a $x^2$. Ist y>x so ist f eine Funktion von y. Bevorzugt ist f für y>x eine Funktion der Form f = a $y^2$.

In einer ganz besonders bevorzugten Form nimmt die Funktion f folgende Form an falls der Wert x des räumlichen Verschiebungsvektors größer ist als der Wert y des räumlichen Verschiebungsvektors, also für x>y ist f:

$$f = \frac{z}{z_{norm}} 0.0017 \cdot x^{1,9462}$$

Falls y>x so ist f:

$$f = \frac{z}{z_{norm}} 0.0017 \cdot y^{1,9462}$$

In der Formel entspricht z dem Wert z des Verschiebungsvektors des betrachteten Raumviertels. Das gilt für x und y entsprechend. Der z-Normwert wird bevorzugt für die jeweilige Tätigkeit (z. B. Stehen in Dunkelheit) und die jeweilige Altersgruppe aus der Tabelle 1 abgelesen. Das Ergebnis (f) wird vom Wert x des Verschiebungsvektor des betrachteten Raumviertels subtrahiert, wenn x>y ist. Es wird vom Wert y subtrahiert, wenn x<y ist. Die derart transformierten Verschiebungsvektoren können auch als effektive Verschiebungsvektoren bezeichnet werden der Form a*=(x*,y*,z*), da sie besonders gut die relevante Veränderung des Körperschwerpunktes des Trägers der Bestrahlungsvorrichtung reflektieren. Insbesondere, sind in den transformierten Verschiebungsvektoren die Winkelgeschwindigkeiten entlang der x- oder y-Bezugsachse (die Werte x* bzw. y*) abgesenkt im Vergleich zu der Winkelgeschwindigkeit entlang der z-Achse (z*=z). Dies reflektiert besonders gut die realen Bedingungen menschlicher Bewegungsabläufe im Raum bzw. während Schwankungen oder Stürzen, durch ungewollte Bewegungen. Durch diese bevorzugte Ausführungsform kann die Leistung der Photonenbestrahlung somit besonders präzise auf den Zustand des Gleichgewichtssinnes des Trägers der Bestrahlungsvorrichtung angepasst werden. Bevorzugt bestimmt das Gerät zur Messung der Veränderung der Körperposition somit die Veränderung Körperposition als einen Verschiebungsvektor der Form a=(x,y,z) mit x,y,z in °/s. Besonders bevorzugt bestimmt das Gerät zur Messung der Veränderung der Körperposition, die Veränderung der Körperposition als einen durch obiges Verfahren transformierten Verschiebungsvektor der Form a* = (x*,y*,z*) wobei x*, y* und z* in °/s vorliegen und die Werte x* und y* durch in dem vorherigen Paragraphen beschriebenes Verfahren angepasst werden.

Eine ungewollte Bewegung wird insbesondere durch ein Überschreiten des gemessen Verschiebungsvektor bevorzugt des durch obiges Verfahren transformierten Verschiebungsvektor im Vergleich zu dem Normwert des maximalen Verschiebungsvektors in dem jeweiligen Raumviertel detektiert. Überraschenderweise hat sich gezeigt, dass für die Detektion von derart ungewollten Bewegungen eine Erhöhung der präventiven Photonenbestrahlung eine besonders protektive Wirkung entfaltet. Vorteilhafterweise wird die Leistung der Photonenbestrahlung dabei insbesondere in Abhängigkeit der Überschreitung der Veränderung der Körperposition von dem entsprechenden Normwert erhöht. Überraschenderweise wird durch eine Erhöhung der Photonenbestrahlung des Innenohres im Falle von starken Veränderungen der Körperposition insbesondere der Gleichgewichtssinn des Trägers gestärkt. Es hat sich gezeigt, dass eine derart angepasste präventive Photonenbestrahlung besonders geeignet ist, um Gleichgewichtsstörungen vorzubeugen. Weiterhin wird durch die Integration des Gerätes zur Messung der Veränderung einer Körperposition mit der Bestrahlungsvorrichtung insbesondere eine Anwendung der Bestrahlungsvorrichtung für Patienten mit Gleichgewichtsstörungen möglich. Dies ermöglicht eine bevorzugte Anwendung der Bestrahlungsvorrichtung für ein ganz neues Patientenkollektiv umfassend Patienten mit Bluthochdruck bedingtem Schwindel, Otolithenfunktionsstörung, Neuritis vestibularis, Bogengangsfunktionsstörungen, Morbis Meniere sowie unterschiedlichen Schwindelsymptomen wie Drehschwindel, Schwankschwindel oder Liftschwindel.

Eine Photonenbestrahlung von Patienten mit Gleichgewichtsstörungen konnte zuvor nicht mit einer optimal eingestellten Photonenstrahlleistung vorgenommen werden. Insbesondere klagten manche Patienten über ein zusätzliches Schwindelgefühl, was nur durch eine Verringerung der Photonenstrahlleistung vermindert werden konnte. Weiterhin wurde jedoch bei Patienten mit einer geringen Photonenstrahlleistung keine Verbesserung des Gleichgewichtsempfindens festgestellt. Überraschenderweise führt eine Erhöhung der Photonenbestrahlung mit Überschreiten der Veränderung der Körperpositionen von den durch die maximalen Verschiebungsvektoren in den jeweiligen Raumviertel hinterlegten Normwerten dazu, dass bei den betreffenden Personen deutlich weniger häufig Körperschwankungen auftreten. Dies konnte anhand der Messung der Verschiebungsvektoren, bzw. der daraus bestimmten Veränderung der Körperposition, nachgewiesen werden. Weiterhin klagten diese Patienten nicht über ein zusätzliches Schwindelgefühl oder Unwohlsein, was darauf schließen lässt, dass die Photonenstrahlleistung durch die beschrieben Anpassung an die Messdaten des Gerätes zu Bestimmung der Veränderung der Körperposition besonders gut eingestellt ist.

In einer besonders bevorzugten Variante umfasst die Bestrahlungsvorrichtung am Körper angebrachte Aktoren, wobei die Aktivität der Aktoren proportional zu der festgestellten Veränderung einer Körperposition ist und innerhalb auf den Bewegungslauf bezogener Grenzen der Werte der Veränderung einer Körperposition die Aktivierung der unterbleibt. Bei dieser Ausführungsform umfasst die Bestrahlungsvorrichtung bevorzugt Aktoren oder Stimulatoren, welche basierend auf den Messdaten des Gerätes zur Bestimmung der Veränderung der Körperposition dem Träger der Vorrichtung ein Signal geben. Die Stärke des elektrischen Widerstandes ist dabei vorteilhafterweise in einer besonderen Ausführungsform der Erfindung so hoch, dass die Aktivität von angeschlossenen Stimulatoren (Aktoren) gleich Null ist. Im Gegensatz dazu werden die Stimulatoren (Aktoren) aktiviert, sobald die gemessenen oder transformierten räumlichen Verschiebungsvektoren die Normwerte der maximalen Verschiebungsvektoren in den jeweiligen Raumvierteln überschreiten. Vorteilhafterweise wird durch die Stimulation bei Überschreitung der Normwerte für die Verschiebungsvektoren der Gleichgewichtssinn geschult. Insbesondere kann durch ein derart gegebenes Feedbacksignal bei auftretenden Schwankungen oder Gleichgewichtsverlusten einer weiteren Verschlechterung des Gleichgewichtssinnes effektiv vorgebeugt werden.

Des Weiteren führt die Integration von Aktoren in die Bestrahlungsvorrichtung zu einer völlig überraschenden Synergiewirkung in Bezug auf die Prävention von Gleichgewichtsstörungen. Es hat sich herausgestellt, dass der Lernerfolg, d.h. die Schulung des Gleichgewichtsinnes, durch den Einsatz von Aktoren bei gleichzeitiger Photonenbestrahlung des Innenohres überraschenderweise eine höhere protektive Wirkung hat als durch eine Summierung der protektiven Wirkungen durch den Einsatz von jeweils nur Aktoren oder des Photonenstrahlers zur Bestrahlung des Innenohres erwartbar wäre. Insbesondere wird durch die Stimulation mit Aktoren, jene Strukturen des Innenohres angeregt, welche den Haarsinneszellen nachgeschaltet sind. Die gesteigerte Aktivität des Innenohres wiederum führt dazu, dass die Photostimulation der bestrahlten Zellen eine besonders hohe protektive Wirkung erzielt.

Bevorzugte Aktoren im Sinne der Erfindung sind Vibrationsaktoren z.B. Unruhemotor 6CH-1201-WL-00, Namiko Corp., Tokyo. Die Drehzahl des Unruhemotors ist hierbei bevorzugt abhängig von der Frequenz der ausgegebenen Impulse. Das Tastverhältnis der ausgegebenen Impulse beträgt in einer bevorzugten Ausführungsform der Erfindung 50%. Wenn dies nicht möglich ist, sollte die Impulsbreite des negativen bzw. positiven Anteils des Impulses 5uS nicht unterschreiten. Der Unruhemotor hat in einer besonders bevorzugten Ausführungsform der Erfindung eine Auflösung von 1,8°. Die Treiberelektronik kann den Motor in diesem Zusammenhang den Motor in Mikroschritttechnologie betreiben. Vorteilhafterweise kann sie so eingestellt sein, dass 64 Impulse die Motorachse um 1,8° weiterbewegen, womit sich die Frequenz (F) des Impulses wie folgt errechnet:

$$F[1/s] = \frac{X[°/s]}{1.8°} \cdot 64$$

, wobei X [°/S] = gewünschte Winkelgeschwindigkeit;

1.8° = Grundauflösung der Schrittweite des Motors

64 = Feinauflösung der Grundauflösung der Schrittweite des Motors ist.

Der Motor ist bevorzugt über einen Zeitraum von etwa einer Sekunde auf die Nenndrehzahl zu aktivieren. Die Frequenz sollte in einer besonders bevorzugten Ausführungsform der Erfindung 25kHz nicht überschreiten.

Weitere bevorzugte Aktoren sind galvanische Stimulatoren, wobei die Stimulatoren durch elektrische Reizung auf der Körperoberfläche, durch elektrische Reizung von motorischen Nerven bzw. der Muskulatur und/oder durch elektrische Reizung von sensorischen Nerven bzw. Sinnesorganen oder Teilen dieser ausgeführt wird.

In einer weiteren bevorzugten Variante ist mindestens ein Aktor eine Lichtquelle, bevorzugt eine Lichtquelle im Blickfeld des Trägers der Bestrahlungsvorrichtung, so dass bei Aktivität der Aktoren der Träger einen Lichtreiz wahrnimmt. Die Intensität des Lichtreizes oder aber die Farbe des Lichtreizes wird dabei in einer bevorzugten Variante, derart gewählt, dass diese eine höhere Signalwirkung aufweist je stärker die bestimmte Veränderung der Körperposition die hinterlegten Normwerten überschreitet.

In einer weiteren bevorzugten Variante ist mindestens ein Aktor ein Schallgeber, so dass bei Aktivität des Aktors der Träger ein akustisches Signal wahrnimmt. In einer besonders bevorzugte Variante wird die Lautstärke und/oder die Frequenz des akustischen Signales gesteigert je stärker die bestimmte Veränderung der Körperposition, von den hinterlegten Normwerten für die jeweiligen Raumviertel sowie Tätigkeiten und Altersgruppen (siehe Tabelle 1) überschreitet.

Weiterhin ermöglicht das Gerät zur Messung der Veränderung der Körperposition aufgrund der Vielzahl der hinterlegten Normwerte eine Anwendung auch jenseits des Medizin- und Rehabereiches. Dies ist bevorzugt durch eine umfassende Programmierbarkeit auch in den Bereichen möglich, wo es um Training des gesunden, noch zu verbessernden Gleichgewichtssinnes geht, wie z. B. bei Balanceübungen im Sport oder beim Training von Para-Troupers beim Militär. In einer bevorzugten Variante dient dabei ein Modulator oder Stellknopf dazu, verschiedene Programme (z. B. 1 bis 5) zu aktivieren, die dann nach Auslegung den Aktor von "sehr leicht" (z. B. bei einem Turner, der eine Gleichgewichtsfähigkeit trainieren will) bis hin zu "sehr schwer" aktivieren (z. B. bei einem Patienten nach einem Schlaganfall, wo Teile des Gleichgewichtszentrums zerstört sind). Vorteilhafterweise weist die Integration des Gerätes zur Messung der Veränderung der Körperposition eine überraschend große Breite der Systemvielfalt auf, da es im Unternormbereich z. B. für den Turner bzw. im Übernormbereich für den schwer erkrankten Patienten eingesetzt werden kann. Ein weiterer, bereits diskutierter Vorteil ist die freie Programmierbarkeit durch Auswahl einzelner Bewegungsprogramme, supportiert durch die Normwerte und die Datenbank gemäß Tabelle 1.

In einer bevorzugten Variante werden weiterhin die hinterlegten Normwerte für die maximalen Verschiebungsvektoren in den jeweiligen Raumvierteln auf den Träger der Bestrahlungsvorrichtung individuell angepasst. Hierzu ist es bevorzugt möglich, dass der Träger der Bestrahlungsvorrichtung einen Input darüber geben kann, welche Bewegungen er als kontrolliert empfindet. Überschreitet beispielsweise eine Veränderung der Körperposition einen hinterlegten Normwert für den maximalen Verschiebungsvektor für die jeweilige Tätigkeit und wird dadurch ein Aktor aktiviert, so kann der Träger durch eine Eingabe kennzeichnen, dass die Bewegung gewollt war. Auf diese Weise erlernt die Bestrahlungsvorrichtung, dass die Veränderung der Körperposition keiner unkontrollierten Bewegung entspricht. Insbesondere, wird aufgrund der Eingabe durch den Träger der überschrittene Normwert durch einen neuen Normwert ersetzt, welcher der Veränderung der Körperposition entspricht, die der Träger als kontrolliert eingestuft hat. Vorteilhafterweise können dadurch die Normwerte für die maximalen Verschiebungsvektoren in den Raumvierteln auf das Gleichgewichtsvermögen des Trägers angepasst werden. Insbesondere, ist dadurch eine optimale Verwendung der Bestrahlungsvorrichtung von Trägern mit einem sehr gut ausgeprägten Gleichgewichtssinn, wie bespielweise eines Turners, genauso möglich wie eine Verwendung von Personen mit starken Gleichgewichtsstörungen, welche zum Beispiel an häufig auftretendem Schwindel leiden. Weiterhin kann durch diese bevorzugte Ausführungsform die Leistung der präventiven Photonenbestrahlung besonders gut an den Gleichgewichtssinn des Trägers angepasst werden. Dadurch wird insbesondere eine besonders wirkungsvolle präventive Photonenbestrahlung für Personen ermöglicht, welche an Gleichgewichtsstörungen leiden. Dies war mit Verfahren oder Vorrichtungen aus dem bekannten Stand der Technik nicht der Fall.

In einer weiteren bevorzugten Ausführungsform ist der Photonenstrahler ein Laser besonders bevorzugt eine Laserdiode, ganz besonders bevorzugt eine Laserdiode umfassend ein Halbleitermaterial. Die bevorzugte Wahl eines Laser oder einer Laserdiode als Strahlungsquelle der Bestrahlungsvorrichtung ermöglicht eine besonders genaue Einstellung der Photonenstrahlleistung über die Ausgangsleistung des Lasers. Insbesondere ist die Ausgangsleistung von Lasern oder Laserdioden über lange sowie über kurze Zeiträume besonders stabil regelbar Somit kann die automatisierte Regelungseinheit der Ausgangsleistung eines Lasers oder einer Laserdiode besonders adaptiv und präzise gesteuert werden. Weiterhin emittieren Laser oder Laserdioden insbesondere Strahlen mit einer geringen Strahldivergenz. Dies führt dazu, dass eine besonders

gute Fokussierung der Photonenstrahlen und somit zielgerichtete Bestrahlung von Regionen oder Teilbereichen des Innenohres möglich ist. Überraschenderweise werden dadurch Nebenwirkungen durch die Absorption von Photonen in anderen Geweberegion zum Beispiel des äußeren Gehörganges vermieden und der Tragekomfort erhöht. Die besonders bevorzugt Wahl von Halbleiter-Laserdioden wirkt sich weiterhin aus mehreren Gründen vorteilhaft auf den Betrieb und die Herstellung der Bestrahlungsvorrichtung aus. Zum einen sind Halbleiter-Laserdioden kostengünstig. Zum anderen sind Halbleiter-Laserdioden aufgrund Ihrer geringen Größe im Vergleich zu anderen Laserarten wie Gaslaser etc. besonders geeignet, um in die Bestrahlungsvorrichtung integriert zu werden.

In einer ganz besonders bevorzugten Ausführungsform ist der Photonenstrahler eine Leuchtdiode ganz besonders bevorzugt eine Leuchtdiode umfassend ein Halbleitermaterial. Überraschenderweise haben Untersuchungen gezeigt, dass die Bestrahlung des Innenohres mit Hilfe von Leuchtdioden eine besonders hohe protektive Wirkung aufweist. Insbesondere ist die protektive Wirkung der von Leuchtdioden emittierten Photonenstrahlung erhöht gegenüber der protektiven Wirkung von Lasern. Dies lässt sich zumindest teilweise darauf zurückführen, dass die Leuchtdioden im Gegensatz zu Lasern eine inkohärente Strahlung abgeben, welche bevorzugt die Schutzmechanismen der Haarsinneszellen aktiviert. Weiterhin ist die Applikation der Photonenstrahlung durch Leuchtdioden in einer besonders gleichmäßigen Verteilung über das Innenohr möglich. Dies führt dazu, dass bei einer besonders hohen Anzahl von Haarsinneszellen eine protektive Wirkung erzielt wird. Vorteilhafterweise ist mit Leuchtdioden weiterhin eine ausgezeichnete Fokussierung der Photonenstrahlen und somit zielgerichtete Bestrahlung von Regionen oder Teilbereichen des Innenohres möglich. Insbesondere können durch die Verwendung von Leuchtdioden Nebenwirkungen vermieden werden, die durch die Bestrahlung anderer Geweberegionen, wie zum Beispiel des äußeren Gehörganges auftreten. Zudem hat sich gezeigt, dass die Leuchtdioden eine sehr geringe Wärmentwicklung auch bei hoher Photonenstrahlleistung aufweisen. Insbesondere wird dadurch der äußere Gehörgang nicht oder nur in geringem Maße erwärmt und der Tragekomfort erhöht. Weiterhin sind Leuchtdioden besonders energieeffizient, da sie elektrische Energie mit einem besonders hohen Wirkungsgrad in Photonenstrahlung umsetzen. Die geringe Wärmeentwicklung der Leuchtdioden selbst bei einer hohen Photonenstrahlleistung erleichtert zu dem die Integration der Leuchtdiode in die Bestrahlungsvorrichtung. Bei Nutzung der Leuchtdioden als Strahlungsquelle ist die Gesamtwärmeentwicklung der Bestrahlungsvorrichtung zudem verringert. Dies minimiert potentielle Nebenwirkungen aufgrund einer betriebsbedingten Temperaturerhöhung der Bestrahlungsvorrichtung und führt zu einem angenehmeren Empfinden beim Träger der Bestrahlungsvorrichtung. Weiterhin ist die geringe Größe der Leuchtdiode besonders vorteilhaft, um sie in die Bestrahlungsvorrichtung zu integrieren, und führt bevorzugt zu einer geringen Größe und Gewicht der gesamten Bestrahlungsvorrichtung. Die durch die Verwendung von Leuchtdioden ermöglichte geringe Größe, Gewicht und Wärmeentwicklung der bevorzugten Bestrahlungsvorrichtung wirkt sich dabei besonders vorteilhaft auf die Trageakzeptanz aus und ermöglicht einen protektiven Einsatz der Bestrahlungsvorrichtung über einen langen Zeitraum.

In einer ganz besonders bevorzugten Ausführungsform liegt die Wellenlänge der Photonenstrahlen zwischen 600 nm und 1200 nm, bevorzugt zwischen 700 nm und 900 nm, besonders bevorzugt bei 790 nm und 820 nm, insbesondere 808 nm. Insbesondere hat sich überraschenderweise gezeigt, dass die protektive Wirkung von Photonenstrahlung auf das Innenohr von der Wellenlänge des Photonenstrahles abhängt. Insbesondere hat sich gezeigt, dass die Bestrahlung des Innenohres mit Licht einer Wellenlänge im Nahinfrarotbereich zwischen 600 nm und 1200 nm, bevorzugt zwischen 700 nm und 900 nm und besonders bevorzugt bei 790 nm und 820 nm, insbesondere bei 808 nm eine besonders hohe protektive Wirkung auf das Innenohr hat und somit der Ausbildung oder Verschlechterung von Schwerhörigkeit und/oder Gleichgewichtsstörungen besonders effektiv vorbeugt. Mit bei 808 nm ist insbesondere auch beispielhaft 807,5 nm, 808,8 nm, 806,9 nm oder auch 808,2 nm gemeint. Gleichermaßen ist mit bei 790 nm insbesondere auch beispielhaft 789,2 nm, 791,2 nm, 788,7 nm oder auch 790,3 nm gemeint. Ebenfalls ist mit bei 820 nm insbesondere auch beispielhaft 821,4 nm, 818,3 nm, 819,1nm oder auch 820,5 nm gemeint.

Die besonders stark vorbeugende Wirkung der Photonenstrahlen gemäß der besonders bevorzugten Bereiche kann insbesondere auf das Absorptionsspektrum von Molekülen zurückgeführt werden, welche an dem Mechanismus zur Protektion der Haarsinneszellen beteiligt sind. Wie eingangs beschrieben, spielt insbesondere Cytochrom-c-Oxidase (CCO) eine zentrale Rolle in dem Mechanismus, auf welchem die protektiven Wirkung der erfindungsgemäßen Bestrahlungsvorrichtung basiert. Insbesondere durch einen Überlapp der genannten bevorzugten Wellenlängenbereichen mit dem Absorptionsspektrum von CCO weist somit die erfindungsgemäße Photonenbestrahlung eine besonders hohe protektive Wirkung auf die Haarsinneszellen auf. Weiterhin hängt die Dispersion bzw. die Streuung der Photonenstrahlung im biologischen Gewebe von der Wellenlänge ab. Untersuchungen haben gezeigt, dass Photonenstrahlung mit einer Wellenlänge zwischen 600 nm und 1200 nm, bevorzugt zwischen 700 nm und 900 nm und besonders bevorzugt bei 808 nm ein besonders geeignetes Streuverhalten im Gewebe des Innenohres aufweisen. Dieses geeignete Streuverhalten führt insbesondere dazu, dass die Photonenstrahlung auch Haarsinneszellen in tieferliegenden Gewebeschichten erreicht. Daraus resultierend kann eine besonders hohe Schutzwirkung erzielt werden und besonders wirkungsvoll Schwerhörigkeit und/oder Gleichgewichtsstörungen vorgebeugt werden, wenn das Innenohr mit der erfindungsgemäßen Bestrahlungsvorrichtung mit Photonen der bevorzugt genannten Wellenlängenbereiche bestrahlt wird.

In besonders bevorzugten Ausführungsformen umfassen die Laserdioden oder die Leuchtdioden ein Halbmaterial ausgewählt aus der Gruppe umfassend

Galliumarsenid (GaAs), Aluminiumgalliumarsenid (AlGaAs), Indiumgalliumarsenid (InGaAs), Galliumarsenidphosphid (GaAsP), Aluminiumgalliumindiumphosphid (AlGaInP), Galliumphosphid (GaP), Galliumarsenidphosphid (GaAsP), Aluminiumgalliumindiumphosphid (AlGaInP) und Galliumarsenidphosphid (GaP). Diese Halbleitermaterialien sind besonders geeignet um Photonenstrahlen im Nahinfrarotbereich zu generieren. Insbesondere sind die Materialien besonders geeignet um mit einem hohen Wirkungsgrad Photonenstrahlen der Wellenlängen von 600 nm und 1200 nm zu erzeugen. Weiterhin sind Laserdioden oder Leuchtdioden hergestellt unter Verwendung dieser Halbleitermaterialien besonders kostengünstig, energieeffizient und erzeugen Photonenstrahlen bei geringer Wärmeentwicklung.

In einer weiteren bevorzugten Ausführungsform kann die automatisierte, messdatenbasierte Regelungseinheit die Ausgangsleistung des Photonenstrahlers in einem Bereich einstellen, der zwischen 0.1 mW und 1000 mW, bevorzugt zwischen 0.5 mW und 300 mW, besonders bevorzugt zwischen 1mW und 120 mW liegt. Vorteilhafterweise ist eine Bestrahlung des Innenohres mit einer Photonenstrahlleistung der genannten kleiner werdenden Bereiche besonders geeignet um Schädigungen der Haarsinneszellen vorzubeugen. Die elektronische Ausführung der automatisierten, messdatenbasierten Regelungseinheit erlaubt daher bevorzugt eine besonders präzise Regelung der Ausgangsleistung des Photonenstrahles in den genannten Bereichen. Durch eine bevorzugte präzise und schnelle Ansteuerung der Ausgangsleistung zwischen 0.1 mW und 1000 mW, bevorzugt zwischen 0.5 mW und 300 mW, besonders bevorzugt zwischen 1mW und 120 mW ist somit eine besonders adaptive Steuerung der Photonenleistung im biologisch relevanten Leistungsbereich möglich. Dies führt zu einer Erhöhung der präventiven Wirkung der Bestrahlungsvorrichtung auf das Innenohr. Insbesondere führt die Bestrahlung des Innenohres mit einer Photonenstrahlleistung im besonders bevorzugten Bereich von 1 mW bis 120 mW in Kombination mit den besonders bevorzugten Wellenlängenbereichen der Photonenstrahlung bei 790 nm, bei 820 nm oder insbesondere bei 808 nm zur einer besonders protektiven Wirkung auf die auditorischen und vestibulären Strukturen des Innenohres. Die dadurch erreichte besonders wirkungsvolle Prävention von Hör- und/oder Gleichgewichtsstörungen ist nicht bekannt aus dem Stand der Technik.

In einer besonders bevorzugten Ausführungsform wird der Photonenstrahl durch ein Photonenstrahlleitungssystem geführt. Das experimentelle Vergleichen verschiedener Ausführungsformen hat dabei gezeigt, dass es besonders vorteilhaft ist, den Photonenstrahler nicht direkt auf das Innenohr strahlen zu lassen, sondern durch ein Photonenstrahlleitungssystem auf das gesamte Innenohr oder auf vorbestimmte Regionen des Innenohres zu leiten. Eine besonders hohe präventive Wirkung konnte dabei erzielt werden, wenn die bevorzugt bestrahlten, vorbestimmten Regionen des Innenohres bevorzugt die Kochlea sowie das Vestibulum, umfassend die Otolithenorgane und Ampullen der Bogengänge, betreffen. Dieses konnte bevorzugt dadurch erreicht werden, dass der Photonenstrahl durch das Photonenstrahlleitsystem derart geleitet wurde, dass der Photonenstrahl beim Bestrahlen einer Region des Innenohres einen bevorzugten Durchmesser von 12-18 mm besonders bevorzugt von 15 mm aufweist. Die genannten, bevorzugten Dimension lassen sich dabei nicht in einfach aus der Anatomie des Innenohres ableiten. Auf Basis der Anatomie würde man insbesondere einen kleineren Durchmesser wählen. D.h., bei den Dimensionen und Eigenschaften der Vorrichtung handelt es sich nicht um eine Auswahl aus gängigen Größen und Eigenschaften, die für den Fachmann nur eine Gestaltungsmöglichkeit sind, die er ohne erfinderisches Zutun vorsehen würde.

Vorteilhafterweise führen jedoch die bevorzugten Durchmesser überraschend zu einer besonders hohen protektiven Wirkung. Dies liegt zu mindestens teilweise an einem unerwarteten Fokussierungseffekt der Photonenstrahlen beim Durchdringen von Gewebeschichten, welche vor den Haarsinneszellen liegen.

In besonders bevorzugten Ausführungsformen umfasst das Photonenstrahlleitungssystem weiter ein System umfassend Linsen und/oder Spiegel, welche den Photonenstrahl bündeln, aufweiten oder kollimieren. Durch dieses System aus Linsen und Spiegeln ist es insbesondere möglich, den Photonenstrahl derart einzustellen, dass er eine vorbestimmte Region des Innenohres bestrahlt und beim Auftreffen auf das Innenohr einen bevorzugten Durchmesser von 12 - 18 mm besonders bevorzugt von 15 mm aufweist. Bei diesen Durchmessern handelt es sich nicht um eine Auswahl aus gängigen Größen und Eigenschaften, die für den Fachmann nur eine Gestaltungsmöglichkeit sind, die er ohne erfinderisches Zutun vorsehen würde. Die dadurch bevorzugt erreichte homogene Bestrahlung der Kochlea und des Vestibulums des Innenohres führt dazu, dass der Photonenstrahl auf überraschend viele Haarsinneszellen eine protektive Wirkung entfaltet.

In einer weiteren bevorzugten Ausführungsform umfasst das Photonenstrahlleitungssystem ein Lichtleitkabel, welches einen bevorzugten Außendurchmesser von 1 bis 8 mm, besonders bevorzugt von 3 mm bis 5 mm umfasst. Das Lichtkabel ist dabei bevorzugt flexibel ausgeführt. Somit kann das Lichtleitkabel den Photonenstrahl von der Strahlungsquelle zum Innenohr leiten. D.h., bei den genannten Dimensionen und Eigenschaften handelt es sich nicht um eine Auswahl aus gängigen Größen und Eigenschaften, die für den Fachmann nur eine Gestaltungsmöglichkeit sind, die er ohne erfinderisches Zutun vorsehen würde. Insbesondere führen die bevorzugten Außenmaße des Lichtleitkabels dazu, dass die Bestrahlungsvorrichtung in einer bevorzugten Ausführungsform in den äußeren Gehörgang eingeführt werden kann und dort einen besonders guten Halt aufweist. Somit führt der Einsatz eines Lichtleitkabels zu einem besonders hohen Tragekomfort für den Träger der Bestrahlungsvorrichtung, welches die Compliance des Trägers und somit den effektiven Bestrahlungszeitraum erhöht. In besonders bevorzugten Ausführungsformen besteht das optische Kabel aus Glasfaser oder polymeren optischen Fasern. Diese Materialien weisen eine besonders hohe Effektivität bei der Weiterleitung der Photonenstrahlung auf und führen somit bevorzugt zu einer energieeffizienten Bestrahlung des Innenohres. Weiterhin führt die Verminderung von Verlusten von Photonenleistung in dem Lichtleitkabel durch den Einsatz von Glasfasern oder polymeren optischen Fasern zu einer verminderten Wärmeentwicklung, welche den Komfort für den Träger der Bestrahlungsvorrichtung überraschend erhöht. Da auch eine geringe Wärmentwicklung beim Träger zu einem Fremdheitsgefühl führt, wird durch die bevorzugte Ausführungsform die Trageakzeptanz deutlich erhöht.

In einer bevorzugten Ausführungsform liegt der Photonenstrahler und/oder das Photonenstrahlleitungssystem in einer Haltevorrichtung umfassend einen stabförmigen Schaft, an dessen einem Ende eine Öffnung und/oder ein Fenster positioniert ist, derart eingebettet vor, dass der Photonenstrahl mindestens teilweise aus der Öffnung oder dem Fenster austritt. In einer besonders bevorzugten Ausführungsform weist der stabförmige Schaft weiterhin einen Durchmesser von 0,5 mm bis mm 1mm und eine Länge von 3 bis 5 mm auf. Die derart bevorzugte Ausgestaltung einer Haltevorrichtung führt dazu, dass die Bestrahlungsvorrichtung besonders leicht in einen äußeren Gehörgang eingeführt werden kann und effektiv das Innenohr bestrahlt. Die bevorzugten genannten Längen und Dimensionen ergeben sich dabei nicht automatisch aus der Anatomie des äußeren Gehörganges des Trägers. Stattdessen sind sie kleiner gewählt, so war es völlig überraschend, dass die bevorzugten Längen und Durchmesser eine besonders sichere Positionierung der Vorrichtung im Ohr auch beim Sport (selbst beim Reiten oder Tauchen) ermöglichen. Durch geometrische Ausgestaltung der Haltevorrichtung ist die Verwendung der Bestrahlungsvorrichtung zur effektiven präventiven Bestrahlung des Innenohres besonders einfach und sicher durchzuführen. Insbesondere sichert die geometrische Ausgestaltung der Haltevorrichtung, und dabei insbesondere die Maße des stabförmigen Schaftes, eine wirkungsvolle Bestrahlung der bevorzugten Regionen des Innenohres umfassend Kochlea und Vestibulum. Dadurch wird insbesondere vermieden, dass das Innenohr nicht ausreichend homogen bestrahlt wird. Weiterhin wird insbesondere vermieden, dass vom Innenohr abweichende Regionen bestrahlt werden und es dadurch zu unerwünschten Nebenwirkungen kommt.

In einer ganz bevorzugten Ausführungsform weist der stabförmige Schaft an einem Ende ein Silikonschirm mit einem Durchmesser von 3 mm bis 15 mm auf. Bei einer Verwendung der Bestrahlungsvorrichtung bei der die Vorrichtung in den äußeren Gehörgang eingeführt wird, führt der Silikonschirm zu einer überraschend stabilen Positionierung der Vorrichtung im äußeren Gehörgang und somit zu einer überraschend genauen Bestrahlung der bevorzugten Region des Innenohres umfassend Kochlea und Vestibulum. Weiterhin weist der Silikonschim eine besonders hohe Biokompatibilität auf und erhöht den Tragekomfort. Somit wird durch den Silikonschirm in besonderem Maße das Tragen der Bestrahlungsvorrichtung über einen langen Zeitraum und eine damit einhergehende langfristige, präventive Bestrahlung, unterstützt. Weiterhin verhindert der Silikonschirm überraschend das Eindringen von Bakterien oder Keimen durch den äußeren Gehörgang und schützt dadurch das Innenohr zusätzlich. Zudem führt der Silikonschirm zu einer besonders dämpfenden Lagerung des Photonenstrahlers, was überraschend zu einer besonderes homogenen Photonenbestrahlung des Innenohres führt, selbst während dynamischer Tätigkeiten wie beim Sport, Tanzen oder Treppensteigen.

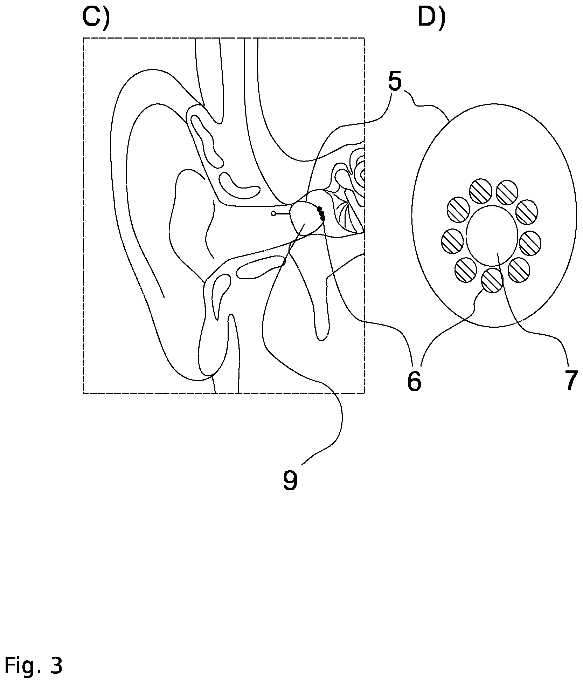

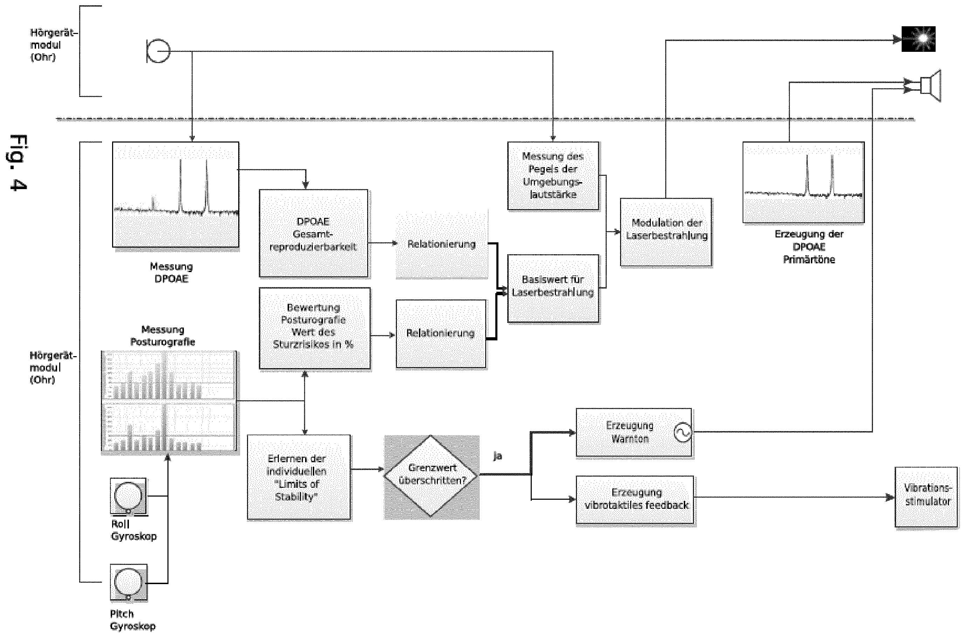

In einer bevorzugten Ausführungsform liegt Bestrahlungsvorrichtung in Kombination mit einem Hörgerät vor. Vorteilhafterweise ist die präventive Photonenbestrahlung besonders sinnvoll bei Personen mit einem bereits geschädigten Hörvermögen, insbesondere bei Patienten mit Schwerhörigkeit. Wie beschrieben kann insbesondere durch die Anpassung der Bestrahlungsleistung an den Zustand des Innenohres, insbesondere an das Hörvermögen des Trägers der Bestrahlungsvorrichtung eine besonders hohe protektive Wirkung erzielt werden. Insbesondere, kann durch die Verwendung der präventiven Bestrahlungsvorrichtung einer weiteren Verschlechterung des Hörvermögens effektiv vorgebeugt werden. Vorteilhafterweise erhöht die Kombination der Bestrahlungsvorrichtung mit einem Hörgerät die Trageakzeptanz der Bestrahlungsvorrichtung. Dadurch erfolgt die präventive Bestrahlung während des gesamten Zeitraums für den das Hörgerät getragen wird, dies betrifft insbesondere den gesamten Alltag mit 10 Stunden oder mehr täglich. Ein Patient mit Schwerhörigkeit ist insbesondere durch ein langjähriges Tragen eines Hörgerätes an ein solches Tragen gewöhnt und empfindet die zusätzliche Integration eines Photonenstrahlers nicht als belastend. Im Gegenteil Probanden tragen eine kombinierte Bestrahlungsvorrichtung mit einem Hörgerät sogar häufiger als nur ein Hörgerät, da sie durch die gleichzeitige präventive Bestrahlung zusätzlich motiviert werden. Vorteilhafterweise erhöht die Kombination der Bestrahlungsvorrichtung mit einem Hörgerät weiterhin den Tragekomfort. Durch eine bevorzugte Integration des Photonenstrahlers in ein Hörgerät muss vorteilhafterweise nur das Hörgerät in den äußeren Gehörgang eingeführt werden. Überraschenderweise kann eine durch eine solche Integration des Photonenstrahlers in ein Hörgerät das Innenohr besonders stabil und zuverlässig bestrahlt werden. In einer bevorzugten Ausführungsform wird dabei die Bestrahlungsvorrichtung mit einem schallverstärkenden Hörgerät bevorzugt mit einem Hinter-dem-Ohr Gerät oder einem Im-Ohr-Gerät kombiniert. Eine bevorzugte Kombination der Bestrahlungsvorrichtung mit einem Hinter-dem-Ohr Gerät ist in Fig. 3 dargestellt. Eine bevorzugte Kombination der Bestrahlungsvorrichtung mit einem Im-Ohr-Gerät Gerät ist in Fig. 4 dargestellt. Überraschenderweise ist die Bestrahlung des Innenohres durch die Kombination der Bestrahlungsvorrichtung mit einem Hinter-dem-Ohr Gerät oder einem Im-Ohr-Gerät besonders vorteilhaft. Durch eine Anordnung des Photonenstrahlers bevorzugt mehrere Leuchtdioden ringförmig am Ende des Hörgerätes wird eine überraschend homogene Bestrahlung des Innenohres ermöglicht. Dadurch wird insbesondere eine protektive Wirkung der Photonenbestrahlung auf besonderes viele Haarsinneszellen ermöglicht. Weiterhin war es völlig überraschend, dass die Integration von Photonenstrahlern in die schallverstärkenden Hörgeräte die Funktion der schallverstärkenden Hörgeräte nicht komprimiert. Weiterhin war es völlig überraschend, dass sich durch die Kombination von der Bestrahlungsvorrichtung mit einem schallverstärkenden Hörgerät funktionelle Synergien ergeben. So kann vorteilhafterweise das Mikrofon des Hörgerätes ebenfalls für die Messung von otoakustischen Emissionen eingesetzt werden. Des Weiteren kann das Messmikrofon des schallverstärkenden Gerätes verwandt werden um die Umgebungslautstärke, insbesondere den Schalldruckpegel in der Umgebung zu bestimmen. Weiterhin kann vorteilhafterweise der Schallgeber des Hörgerätes für die Aussendung von akustischen Signalen zur Anregung von otoakustischen Emissionen verwandt werden. Bevorzugt kann weiterhin der Schallgeber des schallverstärkenden Hörgerätes als Aktor genutzt werden, um dem Träger der Bestrahlungsvorrichtung ein akustisches Warnsignal zu senden, welches darauf hinweist das die Veränderung der Körperposition einen Normwert überschreitet. Es war völlig überraschend, dass eine solche doppelte und sogar dreifache funktionelle Nutzung eines Schallgebers bzw. eines Messmikrofones für die Messung von akustischen Signale, für die Messung von evozierten otoakustischen Emission, für die Schulung des Gleichgewichtsinnes durch Warnsignale sowie für ein schallverstärkendes Hörgerät möglich ist. Die Kombination von einer Bestrahlungsvorrichtung mit einem schallverstärkenden Hörgerät führte somit zu einer überraschend kostengünstigen und energieeffizienten Ausführungsform.

In einer weiteren bevorzugten Ausführungsform ist das Hörgerät ein implantierbares Hörgerät oder ein Kochlea-Implantat. Die Kombination der Bestrahlungsvorrichtung mit einem implantierbaren Hörgerät oder einem Kochlea-Implantat ist dabei besonders vorteilhaft, da dies eine dauerhafte präventive Bestrahlung des Innenohres mit zu 24 h täglich über mehrere Monate oder Jahre ermöglicht. Des Weiteren führt die Kombination der Bestrahlungsvorrichtung mit einem implantierbarem Hörgerät oder einem Kochlea-Implantat zu einer besonders stabilen Einbettung der Bestrahlungsvorrichtung und somit zu einer besonders stabilen Bestrahlung des Innenohres. Weiterhin kann bevorzugt durch die Kombination der Bestrahlungsvorrichtung mit einem implantierbarem Hörgerät oder einem Kochlea-Implantat der Photonenstrahler oder aber das Photonenstrahlleitungsystem besonders nah an die auditorischen und/oder vestibulären Strukturen des Innenohres positioniert werden. Dies führt zu einer besonders erhöhten protektiven Wirkung durch eine erfindungsgemäße Verwendung der Bestrahlungsvorrichtung und beugt überraschend wirkungsvoll langfristig Hör- und/oder Gleichgewichtsschäden vor.

In einer bevorzugten Ausführungsform sind Komponenten der erfindungsgemäßen Bestrahlungsvorrichtung zur funklosen Datenübertragung bevorzugt mit Hilfe der Bluetooth Technologie miteinander verbunden. So sind beispielsweise bevorzugt mindestens zwei Komponenten, ausgewählt aus einer Gruppe umfassend die automatisierte, messdatenbasierte Regelungseinheit zur Steuerung der Ausgangsleistung des Photonenstrahlers, das Gerät zur Messung akustischer Signale der Umgebung, das Gerät zur Messung von evozierten otoakustischen Emissionen des Innenohres, und das Gerät zur Messung der Veränderung der Körperposition des Trägers, mit einander zur funklosen Datenübertragung bevorzugt mit Hilfe der Bluetooth Technologie miteinander verbunden.

Die Erfindung betrifft zudem ein System zur protektiven Bestrahlung eines Innenohres eines Trägers zur Prävention von Hör- und/oder Gleichgewichtstörungen umfassend eine erfindungsgemäße Bestrahlungsvorrichtung, wobei

a) Signale über den Träger der Bestrahlungsvorrichtung und/oder die Umgebung des Trägers gemessen werden,

b) die Ausgangsleistung des Photonenstrahlers (P) zur protektiven Bestrahlung des Innenohres basierend auf diesen Messdaten berechnet wird,

c) die Ausgangsleistung des Photonenstrahlers auf den berechneten Wert P eingestellt wird und die Bestrahlung des Innenohres mit der Photonenstrahlleistung P erfolgt.

Bei dem erfindungsgemäßen System, welches eine Gruppe von miteinander verbundenen bzw. wirkverbundenen Vorrichtungselementen betrifft, wirken mehrere Vorrichtungskomponenten miteinander, um das erfindungsgemäße Ziel zu erreichen. Überrascherderweise kann mit diesem System die erfindungsgemäße Aufgabe besonders gut gelöst werden.

Weiterhin betrifft die Erfindung neben der Vorrichtung, der Verwendung der Vorrichtung, dem System, welches die erfindungsgemäße Vorrichtung zusammen mit anderen Komponenten umfasst auch ein Verfahren zur protektiven Bestrahlung des Innenohres zur Prävention von Hör- und/oder Gleichgewichtstörungen unter Verwendung der erfindungsgemäßen Bestrahlungsvorrichtung umfassend:

a) Messung von Signalen über den Träger der Bestrahlungsvorrichtung und/oder die Umgebung des Trägers.

b) Berechnung der Ausgangsleistung der Photonenstrahlung (P) zur protektiven Bestrahlung des Innenohres basierend auf diesen Messdaten

c) Steuerung der Ausgangsleistung des Photonenstrahlers auf den berechneten Wert P und Bestrahlung des Innenohres mit der Photonenstrahlleistung P.

Technische Merkmale, die für das erfindungsgemäße Verfahren zur protektiven Bestrahlung des Innenohres zur Prävention von Hör- und/oder Gleichgewichtsstörungen offenbart wurden, gelten insbesondere auch für die erfindungsgemäße Strahlungsvorrichtung sowie für das erfindungsgemäße System. Der durchschnittliche Fachmann erkennt, dass vor allem die Merkmale der bevorzugten Ausführungsformen des erfindungsgemäßen Verfahrens auch für die technische Darstellung der erfindungsgemäßen Bestrahlungsvorrichtung und des erfindungsgemäßen Systems herangezogen werden können (z.B. ist im Zusammenhang mit dem erfindungsgemäßen ein bevorzugter Schalldruckpegelgrenzwert von 85 dB offenbart; der Fachmann entnimmt dieser Offenbarung, dass dieser bevorzugte Schalldruckpegelgrenzwert vorteilhafterweise auch bei der erfindungsgemäßen Bestrahlungsvorrichtung und/oder bei dem erfindungsgemäßen System zum Einsatz kommen kann).

D.h., Merkmale des Verfahren zur protektiven Bestrahlung des Innenohres zur Prävention von Hör- und/oder Gleichgewichtstörungen sowie Merkmale der bevorzugten Ausführungsformen dieses Verfahrens kann der durchschnittliche Fachmann ebenfalls für die erfindungsgemäße Bestrahlungsvorrichtung, deren Verwendung sowie für das System zur protektiven Bestrahlung eines Innenohres umsetzen. Bevorzugt weisen Ausführungsformen der erfindungsgemäßen Bestrahlungsvorrichtung insbesondere Merkmale auf, welche für die Durchführung des Verfahrens sowie bevorzugter Ausführungsformen des Verfahrens geeignet sind. Offenbarte technische Merkmale, die im Zusammenhang mit dem Verfahren und den bevorzugten Ausführungsformen offenbart wurden, können somit insbesondere auch bei der erfindungsgemäßen Bestrahlungsvorrichtung sowie dem Vorrichtungssystem, dem erfindungsgemäßen System, zur protektiven Bestrahlung eines Innenohres realisiert werden.

Überraschender Weise kann mit dem Verfahren zur Bestrahlung des Innenohres besonders wirkungsvoll Hörschäden und/oder Gleichgewichtstörungen vorgebeugt werden. Durch die Messung von Signalen über den Träger der Bestrahlungsvorrichtung können insbesondere solche Daten aufgenommen, welche Auskunft geben über Funktionalität des Gehör- oder Gleichgewichtsinnes des Trägers der Bestrahlungsvorrichtung. Diese Daten beschreiben bevorzugt den Zustand der auditorischen und/oder vestibulären Strukturen des Innenohres des Trägers der Bestrahlungsvorrichtung. Dabei umfassen die auditorischen und/oder vestibulären Strukturen insbesondere die äußeren Haarsinneszellen, die Spiralganglienzellen, die innere Haarsinneszellen sowie daran nachgeschaltete Neuronen zur Verarbeitung akustischer Signale und Signale zur Bestimmung der Körperposition. Bevorzugt werden solche Daten dabei durch Messungen von evozierten otoakustischen Emissionen gewonnen. Weiterhin führt die Messung von Signalen über die Umgebung des Trägers dazu, dass in dem Verfahren Daten über externe Faktoren aufgenommen werden, welche den Zustand der auditorischen und/oder vestibulären Strukturen des Trägers beeinflussen. Zu diesen umgebungsbedingten Faktoren (externen Faktoren) gehört insbesondere der Umgebungsgeräuschpegel. Ein lärmbedingter, erhöhter Geräuschpegel führt insbesondere zu einer Verschlechterung des Zustandes des Innenohres, d.h. zu einer Verschlechterung der Funktionalität des Innenohres für den Gehör- und/oder Gleichgewichtssinn. Die Verschlechterung des Zustandes des Innenohres kann dabei insbesondere zurückgeführt werden auf eine degenerative Entwicklung der auditiorischen und/oder vestibulären Strukturen des Innenohres. Bevorzugt wirkt das Verfahren unter Verwendung der Bestrahlungsvorrichtung einer solchen degenerativen Entwicklung entgegen. Durch die Berechnung der Ausgangsleistung des Photonenstrahlung (P) zur protektiven Bestrahlung des Innenohres basierend auf den Messdaten über den Träger der Bestrahlungsvorrichtung und/oder die Umgebung des Trägers und der Steuerung der Ausgangsleistung auf diesen berechneten Wert, wird einer degenerativen Entwicklung des Innenohres besonders wirkungsvoll entgegengewirkt. Überraschender Weise zeigt dabei die Anpassung der Ausgangsleistung an Bedürfnisse des Trägers der Bestrahlungsvorrichtung eine besonders hohe präventive Wirkung im Vergleich zu Präventionsverfahren, die mit einer konstanten Leistung das Innenohr bestrahlen. Bevorzugt können die Verfahrensschritte a) bis c) dabei mehrmals in regelmäßigen Zeitabständen hintereinander ausgeführt werden. Der Bestrahlungszeitraum des Verfahrens kann dabei bevorzugt mehr als 10 Stunden täglich betragen und über mehrere Tage, Wochen, Monate oder Jahre erfolgen. Durch die kontinuierliche Aufnahme von Messdaten und der Anpassung der Photonenstrahlleistung an diese Messdaten ist eine präventive Bestrahlung mit einer optimalen Anpassung an den protektiven Wirkmechanismus von einer Photonenbestrahlung an das Innenohr möglich. Wie eingangs beschrieben, haben Experimente gezeigt, dass die protektive Wirkung einer Photonenbestrahlung des Innenohres kritisch von der Leistung der Photonenbestrahlung abhängt. Insbesondere umfasst der Wirkmechanismus der protektiven Photonenstimulation eine Anregung von transmembranen Komplexen der Atmungskette insbesondere umfassend die Cytochrom-C-Oxidase. Weiterhin weist der protektive Wirkmechanismus der Photonenbestrahlung insbesondere eine zweiphasige Abhängigkeit von der Photonenstrahlleistung auf. Wie Experimente gezeigt haben, führt dies insbesondere dazu, dass eine zu geringe Photonenstrahlleistung nur eine geringe protektive Wirkung erzielt. Weiterhin wird die protektive Wirkung durch eine stetige Erhöhung der Photonenstrahlleistung nicht stetig gesteigert. Stattdessen führt eine zu hohe Photonenstrahlleistung dazu, dass die auditorischen und/oder vestibulären Strukturen durch die zu hohe Photonenstrahlung negativ beeinflusst werden und degenerieren können. Eine zu hohe Photonenstrahlung führt daher nur zu einer geringen protektiven Wirkung oder aber sogar zu Nebenwirkungen. Eine zu hohe Photonenstrahlleistung führt daher überraschend nicht zu einer wirkungsvollen Prävention von Gehör -und/oder Gleichgewichtsstörungen, sondern kann durch das Auftreten von unerwünschten Nebenwirkungen sogar einer minimierten, protektiven Wirkung führen. Überraschenderweise zeigt die Einstellung der Photonenstrahlleistung auf einen optimalen Wert, welcher basierend auf Messdaten berechnet wird, eine besonders hohe protektive Wirkung. Die Einstellung der Photonenstrahlung in dem Verfahren auf einen für eine Prävention von Hör- oder Gleichgewichtsstörungen optimierten Wert lässt sich dabei vorteilhafter Weise durch die automatisierte, messdatenbasierte Regelungseinheit umsetzen.

In einer bevorzugten Variante umfassen die Messdaten evozierte otoakustischen Emissionen der äußeren Haarzellen des Innenohres und aus diesen Messdaten wird mindestens ein erster Parameter A zur Berechnung der Ausgangsleistung der Photonenstrahlung P bestimmt. Vorteilhafterweise reflektiert die Messung von evozierten otoakustischen Emissionen den Zustand der Haarsinneszellen des Innenohres. Dabei weisen Haarsinneszellen eine hohe Funktionalität für die Wahrnehmung von akustischen Signalen und/oder des Gleichgewichts auf, wenn sie sich zu otoakustischen Emissionen anregen. Vorteilhafterweise ist es somit möglich die Ausgangsleistung der Photonenstrahlung auf einen für die präventive Bestrahlung optimalen Wert einzustellen, welcher unter Berücksichtigung des Zustandes der Haarsinneszellen des Innenohres, d.h. der effektiven Funktionalität des Innenohres als Organ für den Gehör- und/oder Gleichgewichtssinn, berechnet wird. Unter anderem aus Standardbüchern wie dem oben genannten *Otoacoustic Emission - Clinical Applications* sind dem Fachmann geeignete Methoden zur Messung von otoakustischen Emissionen und die Berechnung von biologisch relevanten Parametern bekannt. In einer besonders bevorzugten Variante wird in dem Verfahren mit Hilfe von otoakustischen Emissionen der Parameter der Reproduzierbarkeit bestimmt. Dabei werden mehrere akustische Signale an das Innenohr ausgesandt und für jedes dieser akustischen Signale wird bestimmt, ob eine otoakustische Emission durch das akustische Signal evoziert wurde. Die Reproduzierbarkeit berechnet sich aus dem Verhältnis der Anzahl von detektierten, evozierten otoakustischen Emissionen zu der Anzahl der abgesandten akustischen Signale. Werden zum Beispiel insgesamt 100 akustische Signalen ausgesandt und zu diesen 100 Signalen 60 otoakustische Emissionen detektiert, so beträgt die Reproduzierbarkeit 60%. Vorteilhafterweise reflektiert die Reproduzierbarkeit als ein besonders aussagekräftiger Parameter die Funktionalität des Innenohres auf akustische Signale zu reagieren. Die Reproduzierbarkeit ist daher ein besonders relevanter Parameter zur Bestimmung der biologischen Funktion des Innenohres. In einer besonders bevorzugten Variante wird mit Hilfe der otoakustischen Emissionen die Reproduzierbarkeit einmal im Monat bestimmt.

In einer weiteren bevorzugten Variante umfassen die Messdaten akustische Signale aus der Umgebung des Trägers und aus diesen Messedaten wird mindestens ein zweiter Parameter B zur Berechnung der Ausgangsleistung der Photonenstrahlung P bestimmt wird. Durch die Messung von akustischen Signalen aus der Umgebung des Trägers ist es vorteilhafterweise möglich Messdaten zu aufzunehmen, welche Auskunft geben über die Belastung des Innenohres durch eine Beschallung. Bei einer hohen Beschallung insbesondere durch Umgebungslärm beispielsweise durch Baulärm, Motorenlärm oder auch lauter Musik können die auditorischen und vestibulären Strukturen des Innenohres geschädigt werden. Vorteilhafterweise kann in dem Verfahren durch die Anpassung der Photonenstrahlleistung auf den Umgebungslärm, ein optimaler Wert für die Bestrahlungsleistung eingestellt werden. Dadurch wird eine besonders hohe protektive Wirkung auf das Innenohr umfassend insbesondere lärmempfindliche Haarsinneszellen erreicht. Dem Fachmann sind Methoden bekannt mit denen aussagekräftige Parameter über die Umgebungsgeräusche bestimmen werden können. In einer bevorzugten Variante ist der Parameter B, welcher die Umgebungslautstärke, quantifiziert der Schalldruckpegel. In einer besonders bevorzugten Variante wird der Schalldruckpegel in einem bevorzugten Schallfrequenzbereich von 250 Hz bis 8 000 Hz, besonders bevorzugt in dB (A) Gewichtung bestimmt. Der Schalldruckpegel insbesondere in den genannten Frequenzbereichen ist ein besonders gutes Maß für die biologische Belastung, die das Innenohr durch Umgebungsgeräusche erfährt.

In einer weiteren bevorzugten Variante umfassen die Messdaten Veränderungen der Körperposition des Trägers der Bestrahlungsvorrichtung und aus diesen Messedaten wird mindestens ein dritter Parameter C zur Berechnung der Ausgangsleistung der Photonenstrahlung P bestimmt wird. Durch die Anpassung der Photonenstrahlleistung an Messdaten über die Veränderung der Körperposition kann die protektive Photonenbestrahlung an den Zustand des Gleichgewichtssinnes oder aber an Faktoren, die den Zustand des Gleichgewichtssinnes beeinflussen, angepasst werden. Insbesondere wurde eine vorteilhafte protektive Wirkung festgestellt, wenn die Photonenbestrahlung erhöht wird, wenn eine Störung des Gleichgewichtssinnes vorliegt. Eine Störung des Gleichgewichtssinnes manifestiert sich bei Personen insbesondere durch eine erhöhte Veränderung der Körperposition wie sie beispielsweise bei Schwankungen oder Stürzen auftritt. Bevorzugt beschreibt der Parameter C, wie stark die Veränderung der Körperposition Normwerte für gewollte Bewegungen überschreitet. In einer besonders bevorzugten Ausführungsform wird die Veränderung der Körperposition dabei in Abhängigkeit von den räumlichen Verschiebungsvektoren mit Hilfe von Gyrometern bestimmt. Besonders bevorzugt bestimmt der Parameter C die prozentuale Überschreitung der Veränderung der Körperposition im Vergleich zu hinterlegten Normwerten für maximale Verschiebungsvektoren in den Raumviertel für die jeweilige Tätigkeit und die jeweilige Altersgruppe.

In einer bevorzugte Variante berechnet sich die Ausgangsleistung des Photonenstrahles aus der Summierung von drei positiven Summanden, wobei der erste Summand PA mit Hilfe des Parameter A berechnet wird, der zweite Summand PB mit Hilfe des Parameter B berechnet wird, der dritte Summand PC mit Hilfe des Parameter C berechnet wird und falls die Summe aus PA, PB und PC einen Maximalwert M überschreitet P = M ist. Vorteilhafterweise berechnet sich somit die Gesamtbestrahlung anteilig aus Summanden welche auf Parametern basieren, die entweder Auskunft geben über den Zustand des Innenohres oder Faktoren die den Zustand des Innenohres beeinflussen. Hierbei betrifft insbesondere Parameter A, (berechnet aus Messdaten über die otoakustischen Emissionen) die Funktionalität des Innenohres für den Gehörsinn und Parameter C (berechnet aus Messdaten über die Veränderung der Körperposition) die Funktionalität des Innenohres für den Gleichgewichtssinn. Weiterhin beschreibt Parameter B (berechnet aus Messdaten über akustische Umgebungssignale) insbesondere die Belastung des Innenohres durch Umgebungsgeräusche und daher insbesondere eine potentielle Schädigung des Innenohres und Verschlechterung seiner Funktionalität als Organ des Hör- und Gleichgewichtssinnes. In einer bevorzugten Variante überschreitet die Photonenstrahlleistung dabei einen Maximalwert nicht. Hierzu steuert die messdatenbasierte Regelungseinheit die Leistung des Photonenstrahlers, bevorzugt derart, dass die Leistung des Photonenstrahlers einen Maximalwert von bevorzugt 120mW bis 300 mW nicht übersteigt. Ab Erreichen eines Maximalwertes für die Leistung mit der das Innenohr bestrahlt, bevorzugt ab Erreichen eines Maximalwertes für die Leistung mit der das Innenohr bestrahlt, welcher zwischen 120 mW und 300 mW liegt, können Nebenwirkung auftreten durch die Erwärmung und/oder Überdosierung der Photonenstrahlleistung. Durch die Regelung der computergesteuerten Regelungseinheit, so dass ein Maximalwert für die Photonenstrahlung nicht überschritten wird, können in dem Verfahren Nebenwirkungen der protektiven Bestrahlung minimiert und/oder vermieden werden. Dadurch, dass die Leistung der Photonenbestrahlung nicht 300 mW insbesondere nicht 120 mW übersteigt, wird eine ungewollte Erwärmung des Innenohres, eine Überreizung der Haarsinneszellen und andere Nebenwirkung, wozu zudem Schwindel und/oder Hörbeeinträchtigungen gehören, minimiert oder vermieden.

In einer besonders bevorzugten Variante ist der durch die Messung der evozierten otoakustischen Emission bestimmte Parameter A die Reproduzierbarkeit und der Summand der Photonenstrahlleistung PA, welcher auf dem Parameter A basiert, eine monoton fallende Funktion von A. Vorteilhafterweise wird dadurch die Intensität der Photonenbestrahlung des Innenohres erhöht, wenn die Reproduzierbarkeit für die Messung von otoakustischen Emissionen sinkt. Wie zuvor beschrieben, reflektiert die Reproduzierbarkeit insbesondere die Fähigkeit von Haarsinneszellen akustische Signale wahrzunehmen. Es hat sich vorteilhafterweise gezeigt, dass eine besonders hohe präventive Wirkung erzielt werden kann, wenn die Photonenbestrahlung erhöht wird je schlechter der Zustand des Innenohres, d.h. je niedriger die Reproduzierbarkeit ist. In einer besonders bevorzugten Variante ist dabei für A>=59% PA=1mW und für A<59% PA=(60%-A)*1 mW. Durch eine derartige Berechnung wird PA als Basiswert für die Bestrahlung auf 1 mW festgelegt für eine Reproduzierbarkeit von gleich oder mehr als 59%. Ein Wert der Reproduzierbarkeit von größer gleich 59% indiziert einen guten Gehörsinn und eine hohe Funktionalität der Haarsinneszellen. Vorteilhafterweise wird daher für eine solche Reproduzierbarkeit der Basiswert für die Photonenbestrahlung ausschließlich basierend auf den otoakustischen Emissionen auf 1 mW festgelegt. Liegt jedoch eine Reproduzierbarkeit von weniger als 59% vor ist dies ein Hinweis darauf, dass die Funktionalität der Haarsinneszellen beeinträchtigt ist. Dies kann daran liegen, dass der Träger über zu wenig Haarsinneszellen verfügt, da beispielsweise Haarsinneszellen abgestorben sind, oder aber ein Großteil der verbleibenden Haarsinneszellen nur vermindert auf akustische Reize reagieren. In diesem Fall hat sich ein Verfahren zur präventiven Bestrahlung als besonders effektiv erwiesen, welches die anteilige Photonenstrahlleistung PA linear erhöht, wenn die Reproduzierbarkeit sinkt. Überraschenderweise hat sich gezeigt, dass insbesondere eine lineare Steigerung von PA in Abhängigkeit einer fallenden Reproduzierbarkeit A (PA=(60%-A)*1 mW für A<59%) zu einem optimalen Leistungswert für die Photonenbestrahlung führt, welcher besonders gut einer degenerativen Entwicklung der auditorischen oder vestibulären Strukturen des Innenohres entgegenwirkt.

In einer besonders bevorzugten Variante ist der durch die Messung von akustischen Signale aus der Umgebung des Trägers bestimmte zweite Parameter B der Schalldruckpegel und der Summand der Photonenstrahlleistung PB, welcher auf dem Parameter B basiert, eine monoton steigende Funktion von B. Wie in den vorangegangen Paragraphen beschrieben, ist der Schalldruckpegel der Umgebungslautstärke ein besonders geeigneter Parameter, um die potentielle Belastung des Innenohres durch akustische Signale aus der Umgebung zu quantifizieren. Dies ist insbesondere in einer besonders bevorzugten Variante der Fall, bei welcher der Schalldruckpegel in einem Frequenzbereich von 250 Hz bis 8000 Hz in dB (A) Gewichtung bestimmt wird. Wird in der Umgebung durch die Bestrahlungsvorrichtung ein erhöhter Schalldruckpegel gemessen, so wird das Innenohr durch die erhöhte Umgebungslautstärke belastet und die auditiorischen oder vestibulären Strukturen des Innenohres können geschädigt werden. Überraschenderweise hat sich gezeigt, dass eine Erhöhung der Bestrahlungsleistung des Innenohres bei einem in der Umgebung erhöhten Schalldruckpegel besonders effektiv Hörschäden oder Gleichgewichtsschäden vorbeugt. Insbesondere hat sich gezeigt, dass bei Haarsinneszellen, die durch einen erhöhten Schalldruckpegel belastet werden, beispielsweise durch Lärm, Schädigungen vermieden werden können, wenn diese vorher oder gleichzeitig mit Photonen bestrahlt werden. Dies kann zumindest teilweise darauf zurückgeführt werden, dass die protektive Wirkung der Photonenstrahlung, u.a. durch die Anregung der Cytochrom-C-Oxidase wie eingangs beschrieben, optimiert wird, wenn die durch Lärm belasteten Haarsinneszellen mit einer erhöhten Ausgangsleistung des Photonenstrahlers bestrahlt werden. Vorteilhafterweise kann einer Vorbeugung von Schädigungen der audiotorischen oder vestibulären Strukturen des Innenohres durch eine erhöhte Umgebungslautstärke besonders gut vermieden werden, wenn vorher oder gleichzeitig mit der Lärmbelastung das Innenohr mit Photonen bestrahlt wird. Weiterhin führt eine an die Umgebungslautstärke angepasste präventive Bestrahlung zu einer besonders hohen Schutzwirkung. Das bedeutet, dass insbesondere wenn das Innenohr des Trägers über einen längeren Zeitraum von bevorzugt mindestens einer Woche über bevorzugt mindestens 3 Stunden täglich präventiv bestrahlt wird, auch eine spätere starke Lärmbelastung von zum Beispiel über 100 dB nur zu einem sehr geringen Hörschaden führt. Eine an die alltägliche Lärmbelastung angepasste präventive Photonenbestrahlung vermeidet somit insbesondere auch solche Hörschäden, welche durch eine sehr starke spätere Lärmbelastung auftreten würden. Der protektive Mechanismus der Photonenbestrahlung beugt somit nicht nur Hörschäden vor welche durch eine Belastung während der Bestrahlung auftreten, sondern insbesondere auch solchen Hörschäden, die durch spätere Belastungen beispielsweise durch Lärm auftreten können.

In einer bevorzugten Variante ist die anteilige Photonenstrahlleistung PB für Werte des in der Umgebung gemessenen Schalldruckpegels B, welche unterhalb eines Schalldruckpegelgrenzwertes G liegen 0 mW. Der Wert des Schalldruckpegelgrenzwertes beträgt in einer bevorzugten Variante dabei zwischen 75 dB und 95 dB bevorzugt ungefähr 85 dB, wobei ungefähr 85 dB beispielsweise 85,3 dB, 85,9 dB, 83,5 dB, 84,7 dB, 86 dB oder auch 85,0 dB bedeutet. Überraschenderweise haben Untersuchungen gezeigt, dass die protektive Wirkung der Photonenbestrahlung dann besonders hoch ist, wenn das Innenohr durch eine Beschallung belastet wird, welche durch einen Schalldruckpegel gekennzeichnet ist, welcher höher ist als der Schalldruckpegelgrenzwert, insbesondere für die genannten kleiner werdenden Bereiche. Bei einer Schallbelastung des Innenohres unterhalb eines Grenzwertes für den Schalldruckpegel, führt eine Erhöhung der Photonenbestrahlung in Abhängigkeit vom Schalldruckpegel nicht zu einer besonders gesteigerten protektiven Wirkung. Unterhalb des Schalldruckpegelgrenzwertes erfolgt daher bevorzugt eine Bestrahlung mit einer Leistung P=PA+PC. In Bezug auf die Umgebungslautstärke können die leistungsanteiligen Summanden PA und PC daher als Basiswerte verstanden werden. Mit der Basisleistung P=PA+PC wird das Innenohr bestrahlt, wenn der Träger der Bestrahlungsvorrichtung sich in einer Umgebung befindet, in der die Umgebungslautstärke das Innenohr nicht in hohem Maße belastet, d.h. insbesondere wenn der Schalldruckpegel unterhalb des Schalldruckpegelgrenzwertes liegt.

Die Belastung des Innenohres durch den Umgebungsgeräuschpegel ist insbesondere ab einem Grenzschallpegeldruck von 70 dB, ganz insbesondere ab 85 dB erhöht und führt zu Schädigungen am Innenohr, welche stärker ausfallen je höher der Schalldruckpegel ist. Durch eine Leistungssteigerung der protektiven Photonenbestrahlung in Abhängigkeit vom Schalldruckpegel ab dem Grenzwert G können besonders effektiv Schädigungen am Innenohr vermieden.

In einer ganz bevorzugten Ausführungsform wird ab Erreichen eines Schallpegelgrenzwertes die anteilige Leistung des Photonenstrahles PB auf $PB=2^{floor((B-G)/3dB)+1} * (PA+PC) -(PA+PC)$ eingestellt. Floor bezeichnet dabei die Abrundungsfunktion, welche eine reelle Zahl auf die nächst kleinere ganze Zahl abrundet. D.h. insbesondere ist floor von einer rellen Zahl R gleich der ganzen Zahl N, für die gilt N<=R und N+1 >R, z.B. ist floor(3,3) = 3. PA und PC sind, wie in den vorherigen Paragraphen beschrieben, Summanden der Photonenleistung, welche unabhängig vom gemessenen Schalldruckpegelwert sind. Insbesondere ist die Summe aus PA und PC der Wert der Photonenstrahlleistung mit dem das Innenohr bestrahlt wird, wenn die Umgebungslautstärke unterhalb des Schalldruckpegelgrenzwertes G liegt. PA+PC wird somit auch als Basisleistung bezeichnet. Durch die Bestimmung von PB durch $PB=2^{floor((B-G)/3dB)+1} * (PA+PC) - (PA+PC)$ für Werte des Schalldruckpegels B größer gleich dem Schalldruckgrenzpegelwert G, ergibt sich die Gesamtleistung P für B>=G somit zu $P= 2^{floor((B-G)/3dB)+1} * (PA+PC)$. Bei Erreichen des Schalldruckpegelgrenzwertes, d.h. für B=G ist P=2*(PA+PC) und entspricht somit dem doppelten Wert der Basisleistung (PA+PC). Mit jeder weiteren Steigerung der des Schalldruckpegels B um 3 dB wird die Leistung der Photonenbestrahlung verdoppelt. Die Abrundungsfunktion im Exponenten von $P=2^{floor((B-G)/3dB)+1} * (PA+PC)$ führt dabei dazu, dass jeweils bei einem Wert von B der G um eine Differenz von 3 dB oder ganzzahligen Vielfachen davon übersteigt die Photonenstrahlleistung erhöht wird. Vorteilhafterweise wird dadurch eine hohe Volatilität der Photonenstrahlleistung als Folge von Messschwankungen bei der Bestimmung des Schalldruckpegels vermieden. Überraschenderweise hat die beschriebene Vordopplung der Photonenstrahlleistung bei Anstieg des Schalldruckpegels um 3 dB eine besonders hohe protektive Wirkung. In Experimenten hat sich gezeigt, dass eine derartige Steigerung der Leistung des Photonenstrahles zu einer besonders hohen Schutzwirkung auf das Innenohr führt, so dass der erhöhte Umgebungsgeräuschpegel zu keiner oder zu einer deutlich verminderten Schädigung des Innenohres, insbesondere der Haarsinneszellen, der Spiralganglienzellen oder der Neuronen, führt. Die für B>=G als $2^{floor((B-G)/3dB)+1} * (PA+PC)$ eingestellte Photonenstrahlleistung ist dabei optimal angepasst auf die Belastung des Innenohres durch einen erhöhten Umgebungsgeräuschpegel. Insbesondere ermöglicht die beschriebene Anpassung der Photonenstrahlleistung eine individualisierte Präventionsbestrahlung des Innenohres, welche auf das Schädigungspotential durch den Umgebungslärm optimal eingestellt ist. Dadurch wird die Trageakzeptanz des Gerätes erhöht und eine langfristige alltagsbegleitende Präventionsbestrahlung ermöglicht. Überraschenderweise führt dies zu einer deutlich wirkungsvolleren Vorbeugung von Hör- oder Gleichgewichtsschädigungen im Vergleich zu einer präventiven Bestrahlung, deren Bestrahlungsleistung unabhängig vom Umgebungslärm erfolgt.

In einer weiteren ganz besonders bevorzugten Variante wird der Parameter C bevorzugt einmal wöchentlich, einmal monatlich oder einmal halbjährlich durch die Durchführung eines standardisierten Balancetestes bestimmt und berechnet sich aus den Vorwärts-, Rückwärts- und/oder Seitwärtsbewegungen des Trägers der Bestrahlungsvorrichtung bezogen auf alters-, geschlechts- und übungsspezifische Normwerte. Ganz besonders bevorzugt wird C in % gemessen und entspricht dem Standard Balance Deficit Test (SBDT) composite score. Der SBDT ist ein standardisierter Balancetest in dem der Träger der Bestrahlungsvorrichtung verschiedene Übungen durchgeführt. Insbesondere umfasst der Balancetest bevorzugt folgende Übungen:

- Stehen auf zwei Beinen mit offenen Augen
- Stehen auf zwei Beinen mit geschlossenen Augen
- Stehen auf einem Bein mit offenen Augen
- Stehen auf einem Bein mit geschlossenen Augen
- Acht Tandem Schritte (einen Fuß vor den anderen) mit offenen Augen
- Stehen auf zwei Beinen auf Schaumstoff (Höhe 10 cm, Dichte 25 kg / m3) sowohl mit offenen als auch geschlossen Augen
- Stehen auf einem Bein auf Schaumstoff
- Acht Tandem Schritte auf Schaumstoff
- 3 m gehen während der Kopf kreisend bewegt wird

- 3 m gehen während der Kopf im Rhythmus abwechselnd nach links und rechts gedreht wird
- 3 m gehen während mit dem Kopf im Rhythmus genickt wird
- 3 m mit geschlossenen Augen gehen
- Über vier Barrieren gehen, wobei die Barrieren eine Höhe von 26 cm aufweisen und zwischen den Barrieren jeweils ein Abstand von 1 m besteht
- Hinsetzen und aufstehen

Für jede dieser Übungen wird mit Hilfe des Gerätes zur Messung der Veränderung einer Körperposition bestimmt, wie stark die Veränderung der Körperposition von den Normwerten für die jeweilige Altersgruppe abweicht. Der SBDT composite score quantifiziert die Abweichung der Veränderung der Körperposition von den Normwerten und wird wie folgt berechnet:

$$SBDT\ composite\ score = \frac{\left(\sum_i pi + \sum_i ri\right) * 100}{n * 400}$$

Hierbei bezeichnet n die Anzahl der während des Balancetests durchgeführten Übung. Der index i bezeichnet jeweils die i'te Übung und ist somit i=1,2, ... , n.

Weiterhin ist pi gleich dem bei der i'ten Übung gemessenen "pitch sway" geteilt durch den Normwert des "pitch sway" für die i'te Übung bei der entsprechenden Altersgruppe in %, wobei der "pitch sway" die Vorwärts- bzw. Rückwärtsschwankungen des Trägers quantifiziert. Bevorzugt wird der "pitch sway" mit Hilfe des Gerätes zur Messung der Veränderung der Körperposition umfassend ein Gyrometer als Winkelgeschwindigkeit in °/s entlang der y-Bezugsachse bestimmt und entspricht bevorzugt dem medianen y-Wert der Verschiebungsvektoren, welche während der i'ten Übung bestimmt werden. Weiterhin ist hierbei ri gleich dem bei der i'ten Übung gemessenen "roll sway" geteilt durch den Normwert des "roll sway" für die i'te Übung bei der entsprechenden Altersgruppe in %, wobei der "roll sway" die Seitwärtsschwankungen des Trägers quantifiziert. Bevorzugt wird der "roll sway" mit Hilfe des Gerätes zur Messung der Veränderung der Körperposition umfassend ein Gyrometer als Winkelgeschwindigkeit in °/s entlang der x-Bezugsachse bestimmt und entspricht bevorzugt dem medianen x-Wert der Verschiebungsvektoren, welche während der i'ten Übung bestimmt werden.

Entsprechen beispielsweise die Körperschwankungen des Trägers der Vorrichtung den Normwerten für die jeweiligen Übung, d.h. ist pi und ri für alle i'ten Übungen gleich 100 %, so ist der SBDT composite score gleich 50%. Ein SBDT composite score von unter 50% zeigt an, dass die Körperschwankungen der getesteten Person während der Übungen kleiner sind als die Normwerte für die entsprechende Altersgruppe. Eine kleiner SBDT score insbesondere ein SBDT score von kleiner als 50% kennzeichnet somit einen guten Gleichgewichtssinn. Des Weiteren zeigt ein SBDT composite score von über 50% zeigt an, dass die Körperschwankungen der getesteten Person während der Übungen größer sind als die Normwerte für entsprechende Altersgruppe. Ein großer SBDT score insbesondere ein SBDT score von größer als 50% kennzeichnet somit einen verminderten Gleichgewichtssinn.

In einer besonders bevorzugten Variante entspricht C dem SBDT composite score und für C kleiner als 50% ist PC=0 mW und für C größer gleich 50% ist PC=(C-45%)*0,2 mW. Vorteilhafterweise wird in dieser bevorzugten Ausführungsform die Photonenstrahlleistung basierend auf den Messdaten über die Veränderung der Körperposition nicht erhöht, wenn der getestete Träger der Bestrahlungsvorrichtung einen SBDT composite score von 50% oder weniger hat. In diesem Fall verfügt der Träger über einen guten Gleichgewichtssinn und eine optimale protektive Wirkung wird bevorzugt mit einer Photonenstrahlleistung von P=PA+PC erzielt. Für einen SBDT composite score von größer gleich 50% ist PC=(C-45%)*0,2 mW und die Photonenleistung wird somit linear mit dem SBDT composite score erhöht. Für einen SBDT composite score von beispielsweise 55%, somit C=55%, ist PC=2 mW, für C=60% ist PC = 3 mW usw. Vorteilhafterweise führt die lineare Erhöhung der Photonenstrahlleistung bei Personen mit einem erhöhten SBDT composite score, somit einem verminderten Gleichgewichtssinn, zu einer besonders hohen protektiven Wirkung. Durch eine derartige Anpassung der Photonenstrahlleistung an den Gleichgewichtssinn der Träger der Vorrichtung wird überraschenderweise in besonders hohem Maße eine Hör- und/oder Gleichgewichtsstörungen vorgebeugt. Insbesondere kann durch eine Erhöhung der Leistung der Photonenstrahlung in Abhängigkeit der Schwere der Gleichgewichtsstörung besonders effektiv einer weiteren Verschlechterung der Gleichgewichtsstörung vorgebeugt werden. Häufig ist eine Gleichgewichtsstörung auf eine verminderte Funktionalität oder Anzahl der Haarsinneszellen im Innenohr insbesondere im Vestibulum zurückzuführen. Die bevorzugte Steigerung der Photonenstrahlung im Falle von Gleichgewichtsstörungen passt sich daher dem Zustand und/oder der Anzahl der Haarsinneszellen an und erhöht dadurch in besonderem Maße die protektive Wirkung auf die Haarsinneszellen.

Kurzbeschreibung der Zeichnungen:

Weitere Ziele, Merkmale, Vorteile und Anwendungsmöglichkeiten der erfindungsgemäßen Vorrichtung ergeben sich aus der nachfolgenden Beschreibung von Ausführungsbeispielen anhand der Zeichnungen. Dabei bilden alle beschriebenen und/oder bildlich dargestellten Merkmale für sich oder in beliebiger Kombination den Gegenstand der Erfindung, unabhängig der Zusammenfassung in einzelnen Ansprüchen oder deren Rückbeziehung.

In den Zeichnungen zeigen

Fig.1 eine schematische Darstellung einer Ausführungsform der Bestrahlungsvorrichtung und deren beispielhafte Verwendung;

Fig. 2 eine Kombination der Bestrahlungsvorrichtung mit einem Hinter-dem-Ohr Hörgerät;

Fig. 3 eine Kombination der Bestrahlungsvorrichtung mit einem Im-Ohr Hörgerät;

Fig. 4 das Blockschaltbild einer beispielhaften Verwendung der Bestrahlungsvorrichtung unter Steuerung der Leistung der Photonenbestrahlung;

Fig. 5 eine schematische Darstellung der Verschiebungsvektoren;

Fig. 6 eine Skizze für die Berechnung der Winkelgeschwindigkeit in der z-Achse.

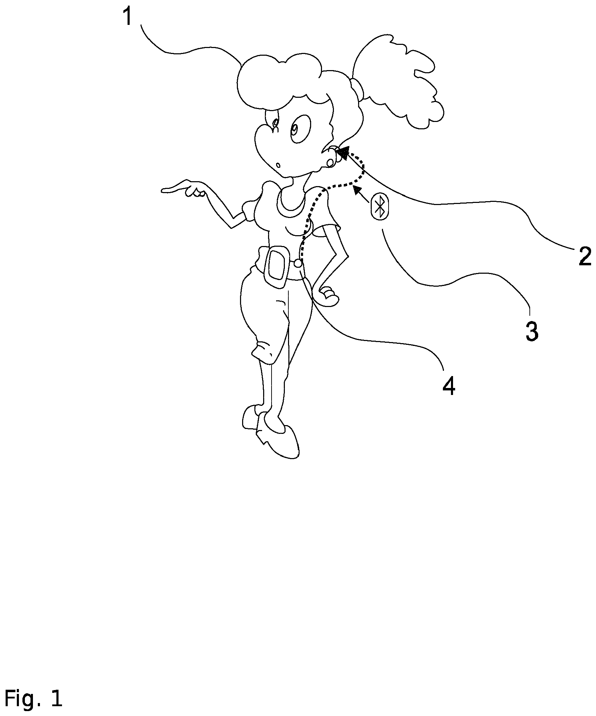

In Fig. 1 ist eine bevorzugte Ausführungsform und beispielhafte Verwendung der Bestrahlungsvorrichtung schematisch dargestellt. An 2 wird das Innenohr des Trägers **1** der Bestrahlungsvorrichtung über den sich im äußeren Gehörgang befindenden Photonenstrahler präventiv mit Nahinfrarotlicht bestrahlt. Die Messung der Umgebungslautstärke sowie die Messung von otoakustischen Emissionen erfolgen ebenfalls an **2**. Messungen der Veränderung der Körperposition des Trägers der Vorrichtung erfolgen mit Hilfe von Gyrometern **4**, welche an der Hüfte des Trägers angebracht sind. Die Messdaten können mit Hilfe der Bluetooth-Technologie **3** kabellos übertragen werden.

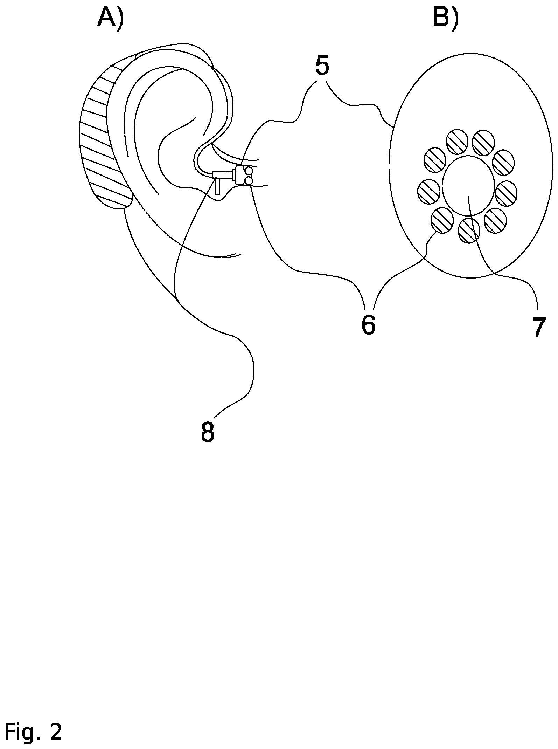

In Fig. 2. ist eine bevorzugte Ausführungsform der Kombination der Bestrahlungsvorrichtung mit einem Hinter-dem-Ohr Hörgerät **8** dargestellt. In A) ist die Kombination der Bestrahlungsvorrichtung mit dem Hinter-dem-Ohr Hörgerät **8** im Querschnitt gezeigt, wie sie bei einem Träger der Vorrichtung zur Verwendung kommt. Die schallverstärkende Einheit des Hörgerätes liegt zusammen mit Leuchtdioden **6** im äußeren Gehörgang des Trägers der Vorrichtung eingeführt vor. Ein Silikonschirm **5** dient der Stabilisation. In B) ist eine Vorderansicht des Silikonschirmes mit den daran befestigten Leuchtdioden **6** gezeigt. Die Leuchtdioden **6** sind ringförmig um die Öffnung **7** angeordnet, durch welche das Hörgerät das Innenohr beschallt.

In Fig. 3 ist eine bevorzugten Ausführungsform der Kombination der Bestrahlungsvorrichtung mit einem Im-Ohr Hörgerät **9** schematisch dargestellt. In C) ist die Kombination der Bestrahlungsvorrichtung mit dem Im-Ohr Hörgerät **9** im Querschnitt gezeigt, wie sie bei einem Träger der Vorrichtung zur Verwendung kommt. Die schallverstärkende Einheit des Hörgerätes liegt zusammen mit Leuchtdioden **6** im äußeren Gehörgang des Trägers der Vorrichtung eingeführt vor. Ein Silikonschirm **5** dient der Stabilisation. In D) ist eine Vorderansicht des Silikonschirmes mit den daran befestigten Leuchtdioden **6** gezeigt. Die Leuchtdioden **6** sind ringförmig um die Öffnung **7** angeordnet, durch welche das Hörgerät das Innenohr beschallt.

In Fig. 4 ist eine bevorzugte Ausführungsform und Verwendung der Bestrahlungsvorrichtung dargestellt. Das Hörgerät-Modul der Bestrahlungsvorrichtung umfasst ein Gerät zur Messung der Reproduzierbarkeit von distorsivproduzierten otoakustischen Emissionen (DPOAE). Weiterhin umfasst die Bestrahlungsvorrichtung ein Hüft-Modul, welches zwei Gyroskope umfasst mit denen eine Posturografie des Trägers der Bestrahlungsvorrichtung durchgeführt wird. Basierend auf den Daten der Posturografie wird das Sturzrisiko des Trägers der Bestrahlungsvorrichtung bestimmt. Sowohl die Gesamtreproduzierbarkeit, welche durch die DPOAE-Messung bestimmt wird, als auch das Sturzrisiko des Trägers der Bestrahlungsvorrichtung dienen der Berechnung eines Basiswertes der Leistung für die Laserbestrahlung des Innenohres mit Nahinfrarotlicht. Weiterhin umfasst das Hörgerät-Modul der Bestrahlungsvorrichtung ein Gerät zur Messung des Pegels der Umgebungslautstärke. Basierend auf dem Wert des Umgebungslautstärkepegels wird die Basisleistung des Lasers derart moduliert, dass eine Nahinfrarot-Bestrahlung des Innenohres mit optimaler, protektiver Wirkung erfolgt.

Mit Hilfe der Gyroskope werden zudem die Veränderungen der Körperposition des Trägers bestimmt und mit Grenzwerten verglichen. Diese Grenzwerte können auf das Alter, das Geschlecht oder die Tätigkeit normiert sein oder von dem Träger individuell angepasst werden. Überschreiten die gemessenen Veränderung der Körperposition, zum Beispiel im Fall von unkontrollierten Körperschwankungen, die Grenzwerte so erfolgt ein akustisches, visuelles, galvanisches und/oder vibrotaktiles Warnsignal an den Träger der Vorrichtung.

In Fig. 5 sind die maximalen Verschiebungsvektoren in jedem Raumviertel des kartesischen Koordinatensystems als gestrichelte Pfeile (a1 bis a4) für eine beispielhafte Veränderung der Körperposition im Raum dargestellt. Durch die maximalen Verschiebungsvektoren für die Bewegung wird eine Ellipse aufgespannt.

In Fig. 6 ist eine schematische Skizze dargestellt mit deren Hilfe die Berechnung der Winkelgeschwindigkeit in der z-Achse ($\alpha$') aus den bestimmten Werten entlang der x- oder y-Bezugsachse erfolgt.

Beispiele:

Im Folgenden soll die Erfindung an Hand von Beispielen näher erläutert werden, ohne auf diese beschränkt zu sein.

In Studien wurden Patienten mit einer peripher bedingten Hör- und Gleichgewichtsstörungen mit der erfindungsmäßen Bestrahlungsvorrichtung versorgt, um der weiteren Abnahme Ihrer Hör- und Gleichgewichtsfunktion entgegenzuwirken. Dazu strahlte eine im Gehörgang liegende Leuchtdiode Licht mit der Wellenlänge von 808 nm und einer Basisbestrahlungsleistung in Richtung der, im Hör- und Gleichgewichtsorgan liegenden, Haarsinneszellen. Die Bestrahlung erfolgte bei den Patienten mit einer Basisleistung solange die Patienten nicht einer schädigenden Umgebungslautstärke ausgesetzt waren. Die Umgebungslautstärke wurde fortlaufend mithilfe eines Mikrofons gemessen. Als nicht schädigende Umgebungslautstärke wurde dabei eine Umgebungslautstärke von weniger als 85 dB eingestuft. Die Höhe der Basisbestrahlungsleistung wurde zum einen monatlich anhand des Umfanges noch vorhandener Haarzellen bestimmt. Dazu führte das Gerät die im Stand der Technik bekannte Messung der otoakustischen Emissionen durch. Wird eine Abnahme der Haarzellantwort, der Reproduzierbarkeit, dabei festgestellt, erhöht das Gerät die Basisbestrahlungsleistung der Bestrahlung in Abhängigkeit vom Haarzellverlust. Bei den betroffenen Patienten wurde eine Reproduzierbarkeit von mehr als 60% festgestellt. PA betrug daher 1 mW. Des Weiteren wurde bei Patienten die Basisleistung in Abhängigkeit durch einen standardisierten Balancetest als SBTD bestimmt (s.o.). Bei den betroffenen Patienten betrug der SBTD score weniger als 50%, daher wurde keine Erhöhung der Basisstrahlleistung vorgenommen, sondern PC betrug 0 mW. Die Basisbestrahlungsleistung betrug bei den Patienten somit 1 mW.

Die meiste Zeit des Tages verbrachten die Patienten in einer nicht schädigenden Umgebungslautstärke. Die Umgebungslautstärke wurde fortlaufend mithilfe eines Mikrofons gemessen. Falls die Umgebungslautstärke in bestimmten Situationen jedoch 85 dB überstieg, wurde bei jeder weiteren Steigerung um 3 dB die Intensität der Bestrahlung verdoppelt. Die Ergebnisse zeigen, dass diese Vorbehandlung den temporären Hörverlust nach einem 3-stündigen ungeschützten Arbeiten mit sehr lauten Motoren (105 dB) um etwa 40 dB verringert und somit die Funktion des Innenohres in erheblichem Maße schützt.

Des Weiteren erhielten Patienten neben der Photonenbestrahlung kurze vibrotaktile Reizungen an der Hüfte in der Richtung, in denen sie zu stark schwankten. Sollte die Patienten kurzzeitig mehr als die doppelte alters- und geschlechtsbezogene Schwankung zeigen, wurde ein kurzer und starker vibrotaktiler oder akustischer Warnhinweis ausgelöst. Die Schwelle für die Auslösung des Warnhinweises wurde von den Patienten durch Ablehnung des Warnhinweises in nicht sturzgefährdeten Situationen an seine Schwankungen im Alltag angepasst, um den Warnhinweis der Sturzprävention vorzubehalten. Diese Individualisierung erfolgt im Gerät durch das Erlernen neuer maximal möglicher Schwankungen, für die der Patient keinen Warnhinweis benötigt.

Das Gerätemodul zur Messung der Veränderung der Körperposition an der Hüfte stand bei dem Patienten in ständigem Funkkontakt durch eine Bluetooth Modul (Fig. 1) mit dem Gerätemodul zur Photonenbestrahlung an oder im Ohr, um die Ergebnisse der Ermittlung der Gleichgewichtsfunktion für die Photonenbestrahlung umzusetzen. In einigen Situationen empfanden Patienten das Tragen beider Module des Gerätes als unkomfortabel. Dann verwandten die Patienten entweder nur das Modul für die Photonenbestrahlung im Gehörgang (z. B. bei Konzertbesuchen) oder das Modul an der Hüfte (z. B. bei Wanderungen). Dies wurde dadurch ermöglicht, dass beide Module unabhängig voneinander einsetzbar sind.

Es wird darauf hingewiesen, dass verschiedene Alternativen zu den beschriebenen Ausführungsformen der Erfindung verwendet werden können, um die Erfindung auszuführen und zu der erfindungsgemäßen Lösung zu gelangen. Die erfindungsgemäße Vorrichtung und das erfindungsmäßige System beschränken sich in ihren Ausführungen somit nicht auf die vorstehenden bevorzugten Ausführungsformen. Vielmehr sind eine Vielzahl von Ausgestaltungsvarianten denkbar, welche von der dargestellten Lösung abweichen können. Ziel der Ansprüche ist es, den Schutzumfang der Erfindung zu definieren. Der Schutzumfang der Ansprüche ist darauf gerichtet, die erfindungsgemäße Vorrichtung und das System sowie äquivalente Ausführungsformen von diesen abzudecken.

Tabelle 1: Normwerte in °/s für 4 maximale räumliche Verschiebungsvektoren a1-a4 in der Form a = (x, y, z) bei konkreten Bewegungsabläufen.

**Altersgruppe: 20-30**

Aufgabe:

| Stehen | a1 = (0,69, 1,36, -0,68) | a2 = (-1,73, 1,36, -0,86) | a3 = (0,69, -1,85, -0,93) | a4 = (-1,73, -1,85, -0,93) |
|---|---|---|---|---|

(fortgesetzt)

**Altersgruppe: 20-30**

| Aufgabe: | | | |
|---|---|---|---|
| Stehen in Dunkelheit | a1 = (0,81, 1,38, -0,69) | a2 = (-1,90, 1,38, -0,95) | a3 = (0,81, -2,29, -1,14) | a4 = (-1,90, -2,29, -1,14) |
| Stehen auf einem Bein | a1 = (3,35, 3,82, -1,91) | a2 = (-4,36, 3,82, -2,18) | a3 = (3,35, -4,92, -2,46) | a4 = (-4,36, -4,92, -2,46) |
| Stehen auf einem Bein in Dunkelheit | a1 = (15,98, 22,54, -11,27) | a2 = (-16,16, 22,54, -11,27) | a3 = (15,98, -20,51, -10,26) | a4 = (-16,16, -20,51, -10,26) |
| Balancieren | a1 = (13,46, 16,68, -8,34) | a2 = (-20,01, 16,68, -10,01) | a3 = (13,46, -25,55, -12,77) | a4 = (-20,01, -25,55, -12,77) |
| Stehen auf weichem Untergrund | a1 = (0,71, 1,83, -0,92) | a2 = (-2,46, 1,83, -1,23) | a3 = (0,71, -3,07, -1,53) | a4 = (-2,46, -3,07, -1,53) |
| Stehen auf weichem Untergrund in Dunkelheit | a1 = (0,94, 1,83, -0,91) | a2 = (-2,39, 1,83, -1,20) | a3 = (0,94, -2,69, -1,35) | a4 = (-2,39, -2,69, -1,35) |
| Stehen auf einem Bein (weicher Untergrund) | a1 = (4,83, 5,95, -2,97) | a2 = (-5,94, 5,95, -2,97) | a3 = (4,83, -6,75, -3,38) | a4 = (-5,94, -6,75, -3,38) |
| Balancieren auf weichem Untergrund | a1 = (17,19, 24,20, -12,10) | a2 = (-25,17, 24,20, -12,59) | a3 = (17,19, -34,65, -17,32) | a4 = (-25,17, -34,65, -17,32) |
| Gehen mit seitlicher Kopfbewegung | a1 = (20,69, 27,20, -13,60) | a2 = (-25,04, 27,20, -13,60) | a3 = (20,69, -27,43, -13,71) | a4 = (-25,04, -27,43, -13,71) |
| Gehen mit vertikaler Kopfbewegung | a1 = (21,41, 27,13, -13,56) | a2 = (-26,20, 27,13, -13,56) | a3 = (21,41, -32,91, -16,45) | a4 = (-26,20, -32,91, -16,45) |
| Gehen in Dunkelheit | a1 = (18,19, 25,36, -12,68) | a2 = (-26,82, 25,36, -13,41) | a3 = (18,19, -33,33, -16,66) | a4 = (-26,82, -33,33, -16,66) |
| Treppensteigen | a1 = (29,06, 33,86, -16,93) | a2 = (-32,96, 33,86, -16,93) | a3 = (29,06, -43,91, -21,95) | a4 = (-32,96, -43,91, -21,95) |
| Gehen über Hindernisse | a1 = (37,81, 49,18, -24,59) | a2 = (-36,01, 49,18, -24,59) | a3 = (37,81, -49,13, -24,57) | a4 = (-36,01, -49,13, -24,57) |
| Gehen | a1 = (21,23, 27,77, -13,88) | a2 = (-30,11, 27,77, -15,06) | a3 = (21,23, -33,94, -16,97) | a4 = (-30,11, -33,94, -16,97) |
| Hinsetzen | a1 = (30,77, 26,80, -15,39) | a2 = (-41,64, 26,80, -20,82) | a3 = (30,77, -41,85, -20,93) | a4 = (-41,64, -41,85, -20,93) |
| Aufstehen | a1 = (53,49, 48,60, -26,75) | a2 = (-29,55, 48,60, -24,30) | a3 = (53,49, -24,24, -26,75) | a4 = (-29,55, -24,24, -14,78) |

**Altersgruppe: 31-40**

| Aufgabe: | | | |
|---|---|---|---|
| Stehen | a1 = (0,92, 1,28, -0,64) | a2 = (-1,75, 1,28, -0,88) | a3 = (0,92, -2,35, -1,17) | a4 = (-1,75, -2,35, -1,17) |
| Stehen in Dunkelheit | a1 = (0,75, 1,14, -0,57) | a2 = (-1,54, 1,14, -0,77) | a3 = (0,75, -2,00, -1,00) | a4 = (-1,54, -2,00, -1,00) |
| Stehen auf einem Bein | a1 = (2,77, 2,95, -1,47) | a2 = (-4,16, 2,95, -2,08) | a3 = (2,77, -3,71, -1,86) | a4 = (-4,16, -3,71, -2,08) |
| Stehen auf einem Bein in Dunkelheit | a1 = (18,34, 20,18, -10,09) | a2 = (-17,27, 20,18, -10,09) | a3 = (18,34, -19,46, -9,73) | a4 = (-17,27, -19,46, -9,73) |
| Balancieren | a1 = (15,30, 16,63, -8,31) | a2 = (-21,55, 16,63, -10,77) | a3 = (15,30, -25,66, -12,83) | a4 = (-21,55, -25,66, -12,83) |
| Stehen auf weichem Untergrund | a1 = (2,57, 3,15, -1,58) | a2 = (-3,62, 3,15, -1,81) | a3 = (2,57, -3,46, -1,73) | a4 = (-3,62, -3,46, -1,81) |
| Stehen auf weichem Untergrund in Dunkelheit | a1 = (0,94, 1,49, -0,75) | a2 = (-2,24, 1,49, -1,12) | a3 = (0,94, -3,25, -1,62) | a4 = (-2,24, -3,25, -1,62) |
| Stehen auf einem Bein (weicher Untergrund) | a1 = (8,86, 7,98, -4,43) | a2 = (-1 0,28, 7,98, -5,14) | a3 = (8,86, -8,01, -4,43) | a4 = (-10,28, -8,01, -5,14) |

(fortgesetzt)

**Altersgruppe: 31-40**

| Aufgabe: | | | |
|---|---|---|---|
| Balancieren auf weichem Untergrund | a1 = (18,30, 23,97, -11,99) | a2 = (-25,94, 23,97, -12,97) | a3 = (18,30, -32,33, -16,16) | a4 = (-25,94, -32,33, -16,16) |
| Gehen mit seitlicher Kopfbewegung | a1 = (21,60, 26,38, -13,19) | a2 = (-25,29, 26,38, -13,19) | a3 = (21,60, -32,72, -16,36) | a4 = (-25,29, -32,72, -16,36) |
| Gehen mit vertikaler Kopfbewegung | a1 = (21,88, 23,20, -11,60) | a2 = (-21,79, 23,20, -11,60) | a3 = (21,88, -28,50, -14,25) | a4 = (-21,79, -28,50, -14,25) |
| Gehen in Dunkelheit | a1 = (16,03, 21,38, -10,69) | a2 = (-23,40, 21,38, -11,70) | a3 = (16,03, -28,18, -14,09) | a4 = (-23,40, -28,18, -14,09) |
| Treppensteigen | a1 = (24,62, 39,98, -19,99) | a2 = (-30,64, 39,98, -19,99) | a3 = (24,62, -41,38, -20,69) | a4 = (-30,64, -41,38, -20,69) |
| Gehen über Hindernisse | a1 = (33,25, 55,01, -27,50) | a2 = (-32,23, 55,01, -27,50) | a3 = (33,25, -55,35, -27,67) | a4 = (-32,23, -55,35, -27,67) |
| Gehen | a1 = (19,95, 25,43, -12,72) | a2 = (-27,39, 25,43, -13,70) | a3 = (19,95, -31,01, -15,51) | a4 = (-27,39, -31,01, -15,51) |
| Hinsetzen | a1 = (37,80, 35,72, -18,90) | a2 = (-40,22, 35,72, -20,11) | a3 = (37,80, -43,72, -21,86) | a4 = (-40,22, -43,72, -21,86) |
| Aufstehen | a1 = (50,08, 49,36, -25,04) | a2 = (-29,87, 49,36, -24,68) | a3 = (50,08, -31,24, -25,04) | a4 = (-29,87, -31,24, -15,62) |

**Altersgruppe: 41-50**

| Aufgabe: | | | |
|---|---|---|---|
| Stehen | a1 = (0,69, 1,08, -0,54) | a2 = (-1,78, 1,08, -0,89) | a3 = (0,69, -2,26, -1,13) | a4 = (-1,78, -2,26, -1,13) |
| Stehen in Dunkelheit | a1 = (0,65, 1,02, -0,51) | a2 = (-1,88, 1,02, -0,94) | a3 = (0,65, -2,43, -1,22) | a4 = (-1,88, -2,43, -1,22) |
| Stehen auf einem Bein | a1 = (6,67, 7,64, -3,82) | a2 = (-7,12, 7,64, -3,82) | a3 = (6,67, -6,76, -3,38) | a4 = (-7,12, -6,76, -3,56) |
| Stehen auf einem Bein in Dunkelheit | a1 = (26,21, 31,79, -15,90) | a2 = (-25,62, 31,79, -15,90) | a3 = (26,21, -33,98, -16,99) | a4 = (-25,62, -33,98, -16,99) |
| Balancieren | a1 = (14,75, 19,55, -9,77) | a2 = (-20,77, 19,55, -10,38) | a3 = (14,75, -32,58, -16,29) | a4 = (-20,77, -32,58, -16,29) |
| Stehen auf weichem Untergrund | a1 = (1,53, 2,09, -1,05) | a2 = (-2,94, 2,09, -1,47) | a3 = (1,53, -3,53, -1,77) | a4 = (-2,94, -3,53, -1,77) |
| Stehen auf weichem Untergrund in Dunkelheit | a1 = (1,24, 146, -0,73) | a2 = (-3,01, 1,46, -1,50) | a3 = (1,24, -3,65, -1,82) | a4 = (-3,01, -3,65, -1,82) |
| Stehen auf einem Bein (weicher Untergrund) | a1 = (11,88, 13,09, -6,55) | a2 = (-11,16, 13,09, -6,55) | a3 = (11,88, -13,13, -6,57) | a4 = (-11,16, -13,13, -6,57) |
| Balancieren auf weichem Untergrund | a1 = (20,97, 24,87, -12,43) | a2 = (-26,00, 24,87, -13,00) | a3 = (20,97, -36,75, -18,37) | a4 = (-26,00, -36,75, -18,37) |
| Gehen mit seitlicher Kopfbewegung | a1 = (18,87, 29,07, -14,53) | a2 = (-26,50, 29,07, -14,53) | a3 = (18,87, -30,44, -15,22) | a4 = (-26,50, -30,44, -15,22) |
| Gehen mit vertikaler Kopfbewegung | a1 = (17,83, 22,81, -11,41) | a2 = (-24,71, 22,81, -12,35) | a3 = (17,83, -29,32, -14,66) | a4 = (-24,71, -29,32, -14,66) |
| Gehen in Dunkelheit | a1 = (16,21, 21,97, -10,98) | a2 = (-23,39, 21,97, -11,69) | a3 = (16,21, -29,72, -14,86) | a4 = (-23,39, -29,72, -14,86) |
| Treppensteigen | a1 = (23,21, 40,15, -20,07) | a2 = (-30,01, 40,15, -20,07) | a3 = (23,21, -46,22, -23,11) | a4 = (-30,01, -46,22, -23,11) |
| Gehen über Hindernisse | a1 = (41,71, 52,30, -26,15) | a2 = (-35,03, 52,30, -26,15) | a3 = (41,71, -53,46, -26,73) | a4 = (-35,03, -53,46, -26,73) |
| Gehen | a1 = (17,78, 26,08, -13,04) | a2 = (-27,76, 26,08, -13,88) | a3 = (17,78, -32,92, -16,46) | a4 = (-27,76, -32,92, -16,46) |

(fortgesetzt)

**Altersgruppe: 41-50**

Aufgabe:

| | | | |
|---|---|---|---|
| Hinsetzen | a1 = (38,42, 31,36, -19,21) | a2 = (-46,14, 31,36, -23,07) | a3 = (38,42, -45,03, -22,51) | a4 = (-46,14, -45,03, -23,07) |
| Aufstehen | a1 = (54,58, 46,19, -27,29) | a2 = (-33,15, 46,19, -23,09) | a3 = (54,58, -28,31, -27,29) | a4 = (-33,15, -28,31, -16,58) |

**Altersgruppe: 51-60**

Aufgabe:

| | | | |
|---|---|---|---|
| Stehen | a1 = (1,31, 1,18, -0,66) | a2 = (-1,82, 1,18, -0,91) | a3 = (1,31, -2,83, -1,42) | a4 = (-1,82, -2,83, -1,42) |
| Stehen in Dunkelheit | a1 = (0,90, 1,10, -0,55) | a2 = (-1,83, 1,10, -0,92) | a3 = (0,90, -2,53, -1,26) | a4 = (-1,83, -2,53, -1,26) |
| Stehen auf einem Bein | a1 = (3,83, 5,00, -2,50) | a2 = (-4,28, 5,00, -2,50) | a3 = (3,83, -5,76, -2,88) | a4 = (-4,28, -5,76, -2,88) |
| Stehen auf einem Bein in Dunkelheit | a1 = (23,80, 35,06, -17,53) | a2 = (-24,60, 35,06, -17,53) | a3 = (23,80, -30,47, -15,23) | a4 = (-24,60, -30,47, -15,23) |
| Balancieren | a1 = (15,06, 18,79, -9,39) | a2 = (-19,62, 18,79, -9,81) | a3 = (15,06, -27,10, -13,55) | a4 = (-19,62, -27,10, -13,55) |
| Stehen auf weichem Untergrund | a1 = (1,60, 1,58, -0,80) | a2 = (-3,23, 1,58, -1,61) | a3 = (1,60, -3,75, -1,87) | a4 = (-3,23, -3,75, -1,87) |
| Stehen auf weichem Untergrund in Dunkelheit | a1 = (1,56, 1,92, -0,96) | a2 = (-2,76, 1,92, -1,38) | a3 = (1,56, -3,34, -1,67) | a4 = (-2,76, -3,34, -1,67) |
| Stehen auf einem Bein (weicher Untergrund) | a1 = (15,44, 17,67, -8,83) | a2 = (-16,37, 17,67, -8,83) | a3 = (15,44, -16,27, -8,13) | a4 = (-16,37, -16,27, -8,18) |
| Balancieren auf weichem Untergrund | a1 = (24,61, 26,51, -13,25) | a2 = (-22,83, 26,51, -13,25) | a3 = (24,61, -29,69, -14,84) | a4 = (-22,83, -29,69, -14,84) |
| Gehen mit seitlicher Kopfbewegung | a1 = (22,53, 31,92, -15,96) | a2 = (-24,79, 31,92, -15,96) | a3 = (22,53, -31,95, -15,97) | a4 = (-24,79, -31,95, -15,97) |
| Gehen mit vertikaler Kopfbewegung | a1 = (16,52, 22,94, -11,47) | a2 = (-21,48, 22,94, -11,47) | a3 = (16,52, -24,11, -12,06) | a4 = (-21,48, -24,11, -12,06) |
| Gehen in Dunkelheit | a1 = (16,14, 20,72, -10,36) | a2 = (-20,44, 20,72, -10,36) | a3 = (16,14, -23,97, -11,98) | a4 = (-20,44, -23,97, -11,98) |
| Treppensteigen | a1 = (26,53, 44,67, -22,34) | a2 = (-27,79, 44,67, -22,34) | a3 = (26,53, -39,80, -19,90) | a4 = (-27,79, -39,80, -19,90) |
| Gehen über Hindernisse | a1 = (43,24, 71,09, -35,55) | a2 = (-34,88, 71,09, -35,55) | a3 = (43,24, -61,64, -30,82) | a4 = (-34,88, -61,64, -30,82) |
| Gehen | a1 = (19,36, 28,46, -14,23) | a2 = (-25,80, 28,46, -14,23) | a3 = (19,36, -29,91, -14,95) | a4 = (-25,80, -29,91, -14,95) |
| Hinsetzen | a1 = (44,06, 42,20, -22,03) | a2 = (-40,96, 42,20, -21,10) | a3 = (44,06, -46,81, -23,41) | a4 = (-40,96, -46,81, -23,41) |
| Aufstehen | a1 = (51,90, 55,69, -27,84) | a2 = (-35,93, 55,69, -27,84) | a3 = (51,90, -38,22, -25,95) | a4 = (-35,93, -38,22, -19,11) |

**Patentansprüche**

1. Bestrahlungsvorrichtung zur Prävention von Hör- und/oder Gleichgewichtstörungen umfassend einen Photonenstrahler zur Bestrahlung eines Innenohres mit einer Wellenlänge zwischen 600 nm und 1200 nm, bevorzugt zwischen 700 nm und 900 nm, ein Gerät zur Messung akustischer Signale der Umgebung, welches den Schalldruckpegel bestimmt, und eine automatisierte Regelungseinheit,

    **dadurch gekennzeichnet, dass**
    die automatisierte Regelungseinheit die Ausgangsleistung des Photonenstrahlers basierend auf dem gemessenen Schalldruckpegel in einem Bereich zwischen 0,1 mW und 1000 mW steuert, wobei die Ausgangsleistung des Photonenstrahlers bis zu einem Schalldruckpegelgrenzwert eine konstante Funktion vom Schalldruckpegel ist und ab dem Schalldruckpegelgrenzwert eine monoton steigende Funktion des Schalldruckpegels ist.

2. Bestrahlungsvorrichtung zur Prävention von Hör- und/oder Gleichgewichtstörungen nach dem vorherigen Anspruch **dadurch gekennzeichnet, dass** der Schalldruckpegelgrenzwert zwischen 75 dB und 95 dB liegt.

3. Bestrahlungsvorrichtung zur Prävention von Hör- und/oder Gleichgewichtstörungen nach einem der vorherigen Ansprüche **dadurch gekennzeichnet, dass** die automatisierte Regelungseinheit die Ausgangsleistung des Photonenstrahlers in einem Bereich zwischen 0,5 mW und 300 mW steuert, wobei ein Maximalwert von 300 mW nicht überschritten wird.

4. Bestrahlungsvorrichtung nach einem der vorherigen Ansprüche **dadurch gekennzeichnet, dass** das Gerät zur Messung akustischer Signale der Umgebung den Schalldruckpegel im einem bevorzugten Schallfrequenzbereich von 50 Hz bis 20000 Hz besonders bevorzugt von 250 Hz bis 8000 Hz misst und ganz besonders bevorzugt in dB (A) Gewichtung bestimmt.

5. Bestrahlungsvorrichtung nach einem der vorherigen Ansprüche **dadurch gekennzeichnet, dass** die Vorrichtung ein Gerät zur Messung von evozierten otoakustischen Emission des Innenohres umfasst, welches Messdaten an die Regelungseinheit zur Steuerung der Ausgangsleistung des Photonenstrahlers überträgt.

6. Bestrahlungsvorrichtung nach einem der vorherigen Ansprüche **dadurch gekennzeichnet, dass** die Bestrahlungsvorrichtung ein Gerät zur Messung der Veränderung einer Körperposition des Trägers der Bestrahlungsvorrichtung umfasst, welches Messdaten an die Regelungseinheit zur Steuerung der Ausgangsleistung des Photonenstrahlers überträgt, wobei bevorzugt die Veränderung einer Körperposition des Trägers der Bestrahlungsvorrichtung dreidimensional im Raum als Veränderung der Winkelgeschwindigkeit von Vorwärts-, Rückwärts- und Seitwärtsbewegungen des Körperschwerpunktes am Träger bestimmt werden

7. Bestrahlungsvorrichtung nach Anspruch **6 dadurch gekennzeichnet, dass** das Gerät zur Messung der Veränderung einer Körperposition des Trägers der Bestrahlungsvorrichtung mehrere orthogonal zueinanderstehende Gyrometer aufweist, welche die Veränderung der Winkelgeschwindigkeit von Vorwärts-, Rückwärts- und Seitwärtsbewegungen des Körpers bestimmen.

8. Bestrahlungsvorrichtung nach einem der Ansprüche **6** oder**7** **dadurch gekennzeichnet, dass** die Bestrahlungsvorrichtung am Körper angebrachte Aktoren umfasst, wobei die Aktivität der Aktoren proportional zu der festgestellten Veränderung einer Körperposition ist und innerhalb auf den Bewegungslauf bezogener Grenzen der Werte der Veränderung einer Körperposition die Aktivierung der Aktoren unterbleibt.

9. Bestrahlungsvorrichtung nach einem der vorherigen Ansprüche **dadurch gekennzeichnet, dass** der Photonenstrahler ein Laser besonders bevorzugt eine Laserdiode ist, ganz besonders bevorzugt eine Laserdiode umfassend ein Halbleitermaterial ausgewählt aus der Gruppe umfassend Galliumarsenid (GaAs), Aluminiumgalliumarsenid (AlGaAs), Indiumgalliumarsenid (InGaAs), Galliumarsenidphosphid (GaAsP), Aluminiumgalliumindiumphosphid (AlGaInP), Galliumphosphid (GaP), Galliumarsenidphosphid (GaAsP), Aluminiumgalliumindiumphosphid (AlGaInP) und Galliumarsenidphosphid (GaP); oder der Photonenstrahler eine Leuchtdiode ist bevorzugt eine Leuchtdiode umfassend ein Halbleitermaterial ausgewählt aus der Gruppe umfassend Galliumarsenid (GaAs), Aluminiumgalliumarsenid (AlGaAs), Indiumgalliumarsenid (InGaAs), Galliumarsenidphosphid (GaAsP), Aluminiumgalliumindiumphosphid (AlGaInP), Galliumphosphid (GaP), Galliumarsenidphosphid (GaAsP), Aluminiumgalliumindiumphosphid (AlGaInP) und Galliumarsenidphosphid (GaP) ist.

10. Bestrahlungsvorrichtung nach einem der vorherigen Ansprüche **dadurch gekennzeichnet, dass** der Photonenstrahl durch ein Photonenstrahlleitungssystem geführt wird, welches bevorzugt ein Lichtleitkabel umfasst mit einem bevorzugten Außendurchmesser von 1 mm bis 8 mm, besonders bevorzugt von 3 mm bis 5 mm, wobei das Photonenstrahlleitungssystem bevorzugt Linsen und/oder Spiegel umfasst, welche den Photonenstrahl bündeln, aufweiten und/oder kollimieren und das Photonenstrahlleitungssystem den Photonenstrahl auf eine vorbestimmte Region des Innenohres führt, wobei die vorbestimmte Region bevorzugt die Kochlea und/oder das Vestibulum umfasst.

**11.** Bestrahlungsvorrichtung nach einem der vorherigen Ansprüche
**dadurch gekennzeichnet, dass** die Bestrahlungsvorrichtung in Kombination mit einem Hörgerät vorliegt, wobei das Hörgerät bevorzugt ein schallverstärkendes Gerät, besonders bevorzugt ein Im-Ohr-Gerät oder ein Hinter-dem-Ohr-Gerät, ist oder das Hörgerät ein implantierbares Hörgerät, bevorzugt ein Kochlea-Implant, ist.

**12.** System zur protektiven Bestrahlung eines Innenohres eines Trägers zur Prävention von Hör- und/oder Gleichgewichtstörungen umfassend eine Bestrahlungsvorrichtung nach einem vorherigen Ansprüche, wobei

a) Signale über den Träger der Bestrahlungsvorrichtung und/oder die Umgebung des Trägers gemessen werden,
b) die Ausgangsleistung des Photonenstrahlers (P) zur protektiven Bestrahlung des Innenohres basierend auf diesen Messdaten berechnet wird,
c) die Ausgangsleistung des Photonenstrahlers auf den berechneten Wert P eingestellt wird und die Bestrahlung des Innenohres mit der Ausgangsleistung P erfolgt,

wobei die Messdaten akustische Signale aus der Umgebung des Trägers umfassen und aus diesen Messedaten mindestens ein Parameter B zur Berechnung der Ausgangsleistung der Photonenstrahlung P bestimmt wird und die Messdaten evozierte otoakustischen Emissionen der äußeren Haarzellen des Innenohres umfassen und aus diesen Messdaten mindestens ein Parameter A zur Berechnung der Ausgangsleistung der Photonenstrahlung P bestimmt wird und/oder die Messdaten Veränderungen der Körperposition des Trägers der Bestrahlungsvorrichtung umfassen und aus diesen Messedaten mindestens ein Parameter C zur Berechnung der Ausgangsleistung der Photonenstrahlung P bestimmt wird, wobei sich die Ausgangsleistung P aus der Summierung von drei positiven Summanden berechnet, wobei der erste Summand PA mit Hilfe des Parameter A berechnet wird, der zweite Summand PB mit Hilfe des Parameter B berechnet wird, der dritte Summand PC mit Hilfe des Parameter C berechnet wird und falls die Summe aus PA, PB und PC einen Maximalwert M überschreitet P = M ist, wobei der Maximalwert bevorzugt zwischen 100 mW und 300 mW und ganz besonders bevorzugt bei 120 mW liegt.

**13.** System nach Anspruch **12**
**dadurch gekennzeichnet, dass** der durch die Messung der evozierten otoakustischen Emission bestimmte Parameter A die Reproduzierbarkeit ist und PA eine monoton fallende Funktion von A ist, wobei bevorzugt für A>=59% PA=1mW ist und für A<59% PA=(60%-A)*1mW ist.

**14.** System nach einem Ansprüche **12** oder **13**
**dadurch gekennzeichnet, dass** der Parameter B der Schalldruckpegel ist und PB eine monoton steigende Funktion von B ist, PB für Werte von B, die unterhalb eines Schalldruckpegelgrenzwertes (G) liegen, 0 mW ist, der Schalldruckpegelgrenzwert G zwischen 75 dB und 95 dB, bevorzugt bei 85 dB, liegt und bevorzugt für B>=G PB berechnet wird durch PB= $2^{floor((B-G)/3dB)+1}$ * (PA+PC) -(PA+PC).

**15.** System nach einem der Ansprüche **12-14**
**dadurch gekennzeichnet, dass** der Parameter C in einem Balancetest bestimmt wird und sich aus den Vorwärts-, Rückwärts- und/oder Seitwärtsbewegungen des Trägers der Bestrahlungsvorrichtung bezogen auf alters-, geschlechts- und übungsspezifische Normwerte berechnet, wobei C bevorzugt in % gemessen wird und dem Standard Balance Deficit Test (SBDT) composite score entspricht, wobei bevorzugt für C kleiner als 50% PC=0 mW und für C größer gleich 50% PC=(C-45%)*0,2 mW ist.

**Claims**

**1.** An irradiation apparatus for the prophylaxis of hearing impairment and/or vertigo, comprising a photon emitter for irradiating an inner ear at a wavelength between 600 nm and 1200 nm, preferably between 700 nm and 900 nm, and a device for measuring acoustic signals of surroundings, wherein said device determines the sound pressure level, and an automated control unit,
**characterized in that**
the automated control unit controls the output power of the photon emitter based on the measured sound pressure level in a range between 0.1 mW and 1000 mW, wherein the output power of the photon emitter is a constant function of the sound pressure level up to a sound pressure level limiting value and a monotonically increasing function from the sound pressure level limiting value.

**2.** The irradiation apparatus for the prophylaxis of hearing impairment and/or vertigo according to the preceding claim, **characterized in that**
the sound pressure level limiting value is between 75 dB and 95 dB.

**3.** The irradiation apparatus for the prophylaxis of hearing impairment and/or vertigo according to the preceding claims, **characterized in that**
the automated control unit controls the output power of the photon emitter in a range between 0.5 mW and 300 mW, wherein a maximum value of 300 mW is not exceeded.

**4.** The irradiation apparatus according to any one of the preceding claims,
**characterized in that** the device for measuring acoustic signals of surroundings measures the sound pressure level in a preferred sound frequency range from 50 Hz to 20000 Hz, particularly preferably from 250 Hz to 8000 Hz, and most preferably determines weighting in dB (A).

**5.** The irradiation apparatus according to any one of the preceding claims,
**characterized in that** the apparatus comprises a device for measuring evoked otoacoustic emissions of the inner ear, wherein said device transmits measured data to the control unit for controlling the output power of the photon emitter.

**6.** The irradiation apparatus according to any one of the preceding claims,
**characterized in that** the irradiation apparatus comprises a device for measuring the change in a bodily position of the wearer of the irradiation apparatus, wherein said device transmits measured data to the control unit for controlling the output power of the photon emitter, wherein the change in a bodily position of the wearer of the irradiation apparatus is preferably determined in three-dimensional space as a change in the angular velocity of forward, backward, and lateral movements of a center of gravity of the wearer's body.

**7.** The irradiation apparatus according to claim **6**,
**characterized in that** the device for measuring the change in a bodily position of the wearer of the irradiation apparatus comprises multiple mutually orthogonal gyrometers that determine the change in the angular velocity of forward, backward, and lateral movements of the body.

**8.** The irradiation apparatus according to one of claims **6** or **7**,
**characterized in that** the irradiation apparatus comprises actuators that are attached to the wearer's body, wherein an activity of the actuators is proportional to a determined change in a bodily position, and the activation does not occur within limits of values of change in a bodily position based on the movement sequence.

**9.** The irradiation apparatus according to any one of the preceding claims,
**characterized in that** the photon emitter is a laser, particularly preferably a laser diode, most preferably a laser diode comprising a semiconductor material selected from the group consisting of gallium arsenide (GaAs), aluminum gallium arsenide (AlGaAs), indium gallium arsenide (InGaAs), gallium arsenide phosphide (GaAsP), aluminum gallium indium phosphide (AlGaInP), gallium phosphide (GaP), gallium arsenide phosphide (GaAsP), aluminum gallium indium phosphide (AlGaInP), and gallium arsenide phosphide (GaP); or **in that** the photon emitter is a light emitting diode, preferably a light emitting diode comprising a semiconductor material selected from the group consisting of gallium arsenide (GaAs), aluminum gallium arsenide (AlGaAs), indium gallium arsenide (InGaAs), gallium arsenide phosphide (GaAsP), aluminum gallium indium phosphide (AlGaInP), gallium phosphide (GaP), gallium arsenide phosphide (GaAsP), aluminum gallium indium phosphide (AlGaInP), and gallium arsenide phosphide (GaP).

**10.** The irradiation apparatus according to any one of the preceding claims,
**characterized in that** the photon beam of the photon emitter is conducted through a photon beam conduction system, which preferably comprises an optical fiber cable having a preferred outer diameter of 1 mm to 8 mm, particularly preferably of 3 mm to 5 mm, wherein the photon beam conduction system preferably comprises lenses and/or mirrors that bundle, expand, and/or collimate the photon beam and the photon beam conduction system conducts the photon beam to a predetermined region of the inner ear, wherein said predetermined region preferably comprises the cochlea and/or the vestibule.

**11.** The irradiation apparatus according to any one of the preceding claims,
**characterized in that** the irradiation apparatus is present in combination with a hearing aid, wherein the hearing aid preferably is a sound-amplifying device, particularly preferably an in-the-ear device or a behind-the-ear device, or the hearing aid is an implantable hearing aid, preferably a cochlear implant.

12. A system for protective irradiation of an inner ear of a wearer for the prophylaxis of hearing impairment and/or vertigo, comprising an irradiation apparatus according to any one of the preceding claims, wherein

a) signals concerning the wearer of the irradiation apparatus and/or surroundings of the wearer are measured,
b) an output power (P) of the photon emitter for protective irradiation of the inner ear is calculated based on this measured data,
c) the output power of the photon emitter is set to the calculated value P, and the irradiation of the inner ear is effected with the output power P,

wherein the measured data comprise acoustic signals from the wearer's surroundings and at least a parameter B for calculating the output power P of the photon radiation is determined based on said measured data, and the measured data comprise evoked otoacoustic emissions of the outer hair cells of the inner ear, and at least a parameter A for calculating the output power P of the photon radiation is determined from this measured data and/or the measured data comprise changes in the bodily position of the wearer of the irradiation apparatus, and at least a parameter C for calculating the output power P of the photon radiation is determined from this measured data wherein the output power P is calculated from the sum of three positive summands, wherein the first summand PA is calculated using parameter A, the second summand PB is calculated using parameter B, and the third summand PC is calculated using parameter C, and if the sum of PA, PB, and PC exceeds a maximum value M, then P=M, wherein the maximum value is preferably between 100 mW and 300 mW, and most preferably is 120 mW.

13. The system according to claim **12**,
**characterized in that** parameter A, determined by measuring the evoked otoacoustic emissions, is reproducibility, and PA is a monotonically decreasing function of A, wherein preferably PA=1 mW for A>=59% and PA=(60%-A)\*1 mW for A<59%.

14. The system according to one of claims **12** or **13, characterized in that** parameter B is the sound pressure level and PB is a monotonically increasing function of B, PB is 0 mW for values of B that are below a sound pressure level limiting value (G), the sound pressure level limiting value G being between 75 dB and 95 dB, preferably at 85 dB, and preferably being calculated for B>=G PB by PB = $2^{\text{floor}((B-G)/3dB)+1}$ \* (PA+PC) - (PA+PC).

15. The system according to one of claims **12-14,**
**characterized in that** parameter C is determined in a balance test, and is calculated from the forward, backward, and/or lateral movements of the wearer of the irradiation apparatus, based on standard values that are specific to age, gender, and exercise, wherein C is preferably measured in %, and corresponds to the standard balance deficit test (SBDT) composite score, wherein preferably PC=0 mW for C less than 50%, and PC=(C-45%)\*0.2 mW for C greater than or equal to 50%.

**Revendications**

1. Dispositif d'irradiation pour la prévention des troubles de l'audition et/ou de l'équilibre, comprenant un émetteur de photons pour l'irradiation d'une oreille interne avec une longueur d'onde comprise entre 600 nm et 1 200 nm, de préférence entre 700 nm et 900 nm, un appareil pour la mesure de signaux acoustiques des environs, lequel détermine le niveau de pression acoustique, et une unité de réglage automatisée, **caractérisé en ce que** l'unité de mesure automatisée commande la puissance de sortie de l'émetteur de photons, sur la base du niveau de pression acoustique mesurée, dans une plage comprise entre 0,1 mW et 1 000 mW, dans lequel la puissance de sortie de l'émetteur de photons est une fonction constante de niveau de pression acoustique jusqu'à une valeur limite de niveau de pression acoustique, et est une fonction croissante de façon monotone du niveau de pression acoustique à partir de la valeur limite de niveau de pression acoustique.

2. Dispositif d'irradiation pour la prévention des troubles de l'audition et/ou de l'équilibre selon la revendication précédente,
**caractérisé en ce que**
la valeur limite de niveau de pression acoustique est comprise entre 75 dB et 95 dB.

3. Dispositif d'irradiation pour la prévention des troubles de l'audition et/ou de l'équilibre selon l'une des revendications précédentes,
   **caractérisé en ce que**
   l'unité de réglage automatisée commande la puissance de sortie de l'émetteur de photons dans une plage comprise entre 0,5 mW et 300 mW, dans lequel une valeur maximale de 300 mW n'est pas excédée.

4. Dispositif d'irradiation selon l'une des revendications précédentes,
   **caractérisé en ce que** l'appareil de mesure des signaux acoustiques des environs mesure le niveau de pression acoustique dans une plage de fréquence acoustique préférée de 50 Hz à 20 000 Hz, de manière particulièrement préférée de 250 Hz à 8 000 Hz, et détermine de manière tout particulièrement préférée la pondération en dB (A).

5. Dispositif d'irradiation selon l'une des revendications précédentes,
   **caractérisé en ce que** le dispositif comprend un appareil de mesure d'émissions oto-acoustiques évoquées de l'oreille interne, lesquelles données de mesure sont transmises à l'unité de réglage pour la commande de la puissance de sortie de l'émetteur de photons.

6. Dispositif d'irradiation selon l'une des revendications précédentes,
   **caractérisé en ce que** le dispositif d'irradiation comprend un appareil de mesure du changement d'une position de corps du porteur du dispositif d'irradiation, lequel transmet les données de mesure à l'unité de réglage pour la commande de la puissance de sortie de l'émetteur de photons, dans lequel le changement d'une position de corps du porteur du dispositif d'irradiation est déterminé de préférence de manière tridimensionnelle dans l'espace en tant que changement de la vitesse angulaire des mouvements vers l'avant, vers l'arrière et latéraux du centre de gravité du corps sur le porteur.

7. Dispositif d'irradiation selon la revendication **6,**
   **caractérisé en ce que** l'appareil de mesure du changement d'une position de corps du porteur du dispositif d'irradiation présente plusieurs gyromètres disposés de manière orthogonale l'un par rapport à l'autre, lesquels déterminent le changement de la vitesse angulaire des mouvements vers l'avant, vers l'arrière et latéraux du corps.

8. Dispositif d'irradiation selon la revendication **6** ou **7, caractérisé en ce que** le dispositif d'irradiation comprend des actionneurs fixés sur le corps, dans lequel l'activité des actionneurs est proportionnelle au changement constaté d'une position de corps et l'activation des actionneurs n'est pas effectuée à l'intérieur des limites se rapportant au cours du mouvement des valeurs du changement d'une position de corps.

9. Dispositif d'irradiation selon l'une des revendications précédentes, **caractérisé en ce que** l'émetteur de photons est un laser, de manière particulièrement préférée une diode laser, de manière tout particulièrement préférée une diode laser comprenant un matériau semi-conducteur choisi dans le groupe comprenant l'arséniure de gallium (GaAs), l'arséniure de gallium d'aluminium (AlGaAs), l'arséniure de gallium d'indium (InGaAs), le phospho-arséniure de gallium (GaAsP), le phosphure d'indium de gallium d'aluminium (AlGaInP), le phosphure de gallium (GaP), le phospho-arséniure de gallium (GaAsP), le phosphure d'indium de gallium d'aluminium (AlGaInP) et le phospho-arséniurephospho-arséniure de gallium (GaP) ; ou l'émetteur de photons est une diode électroluminescente, de préférence une diode électroluminescente comprenant un matériau semi-conducteur choisi dans le groupe comprenant l'arséniure de gallium (GaAs), l'arséniure de gallium d'aluminium (AlGaAs), l'arséniure de gallium d'indium (InGaAs), le phospho-arséniure de gallium (GaAsP), le phosphure d'indium de gallium d'aluminium (AlGaInP), le phospho-arséniure de gallium (GaP), le phospho-arséniure de gallium (GaAsP), le phosphure d'indium de gallium d'aluminium (AlGaInP) et le phospho-arséniure de gallium (GaP).

10. Dispositif d'irradiation selon l'une des revendications précédentes, **caractérisé en ce que** le rayon de photons est guidé par un système de conduite de rayon de photons, lequel comprend de préférence un câble de fibres optiques avec un diamètre extérieur préféré de 1 mm à 8 mm, de manière particulièrement préférée de 3 mm à 5 mm, dans lequel le système de conduite de rayon de photons comprend de préférence des lentilles et/ou des miroirs, lesquels rassemblent, élargissent et collimatent le rayon de photons et le système de conduite de rayon de photons guide le rayon de photons sur une région prédéterminée de l'oreille interne, dans lequel la région prédéterminée comprend de préférence la cochlée et/ou le vestibule.

11. Dispositif d'irradiation selon l'une des revendications précédentes,
    **caractérisé en ce que** le dispositif d'irradiation est présent en combinaison avec un appareil auditif, dans lequel l'appareil auditif est de préférence un appareil d'amplification acoustique, de manière particulièrement préférée un appareil dans l'oreille ou un appareil derrière l'oreille, ou l'appareil auditif est un appareil auditif implantable, de préférence un implant cochléaire.

12. Système d'irradiation protective d'une oreille interne d'un porteur pour la prévention des troubles de l'audition et/ou de l'équilibre, comprenant un dispositif d'irradiation selon l'une des revendications précédentes, dans lequel

a) les signaux sur le porteur du dispositif d'irradiation et/ou les environs du porteur sont mesurés,
b) la puissance de sortie de l'émetteur de photons (P) pour l'irradiation protective de l'oreille interne est calculée sur la base de ces données de mesure,
c) la puissance de sortie de l'émetteur de photons est réglée sur la valeur définie P et l'irradiation de l'oreille interne a lieu avec la puissance de sortie P,

dans lequel les données de mesure comprennent des signaux acoustiques provenant des environs du porteur et au moins un paramètre B est déterminé à partir de ces données de mesure pour le calcul de la puissance de sortie du rayonnement de photons P et les données de mesure comprennent des émissions oto-acoustiques évoquées des cellules auditives extérieures de l'oreille interne et au moins un paramètre A est déterminé à partir de ces données de mesure pour le calcul de la puissance de sortie du rayonnement de photons P et/ou les données de mesure comprennent des changements de la position de corps du porteur du dispositif d'irradiation et au moins un paramètre C est déterminé à partir de ces données de mesure pour le calcul de la puissance de sortie du rayonnement de photons P,
dans lequel la puissance de sortie P se calcule par l'addition de trois opérandes positifs, dans lequel le premier opérande PA est calculé à l'aide du paramètre A, le deuxième opérande PB est calculé à l'aide du paramètre B, le troisième opérande PC est calculé à l'aide du paramètre C, et si la somme de PA, PB et PC excède une valeur maximale M, alors P = M, dans lequel la valeur maximale est comprise de préférence entre 100 mW et 300 mW, et s'élève de manière particulièrement préférée à 120 mW.

13. Système selon la revendication 12,
**caractérisé en ce que** le paramètre A déterminé par la mesure de l'émission oto-acoustique évoquée est la reproductibilité et PA est une fonction décroissante de manière monotone de A, dans lequel, de préférence, pour A>=59 %, PA=1 mW et pour A<59 %, PA=(60 %-A)*1mW.

14. Système selon la revendication 12 ou 13,
**caractérisé en ce que** le paramètre B est le niveau de pression acoustique et PB est une fonction croissante de manière monotone de B, PB, pour des valeurs de B qui sont inférieures à une valeur de limite de niveau de pression acoustique (G), s'élève à 0 mW, la valeur de limite de niveau de pression acoustique G est comprise entre 75 dB et 95 dB, de préférence de 85 dB, et de préférence, pour B>=G, PB est calculé par PB= $2^{floor((B-G)/3dB)+1}$ * (PA+PC) - (PA+PC).

15. Système selon l'une des revendications 12 à 14,
**caractérisé en ce que** le paramètre C est déterminé dans un essai d'équilibrage et se calcule à partir des mouvements vers l'avant, vers l'arrière et latéraux du porteur du dispositif d'irradiation par rapport aux valeurs de norme spécifiques à l'âge, au sexe et à l'exercice, dans lequel C est mesuré de préférence en % et l'essai de déficit d'équilibre standard (SBDT) correspond au score composite, dans lequel, de préférence, pour C inférieur à 50 %, PC=O mW et pour C supérieur ou égal à 50 %, PC=(C-45 %)*0,2 mW.

Fig. 1

A)  5  B)

6  7

8

Fig. 2

C)

D)

5

6

7

9

Fig. 3

Fig. 4

Fig. 5

Fig. 6

**IN DER BESCHREIBUNG AUFGEFÜHRTE DOKUMENTE**

**In der Beschreibung aufgeführte Patentdokumente**

- DE 29720442 U1
- DE 29808193 U1
- US 20130172960 A1
- US 2011295331 A1
- US 2013023962 A1
- US 2010174329 A1
- US 2011282417 A1

**In der Beschreibung aufgeführte Nicht-Patentliteratur**

- **PRELL et al.** *Hear Res.,* 2007, vol. 226 (1-2), 22-43
- Otoacoustic Emission - Clinical Applications. Thieme, 2007